# EUROPÄISCHE PATENTSCHRIFT

(11) **EP 0 588 146 B1**
(45) Veröffentlichungstag und Bekanntmachung des Hinweises auf die Patenterteilung: **11.11.1998**
(21) Anmeldenummer: 93113887.9
(22) Anmeldetag: 31.08.1993
(51) Int. Cl.: C07D 401/04, C07D 405/14, C07D 213/53, A01N 43/54, C07D 213/46, A01N 43/40, C07D 213/76

(54) **Substituierte Pyridin-Derivate und diese enthaltenden Schädlingsbekämpfungsmittel**
Substituted pyridin derivatives and parasiticides containing them
Dérivés de pyridine substitués et parasiticides les contenant

(30) Priorität: 10.09.1992 DE 4230215
(43) Veröffentlichungstag der Anmeldung: 23.03.1994
(73) Patentinhaber: BASF Aktiengesellschaft, 67063 Ludwigshafen (DE)
(72) Erfinder: Mueller, Thomas, Dr., W-6719 Hessheim (DE); Eicken, Karl, Dr., W-6706 Wachenheim (DE); Harreus, Albrecht, Dr., D-6700 Ludwigshafen (DE); Koenig, Hartmann, Dr., D-6703 Limburgerhof (DE); Rentzea, Costin, Dr., D-6900 Heidelberg (DE); Ammermann, Eberhard, Dr., D-6148 Heppenheim (DE); Lorenz, Gisela, Dr., D-6730 Neustadt (DE)

(56) Entgegenhaltungen:
- DE-A- 4 001 557
- CANADIAN JOURNAL OF MICROBIOLOGY, Bd.5, Nr.4, 1959 Seiten 317 - 321 A. FUNK AND P.V. DIVEKAR 'Caerulomycin, anew antibiotic from streptomyces caeruleus baldacci'
- CANADIAN JOURNAL OF CHEMISTRY, Bd.45, 1967, OTTAWA CA Seiten 1215 - 1223 L.C. VINING 'Caerulomycin'
- CHEMICAL ABSTRACTS, vol. 119, no. 13, 27. September 1993, Columbus, Ohio, US; abstract no. 135507, G. SHUICHI 'Antibiotic SF 2738' Seite 502 ;

## Beschreibung

Die vorliegende Erfindung betrifft neue substituierte Pyridin-Derivate, Verfahren zu ihrer Herstellung, sie enthaltende Mittel und ihre Verwendung als Schädlingsbekämpfungsmittel, insbesondere als Fungizide.

Es ist bekannt, daß substituierte Pyridin-Derivate fungizide Eigenschaften besitzen (vgl. EP 270362, EP 407899, EP431421, Can. J. Microbiol. 5 (1959) 317, Can. J. Chem. 45 (1967) 1215). Die Wirkung dieser Verbindungen ist jedoch insbesondere bei niedrigen Aufwandmengen und -konzentrationen nicht immer befriedigend.

Es wurde nun gefunden, daß substituierte Pyridin-Derivate der Formel I, 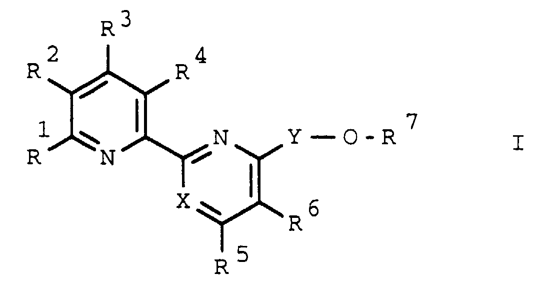 worin
- R¹ =: Wasserstoff, C₁-C₆-Alkyl, C₁-C₄-Alkoxy-C₁-C₄-alkyl, C₁-C₄-Alkylthio-C₁-C₄-alkyl, C₂-C₆-Alkenyl, C₂-C₆-Alkinyl, C₃-C₇-Cycloalkyl, wobei der Cycloalkylrest bis zu dreifach durch C₁-C₄-Alkyl substituiert sein kann, Halogen, Phenyl, Phenoxy-C₁-C₄-alkyl, Phenylmercapto-C₁-C₄-alkyl, Phenyl-C₁-C₄-alkyl, Phenoxy, Phenylmercapto, wobei die sechs letztgenannten Reste im Phenylteil bis zu dreifach durch Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein können,
- R², R³, R⁴ =: unabhängig voneinander Wasserstoff, C₁-C₆-Alkyl, Phenyl, wobei der Phenylrest bis zu dreifach durch Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein kann,
- R⁵ =: Wasserstoff, C₁-C₆-Alkyl, C₃-C₇-Cycloalkyl, C₃-C₇-Cycloalkyl-C₁-C₄-alkyl, wobei die beiden letztgenannten Reste im Cycloalkylteil bis zu dreifach durch C₁-C₄-Alkyl substituiert sein können, C₁-C₄-Haloalkyl, C₁-C₄-Alkoxy-C₁-C₄-alkyl, C₁-C₄-Alkylthio-C₁-C₄-alkyl, C₂-C₆-Alkenyl, C2-C6-Alkinyl, Phenyl, PhePhenyl-C₁-C₄-alkyl, Phenoxy-C₁-C₄-alkyl, Phenylmercapto-C₁-C₄-alkyl, wobei die vier letztgenannten Reste im Phenylteil bis zu dreifach durch Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein können,
- R⁶ =: Wasserstoff, C₁-C₄-Alkyl, C₁-C₄-Alkoxy, C₁-C₄-Alkoxycarbonyl, Halogen, Phenyl, wobei der Phenylrest bis zu dreifach durch Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein kann, oder
R⁵ und R⁶ bilden zusammen eine Polymethylenkette der Formel -(CH₂)ₘ- mit m = 3 oder 4,
- R⁷ =: Wasserstoff, C₁-C₁₂-Alkyl, C₃-C₁₂-Alkenyl, C₃-C₈-Alkinyl, wobei die drei letztgenannten Gruppen bis zu dreifach durch Halogen substituiert sein können, C₁-C₆-Alkoxy-C₂-C₁₀-alkyl, monocyclisches oder polycyclisches C₃-C₁₀-Cycloalkyl oder C₃-C₁₀-Cycloalkylmethyl, wobei diese Ringe bis zu dreifach durch C₁-C₄-Alkyl oder durch einen Phenylrest substituiert sein können,
monocyclisches oder polycyclisches C₅-C₁₀-Cycloalkenyl oder C₅-C₁₀-Cycloalkenylmethyl, wobei diese Ringe bis zu dreifach durch C₁-C₄-Alkyl oder durch einen Phenylrest substituiert sein können, Phenyl, Phenyl-C₁-C₆-alkyl, Phenyl-C₃-C₆-alkenyl, Phenoxy-C₂-C₆-alkyl, wobei die vier letztgenannten Reste im Phenylteil bis zu dreifach durch Halogen, Nitro, Cyano, C₁-C₄-Alkyl,
C₁-C₄-Haloalkyl, C₁-C₄-Alkoxy, C₁-C₄-Haloalkoxy oder C₁-C₄-Alkylthio substituiert sein können,
X = CH oder N, 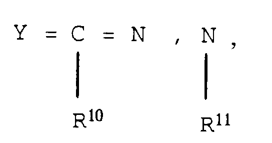
- R¹⁰ =: Wasserstoff, C₁-C₆-Alkyl,
- R¹¹ =: Wasserstoff, C₁-C₆-Alkyl, C₃-C₈-Cycloalkyl, wobei der Cycloalkylrest bis zu dreifach durch C₁-C₄-Alkyl oder durch einen Phenylrest substituiert sein kann, Phenyl, Phenyl-C₁-C₄-alkyl, wobei die beiden letztgenannten Reste im Phenylteil bis zu dreifach durch Halogen, C₁-C₄-Alkyl, C₁-C₄-Alkoxy oder C₁-C₄-Haloalkyl substituiert sein können,

bedeuten, sowie deren pflanzenverträglichen Säureadditionssalze und Metallsalzkomplexe,
eine ausgezeichnete fungizide Wirkung gegen phytopathogene Pilze besitzen.

Wenn Y NH bedeutet, können die Verbindungen in tautomeren Formen vorliegen, die von der Erfindung umfaßt werden.

Wenn Y CR¹⁰ = N bedeutet, können die Verbindungen in stereoisomeren Formen vorliegen, die von der Erfindung umfaßt werden.

Im Hinblick auf ihre fungizide Wirkung werden diejenigen Verbindungen bevorzugt, in denen die Substituenten folgende Bedeutung haben:
- R¹ =: Wasserstoff, C₁-C₆-Alkyl, wie Methyl, Ethyl, Propyl, 1-Methylethyl, Butyl, 1-Methylpropyl, 2-Methylpropyl, 1,1-Dimethylethyl, Pentyl, Hexyl, insbesondere Methyl und Propyl,
C₁-C₃-Alkoxy-C₁-C₂-alkyl, wie Methoxymethyl, Ethoxymethyl, Propyloxymethyl, Methoxyethyl, Ethoxyethyl, Propyloxyethyl, vorzugsweise Methoxymethyl,
Phenyl, Phenyl-C₁-C₂-alkyl, wie Benzyl, Phenylethyl, insbesondere Benzyl, Phenoxy-C₁-C₂-alkyl, wie Phenoxymethyl, Phenoxyethyl, insbesondere Phenoxymethyl,
wobei die drei letztgenannten Reste im Phenylteil bis zu dreifach durch Halogen, wie Fluor, Chlor, Brom, Jod, oder C₁-C₄-Alkyl, wie Methyl, Ethyl, Propyl, 1-Methylethyl, Butyl, 1-Methylpropyl, 2-Methylpropyl, 1,1-Dimethylethyl, substituiert sein können,
- R², R³ =: unabhängig voneinander Wasserstoff, C₁-C₃-Alkyl, wie Methyl, Ethyl, Propyl, 1-Methylethyl, insbesondere Methyl und Ethyl, Phenyl, wobei der Phenylrest bis zu dreifach durch Halogen, wie Fluor, Chlor, Brom, Jod, oder C₁-C₄-Alkyl, wie Methyl, Ethyl, Propyl, 1-Methylethyl, Butyl, 1-Methylpropyl, 2-Methylpropyl, 1,1-Dimethylethyl, substituiert sein kann,
- R⁴ =: Wasserstoff
- R⁵ =: Wasserstoff, C₁-C₆-Alkyl, wie Methyl, Ethyl, Propyl, 1-Methylethyl, Butyl, 1-Methylpropyl, 2-Methylpropyl, 1,1-Dimethylethyl, Pentyl, Hexyl, insbesondere Methyl, Propyl und Butyl, C₃-C₆-Cycloalkyl, wie Cyclopropyl, Cyclobutyl, Cyclopentyl, Cyclohexyl, insbesondere Cyclopentyl, C₅-C₆-Cycloalkyl-C₁-C₃-alkyl, wie Cyclopentylmethyl, Cyclohexylmethyl, Cyclopentylethyl, Cyclohexylethyl, Cyclopentylpropyl, Cyclohexylpropyl, insbesondere Cyclopentylethyl,
Phenyl, Phenyl-C₁-C₂-alkyl, wie Benzyl, Phenylethyl, insbesondere Benzyl, wobei die beiden letztgenannten Reste im Phenylteil bis zu dreifach durch Halogen, wie Fluor, Chlor, Brom, Jod, C₁-C₄-Alkyl, wie Methyl, Ethyl, Propyl, 1-Methylethyl, Butyl, 1-Methylpropyl, 2-Methylpropyl, 1,1-Dimethylethyl, oder C₁-C₄-Alkoxy, wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy und Ethoxy substituiert sein können,
C₁-C₃-Haloalkyl, wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, insbesondere Trifluormethyl;
- R⁶ =: Wasserstoff, C₁-C₄-Alkyl, wie Methyl, Ethyl, Propyl, 1-Methylethyl, Butyl, 1-Methylpropyl, 2-Methylpropyl, 1,1-Dimethylethyl, insbesondere Methyl und Ethyl,
Halogen, wie Fluor, Chlor, Brom, Jod, insbesondere Chlor und Brom,
Phenyl, C₁-C₄-Alkoxy, wie Methoxy, Ethoxy, Propyloxy, 1-Methylethoxy, insbesondere Methoxy, oder
R⁵ und R⁶ bilden zusammen eine Polymethylenkette der Formel -(CH₂)ₘ- mit m = 3 oder 4,
- R⁷ =: Wasserstoff, C₁-C₆-Alkyl, wie Methyl, Ethyl, Propyl, 1-Methylethyl, Butyl, 1-Methylpropyl, 2-Methylpropyl, 1,1-Dimethylethyl, Pentyl, Hexyl, insbesondere Methyl, Ethyl, Propyl, 1-Methylethyl, Butyl, 1-Methylpropyloxy, 2-Methylpropyl und 1,1-Dimethylethyl, C₃-C₆-Alkenyl, wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, insbesondere 2-Propenyl, 2-Butenyl, 3-Butenyl, 1-Methyl-2-propenyl, 2-Methyl-2-propenyl und 3-Methyl-2-butenyl,
C₃-C₆-Alkinyl, wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl,2-Hexinyl, 3-Hexinyl, 4-Hexinyl, 5-Hexinyl, insbesondere 2-Propinyl und 2-Butinyl, wobei die drei letztgenannten Reste bis zu dreifach durch Halogen, wie Fluor, Chlor, Brom, Jod, insbesondere Fluor und Chlor, substituiert sein können,
C₁-C₃-Alkoxy-C₂-C₆-alkyl, wie Methoxyethyl, Methoxypropyl, Methoxybutyl, Methoxypentyl, Methoxyhexyl, Ethoxyethyl, Ethoxypropyl, Ethoxybutyl, Ethoxypentyl, Ethoxyhexyl, Propyloxyethyl, insbesondere Methoxyethyl, Methoxypropyl und Methoxybutyl,
monocyclisches oder polycyclisches C₃-C₁₀-Cycloalkyl, wie Cyclopropyl, Cyclobutyl, Cyclopentyl, Cyclohexyl, Cycloheptyl, Cyclooctyl, Menthyl, Norbornyl, Adamantyl, Tricyclodecanyl, insbesondere Cyclopropyl und Cyclohexyl, oder
monocyclisches oder polycyclisches C₃-C₁₀-Cycloalkylmethyl, wie Cyclopropylmethyl, Cyclobutylmethyl, Cyclopentylmethyl, Cyclohexylmethyl, Cycloheptylmethyl, Cyclooctylmethyl, insbesondere Cyclopropylmethyl oder Cyclohexylmethyl,
wobei diese Ringe ein bis zwei C₁-C₄-Alkylgruppen, wie Methyl, Ethyl, Propyl, 1-Methylethyl, Butyl, 1-Methylpropyl, 2-Methylpropyl und 1,1-Dimethylethyl, insbesondere Methyl und Ethyl, oder einen Phenylrest tragen können,
monocyclisches oder polycyclisches C₅-C₁₀-Cycloalkenyl, wie insbesondere 2-Cyclohexen-1-yl, oder monocyclisches oder polycyclisches C₅-C₁₀-Cycloalkenylmethyl, wie insbesondere 1-Cyclohexenylmethyl, 2-Cyclohexenylmethyl und 3-Cyclohexenylmethyl,
wobei diese Ringe ein bis zwei C₁-C₄-Alkylgruppen, wie Methyl, Ethyl, Propyl, 1-Methylethyl, Butyl, 1-Methylpropyl, 2-Methylpropyl und 1,1-Dimethylethyl, insbesondere Methyl, oder einen Phenylrest tragen können,
Phenyl, Phenyl-C₁-C₆-alkyl, wie Benzyl, 1-Phenylethyl, 2-Phenylethyl, 1-Phenylpropyl, 2-Phenylpropyl, 3-Phenylpropyl, 1-Methyl-1-phenylethyl, 1-Phenylbutyl, 2-Phenylbutyl, 3-Phenylbutyl, 4-Phenylbutyl, 1-Methyl-1-phenylpropyl, 1-Methyl-2-phenylpropyl, 1-Methyl-3-phenylpropyl, 2-Methyl-1-phenylpropyl, 2-Methyl-2-phenylpropyl, 2-Methyl-3-phenylpropyl,
1,1-Dimethyl-2-phenylethyl, 5-Phenylpentyl, 6-Phenylhexyl, insbesondere Benzyl, 2-Phenylethyl und 4-Phenylbutyl, Phenyl-C₃-C₆-alkenyl, wie insbesondere 3-Phenyl-2-propenyl, 4-Phenyl-2-butenyl und 4-Phenyl-3-butenyl,
Phenoxy-C₂-C₆-alkyl, wie Phenoxyethyl, Phenoxypropyl, 2-Phenoxy-2-methylethyl, Phenoxybutyl, Phenoxypentyl, Phenoxyhexyl, insbesondere Phenoxyethyl, 2-Phenoxy-2-methylethyl und Phenoxybutyl,
wobei die vier letztgenannten Reste im Phenylteil bis zu dreifach durch Halogen, wie Fluor, Chlor, Brom, Jod, insbesondere Fluor und Chlor, C₁-C₄-Alkyl, wie Methyl, Ethyl, Propyl, 1-Methylethyl, Butyl, 1-Methylpropyl, 2-Methylpropyl und 1,1-Dimethylethyl, insbesondere Methyl, 1-Methylethyl und 1,1-Dimethylethyl,
C₁-C₃-Haloalkyl, wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl, oder
C₁-C₄-Alkoxy, wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy, substituiert sein können,
X = CH oder N 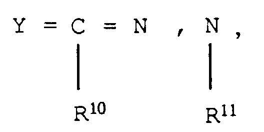
- R¹⁰=: Wasserstoff, C₁-C₄-Alkyl, wie
Methyl, Ethyl, Propyl, 1-Methylethyl, Butyl, 1-Methylpropyl, 2-Methylpropyl, 1,1-Dimethylethyl, insbesondere Methyl,
- R¹¹ =: Wasserstoff, C₁-C₄-Alkyl, wie Methyl, Ethyl, Propyl, 1-Methylethyl, Butyl, 1-Methylpropyl, 2-Methylpropyl und 1,1-Dimethylethyl, insbesondere Methyl,
C₃-C₈-Cycloalkyl, wie Cyclopropyl, Cyclobutyl, Cyclopentyl, Cyclohexyl, Cycloheptyl, Cyclooctyl, insbesondere Cyclopentyl und Cyclohexyl, wobei der Cycloalkylrest bis zu zweifach durch C₁-C₄-Alkyl, wie Methyl, Ethyl, Propyl, 1-Methylethyl, Butyl, 1-Methylpropyl, 2-Methylpropyl und 1,1-Dimethylethyl , insbesondere Methyl und Ethyl, oder durch einen Phenylrest substituiert sein kann,
Phenyl, Phenyl-C₁-C₂-alkyl, wie Benzyl, Phenylethyl, insbesondere Benzyl, wobei die zwei letztgenannten Reste im Phenylteil bis zu dreifach durch Halogen, wie Fluor, Chlor, Brom, Jod, insbesondere Fluor und Chlor, C₁-C₄-Alkyl, wie Methyl, Ethyl, Propyl, 1-Methylethyl, Butyl, 1-Methylpropyl, 2-Methylpropyl und 1,1-Dimethylethyl, insbesondere Methyl, 1-Methylethyl und 1,1-Dimethylethyl, C₁-C₃-Haloalkyl, wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl, oder
C₁-C₄-Alkoxy, wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy, substituiert sein können.

Als Säureadditionssalze eignen sich die pflanzenverträglichen Salze von solchen Säuren, die die fungizide Wirkung von I nicht beeinträchtigen, also z. B. die Iodide, Chloride, Bromide, Sulfate, Dodecylsulfate, Nitrate, Carbonate, Phosphate, Borate, Formiate, Acetate, Propionate, Benzoate, Oxalate, Naphthalinsulfonate, Dodecylbenzolsulfonate, Lactate, Citrate und die Salze mit dem Anion des Saccharins.

Als Metallkomplexe kommen die Komplexe des Kupfers, Zinks, Zinns, Mangans, Eisens, Kobalts oder Nickels in Betracht. Vorzugsweise stellt man die Komplexe aus den freien Basen I und den mineralsauren Salzen, beispielsweise den Chloriden oder Sulfaten, der Metalle her.

Gegenstand der vorliegenden Erfindung ist auch ein Verfahren zur Herstellung der subst. Pyridin-Derivate der Formel I, dadurch gekennzeichnet, daß man
1. eine Verbindung der Formel II, 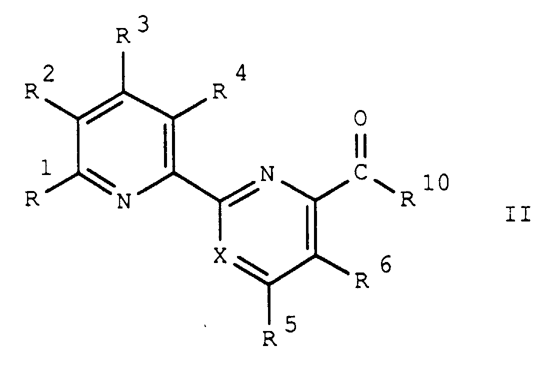 worin R¹ - R⁴, R¹⁰ und X die Bedeutung wie in Formel I gemäß Anspruch 1 oder 2 besitzen, mit einem Hydroxylaminsalz, wie Hydroxylammoniumchlorid oder -sulfat, in Gegenwart einer Base, wie die Carbonate und Hydrogencarbonate von Alkali- und Erdalkalimetallen, z.B. in einem protischen Lösungsmittel, wie Alkoholen oder Alkohol-Wasser-Gemischen, z.B. bei Temperaturen von 10 bis 100°C, bevorzugt 10 bis 70°C, zu einem Oxim der Formel I a 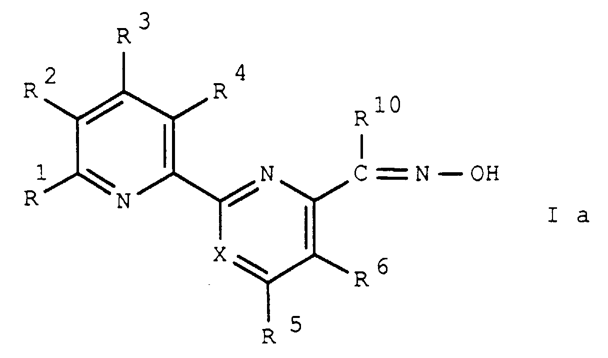 umsetzt, und das Oxim der Formel I a mittels einer Base, wie Natriumhydrid oder Kaliumhydroxid, in ein Oximsalz der Formel I b 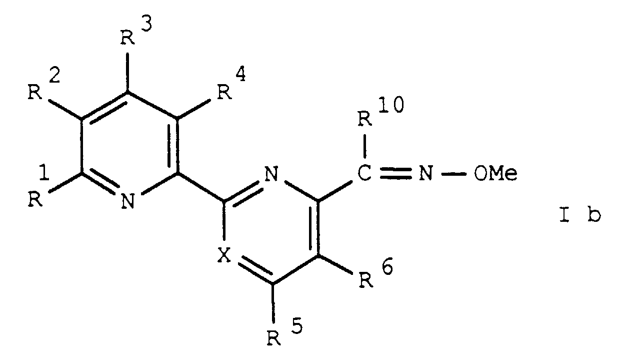 überführt, wobei Me ein Alkali- oder Erdalkaliatom, wie Natrium oder Kalium, darstellt,
   und das Oximsalz der Formel I b mit einer Verbindung der Formel III,

   R⁷-X' III

   worin R⁷ die Bedeutung wie in Formel I gemäß Anspruch 1 oder 2 besitzt, und X' eine nucleofuge Abgangsgruppe, wie Halogen, Tosylat, Mesylat, vorzugsweise Chlor, Brom oder Jod, darstellt, z.B. in einem inerten Lösungsmittel, wie Acetonitril, Dichlormethan, 1,2-Dichlorethan, Toluol, Xylol, Tetrahydrofuran, Dioxan, Diethylenglykoldimethylether, Dimethylformamid oder N-Methylpyrrolidon, z.B. bei Temperaturen von -30 bis 160°C, vorzugsweise -10 bis 110°C, zu einer Verbindung der Formel I c 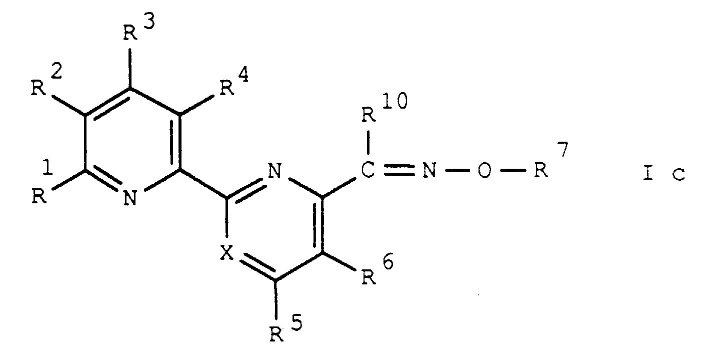 reagieren läßt, oder
2. eine Verbindung der Formel IV a

   H₂N-O-R⁷ • HA IV a

   mit einer Verbindung der Formel II 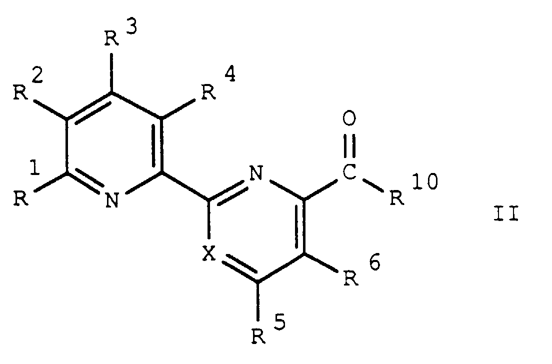 oder einer Verbindung der Formel V, 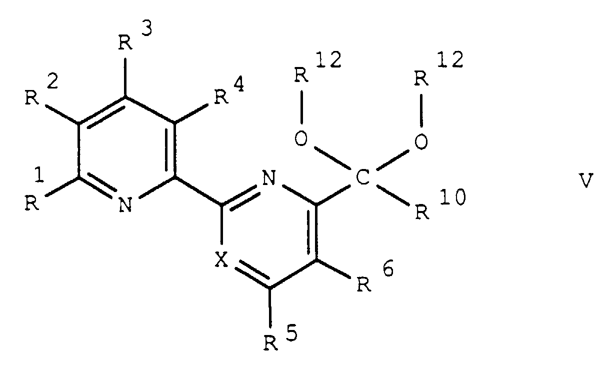 worin R¹ - R⁷, R¹⁰ und X die Bedeutung wie in Formel I gemäß Anspruch 1 oder 2 besitzen, HA eine anorganische oder organische Säure, wie Salzsäure, Schwefelsäure oder Essigsäure, und R¹² = C₁-C₄-Alkyl, wie Methyl, Ethyl, Propyl, Butyl, insbesondere Methyl und Ethyl, oder die beiden Reste R¹² zusammen eine Polymethylenkette der Formel -(CH₂)ₙ- mit n = 2 oder 3 darstellen, z.B. in einem protischen Lösungsmittel, wie Alkoholen oder Alkohol-Wasser-Gemischen, gegebenenfalls in Anwesenheit einer Base, wie die Carbonate und Hydrogencarbonate von Alkali- und Erdalkalimetallen, z.B. bei Temperaturen von 10 bis 110°C, bevorzugt 20 bis 80°C, zu einer Verbindung der Formel Ic umsetzt, oder
3. eine Verbindung der Formel VI 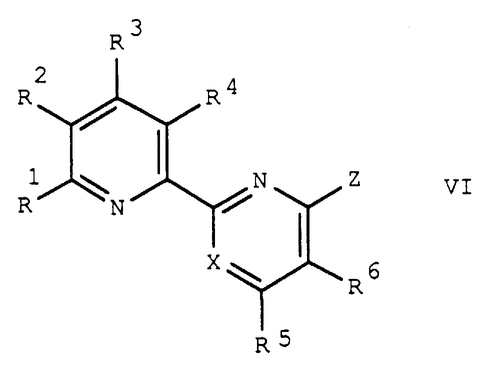 mit einer Verbindung der Formel IV b bzw. IV c, 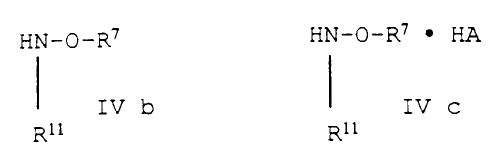 worin R¹ - R⁷, R¹¹ und X die Bedeutung wie in Formel I gemäß Anspruch 1 oder 2 besitzen, Z für Halogen, wie Fluor, Chlor, Brom und Jod, bevorzugt Chlor oder Brom, und HA für eine anorganische oder organische Säure, wie Salzsäure, Schwefelsäure oder Essigsäure stehen, in einem Lösungsmittel, wie Ethanol, Isopropanol, Acetonitril, Dichlormethan, 1,2-Dichlorethan, Toluol, Xylol, Tetrahydrofuran, Dioxan, Diethylenglykoldimethylether, Dimethylformamid oder N-Methylpyrrolidon, z.B. bei Temperaturen zwischen -10°C und der Siedetemperatur des Lösungsmittels, gegebenenfalls in Gegenwart einer Base, wie die Carbonate und Hydrogencarbonate von Alkali- und Erdalkalimetallen, Natriumhydrid, Alkalihydroxide oder -alkoholate, tertiäre Amine, Pyridin oder 4-Dimethylaminopyridin, umsetzt. Bei Verbindungen der Formel VI, in denen X = CH darstellt, kann es besonders vorteilhaft sein, zuerst die Halogengruppe durch eine besonders leicht zu verdrängende Abgangsgruppe, wie z.B. Methylsufinyl oder Methylsulfonyl, zu ersetzen und dann die Umsetzung mit einer Verbindung der Formel IV b bzw. IV c durchzuführen.

Die Verbindungen der Formel III sind allgemein bekannte Verbindungen der organischen Chemie.

Die Verbindungen der Formel IV a-c sind bekannt bzw. lassen sich auf bekannte Weise darstellen (z.B. Houben-Weyl, Methoden der Organischen Chemie, 4. Auflage, Bd. E 16a, S. 214ff [IV a], S. 271ff [IV b, c]).

Die Verbindungen der Formel VI sind bekannt bzw. können auf bekannte Weise dargestellt werden (z.B. EP 234104, EP 259139, EP 270362, EP 407899, J. Org. Chem. 32 (1967) 1591).

Die Verbindungen der Formel II und V, 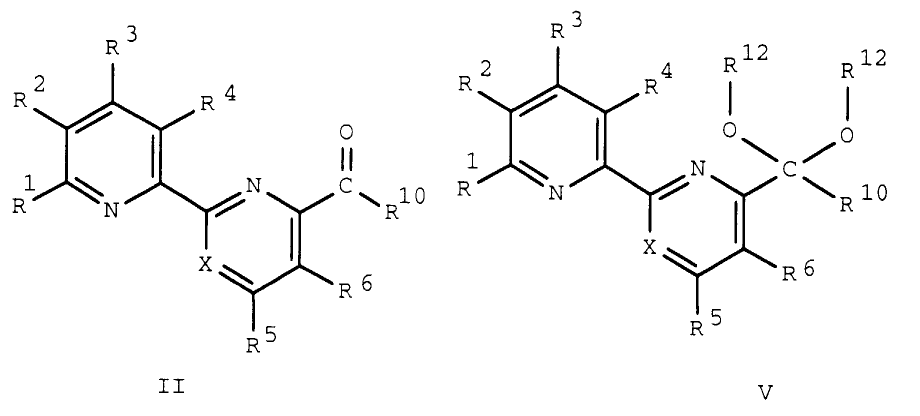 die bei der Herstellung der Verbindungen der Formel I c als Zwischenprodukte verwendet werden und worin
- R¹ =: Wasserstoff, C₁-C₆-Alkyl, C₁-C₄-Alkoxy-C₁-C₄-alkyl, C₁-C₄-Alkylthio-C₁-C₄-alkyl, C₂-C₆-Alkenyl, C₂-C₆-Alkinyl, C₃-C₇-Cycloalkyl, wobei der Cycloalkylrest bis zu dreifach durch C₁-C₄-Alkyl substituiert sein kann, Halogen, Phenyl, Phenoxy-C₁-C₄-alkyl, Phenylmercapto-C₁-C₄-alkyl, Phenyl-C₁-C₄-alkyl, Phenoxy, Phenylmercapto, wobei die sechs letztgenannten Reste im Phenylteil bis zu dreifach durch Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein können,
- R², R³, R⁴ =: unabhängig voneinander Wasserstoff, C₁-C₆-Alkyl, Phenyl, wobei der Phenylrest bis zu dreifach durch Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein kann,
- R⁵ =: Wasserstoff, C₁-C₆-Alkyl, C₃-C₇-Cycloalkyl, C₃-C₇-Cycloalkyl-C₁-C₄-alkyl, wobei die beiden letztgenannten Reste im Cycloalkylteil bis zu dreifach durch C₁-C₄-Alkyl substituiert sein können, C₁-C₄-Haloalkyl, C₁-C₄-Alkoxy-C₁-C₄-alkyl, C₁-C₄-Alkylthio-C₁-C₄-alkyl, C₂-C₆-Alkenyl, C₂-C₆-Alkinyl, Phenyl, Phenyl-C₁-C₄-alkyl, Phenoxy-C₁-C₄-alkyl, Phenylmercapto-C₁-C₄-alkyl, wobei die vier letztgenannten Reste im Phenylteil bis zu dreifach durch
Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein können,
- R⁶ =: Wasserstoff, C₁-C₄-Alkyl, C₁-C₄-Alkoxy, C₁-C₄-Alkoxycarbonyl, Halogen, Phenyl, wobei der Phenylrest bis zu dreifach durch Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein kann, oder
R⁵ und R⁶ bilden zusammen eine Polymethylenkette der Formel -(CH₂)ₘ- mit m = 3 oder 4,
X = CH oder N,
- R¹⁰ =: Wasserstoff, C₁-C₆-Alkyl,
- R¹² =: C₁-C₄-Alkyl oder die beiden Reste R¹² bilden zusammen eine Polymethylenkette der Formel -(CH₂)ₙ- mit n = 2 oder 3, bedeuten, sind neu, ausgenommen die Verbindungen 6-Formyl-2,2'-bipyridin, 6-Acetyl-2,2'-bipyridin, und stellen einen Teil der vorliegenden Erfindung dar.

Von den Verbindungen II und V sind als Zwischenprodukte für die Herstellung von Verbindungen der Formel I c solche bevorzugt, worin die Substituenten folgende Bedeutung besitzen:
- R¹=: Wasserstoff, C₁-C₆-Alkyl, C₁-C₃-Alkoxy-C₁-C₂-alkyl, Phenyl, Phenyl-C₁-C₂-alkyl, Phenoxy-C₁-C₂-alkyl, wobei die drei letztgenannten Reste im Phenylteil bis zu dreifach durch Halogen oder C₁-C₄-Alkyl substituiert sein können,
- R², R³ =: unabhängig voneinander Wasserstoff, C₁-C₃-Alkyl, Phenyl, wobei der Phenylrest bis zu dreifach durch Halogen oder C₁-C₄-Alkyl substituiert sein kann,
- R⁴ =: Wasserstoff,
- R⁵ =: Wasserstoff, C₁-C₆-Alkyl, C₃-C₆-Cycloalkyl, C₅-C₆-Cycloalkyl-C₁-C₃-alkyl, Phenyl, Phenyl-C₁-C₂-alkyl, wobei die beiden letztgenannten Reste im Phenylteil unsubstituiert oder bis zu dreifach durch Halogen, C₁-C₄-Alkyl oder C₁-C₄-Alkoxy substituiert sein können, C₁-C₃-Haloalkyl, C₁-C₄-Alkoxy, C₁-C₄-Alkylthio
- R⁶ =: Wasserstoff, C₁-C₄-Alkyl, Halogen, Phenyl, C₁-C₃-Alkoxy oder
R⁵ und R⁶ bilden zusammen eine Polymethylenkette der Formel -(CH₂)ₘ- mit m = 3 oder 4,
X = CH oder N,
- R¹⁰ =: Wasserstoff, C₁-C₄-Alkyl,
- R¹² =: C₁-C₄-Alkyl oder die beiden Reste R¹² bilden zusammen eine Polymethylenkette der Formel -(CH₂)ₙ- mit n = 2 oder 3.

Ein weiterer Gegenstand der vorliegenden Erfindung ist ein Verfahren zur Herstellung der Verbindungen II, dadurch gekennzeichnet, daß man
1. eine Verbindung der Formel V, 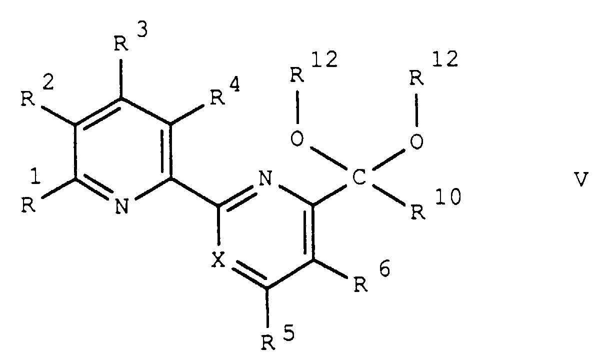 auf bekannte Weise (z. B. Houben-Weyl, Methoden der Organischen Chemie, 4. Auflage, Bd E 3, S. 362ff) in Gegenwart einer anorganischen oder organischen Säure, wie Salzsäure, Schwefelsäure, Ameisensäure oder Essigsäure, gegebenenfalls in Gegenwart eines Lösungsmittels, wie Wasser, Aceton, Methanol, Ethanol, Isopropanol, Dioxan, Tetrahydrofuran, Acetonitril oder Dimethylformamid, z.B. bei Temperaturen zwischen 20 und 100°C hydrolysiert, oder
2. eine Verbindung der Formel VII, 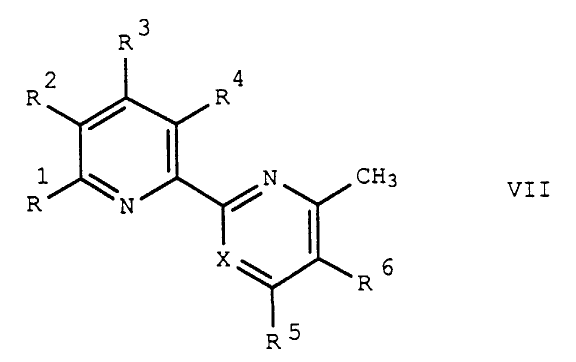 worin R¹ - R⁶ und X die Bedeutung wie in Formel I gemäß Anspruch 1 oder 2 besitzen, auf an sich bekannte Weise (z.B. Houben-Weyl, Methoden der Organischen Chemie, 4. Auflage, Bd E 3, S. 231ff) mit einem Oxidationsmittel, wie z.B. SeO₂ in Ethanol, tert.-Butanol, Dioxan, Pyridin oder Essigsäure z.B. bei Temperaturen zwischen 20°C und der Siedetemperatur des Lösungsmittels, zu einer Verbindung der Formel II a 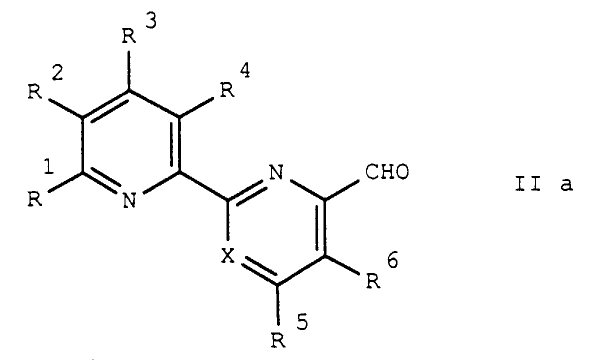 umsetzt, oder
3. eine Verbindung der Formel VI a, 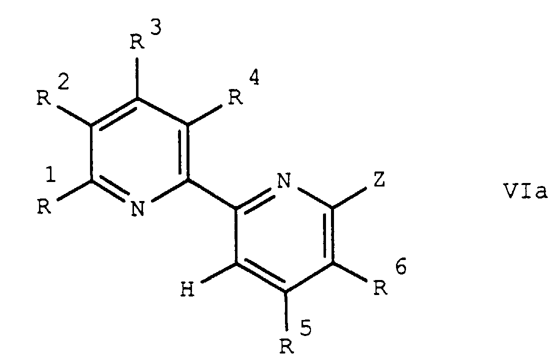 worin R¹ - R⁶ die Bedeutung wie in Formel I gemäß Anspruch 1 oder 2 besitzen und Z für Halogen, wie Fluor, Chlor, Brom und Jod, bevorzugt Brom oder Jod, steht, auf an sich bekannte Weise (z. B. Houben-Weyl, Methoden der Organischen Chemie, 4. Auflage, Bd. E 3, S. 115ff bzw. S. 130ff)
   a) mit einer Li-organischen Verbindung der Formel VIII,

      R¹³-Li VIII

      worin R¹³ = C₁-C₆-Alkyl, wie Methyl, Ethyl, Propyl, 1-Methylethyl, Butyl, 1-Methylpropyl, 2-Methylpropyl, 1,1-Dimethylethyl, Pentyl, Hexyl, insbesondere Butyl, 1-Methylpropyl und 1,1-Dimethylethyl, oder Phenyl darstellt, in einem Lösungsmittel, wie Diethylether, Tetrahydrofuran, Pentan oder Hexan, oder Gemischen der genannten Lösungsmittel bei Temperaturen von -90 bis 0°C, bevorzugt -80 bis -40°C, zu einer Verbindung der Formel IX a 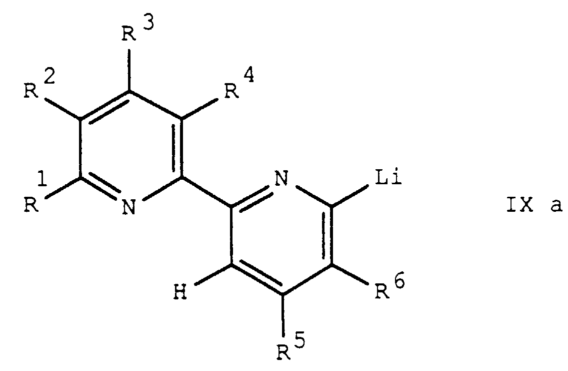 oder
   b) mit Mg
      in einem Lösungsmittel, wie Diethylether oder Tetrahydrofuran, bei Temperaturen von 0 bis 40°C zu einer Verbindung der Formel IX b 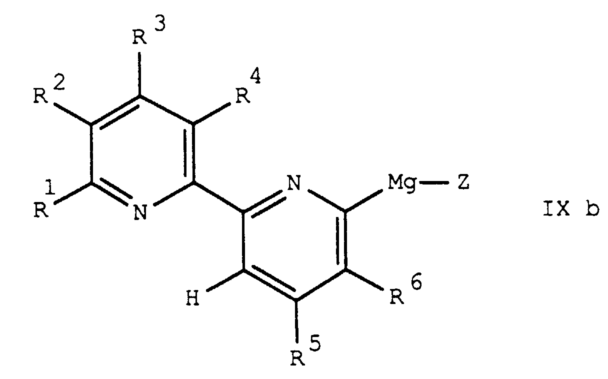 reagieren läßt, und die metallorganische Verbindung der Formel IX a bzw. IX b mit einer Verbindung der Formel X, 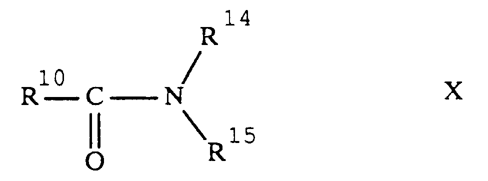 worin R¹⁰ die Bedeutung wie in Formel I gemäß Anspruch 1 oder 2 besitzt und R¹⁴, R¹⁵ = unabhängig voneinander C₁-C₄-Alkyl, wie Methyl, Ethyl, Propyl, 1-Methylethyl, Butyl, 1-Methylpropyl, 2-Methylpropyl und 1,1-Dimethylethyl, insbesondere Methyl, C₁-C₄-Alkoxy, wie Methoxy, Ethoxy, Propyloxy, 1-Methylethoxy, Butyloxy, 1-Methylpropyloxy, 2-Methylpropyloxy und 1,1-Dimethylethoxy, insbesondere Methoxy,
      oder C₅-C₇-Cycloalkyl, wie Cyclopentyl, Cyclohexyl, Cycloheptyl, darstellen oder R¹⁴ und
      R¹⁵ zusammen mit dem Stickstoffatom, an das sie gebunden sind, einen 5- bis 7-gliedrigen Heterocyclus mit 1 oder 2 gleichen oder verschiedenen Heteroatomen, vorzugsweise mit den Heteroatomen Stickstoff und/oder Sauerstoff, bilden, insbesondere Piperidin oder Morpholin, umsetzt.

Verbindungen der Formel VII sind bekannt bzw. lassen sich auf bekannte Weise darstellen (z.B. EP 234104, US 4,927,827, Tetrahedron Lett. 31 (1990) 4625).

Verbindungen der Formel VI a sind bekannt bzw. lassen sich auf bekannte Weise darstellen (z.B. J. Organomet. Chem. 56 (1973) 53, Tetrahedron Lett. 31 (1990) 4625).

Verbindungen der Formel VIII sind bekannt und leicht zugänglich (z.B. Houben-Weyl, Methoden der organische Chemie, 4. Auflage. Bd. 13, 1, S. 87ff).

Verbindungen der Formel X sind allgemein bekannte Verbindungen der organischen Chemie.

Ein weiterer Gegenstand der vorliegenden Erfindung ist ein Verfahren zur Herstellung der Verbindung V a, 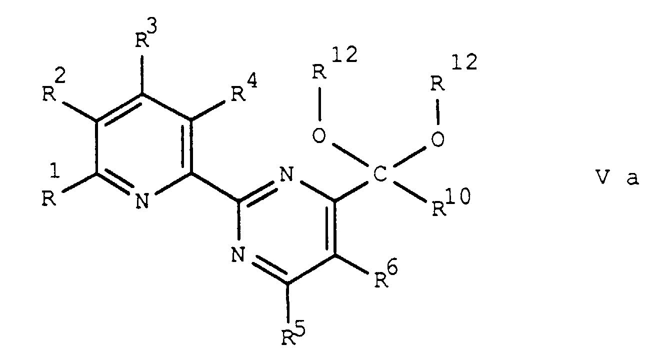 worin R¹ - R⁶, R¹⁰ und R¹² die Bedeutung wie in Formel V gemäß Anspruch 9 oder 10 besitzen, dadurch gekennzeichnet, daß man ein Picolinamidin-Derivat der Formel XI a bzw. XI b, 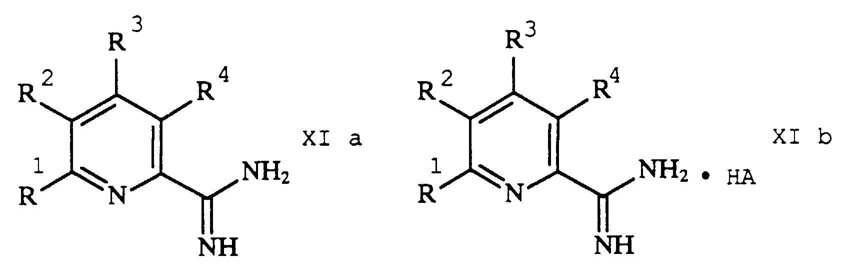 worin R¹ - R⁴ die Bedeutung wie in Formel I gemäß Anspruch 1 oder 2 besitzen und HA für eine anorganische oder organische Säure, wie Salzsäure, Schwefelsäure oder Essigsäure, steht, in einem polaren Lösungsmittel, wie Wasser, Methanol, Ethanol, tert.-Butanol, Acetonitril oder Dimethylformamid, gegebenenfalls in Gegenwart einer Base, wie Natriummethanolat, -ethanolat, Triethylamin oder die Carbonate und Hydrogencarbonate der Alkali- und Erdalkalimetalle, z.B. bei Temperaturen zwischen -10°C und der Siedetemperatur des Lösungsmittels
1) mit einer Dicarbonylverbindung der Formel XII, 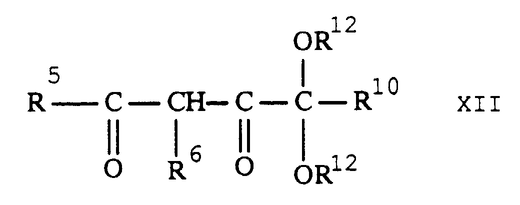 oder
2) mit einem Keton der Formel XIII, 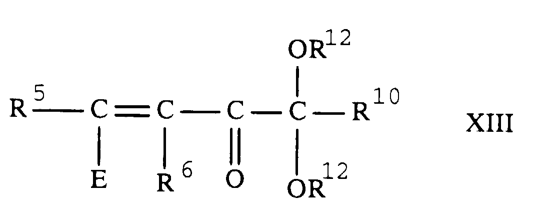 worin R⁶ und R¹⁰ die Bedeutung wie in Formel I gemäß Anspruch 1, R⁵ die Bedeutung wie in Formel I gemäß Anspruch 1, und R¹² die Bedeutung wie in Formel V gemäß Anspruch 9 oder 10 besitzen, und E für OR¹⁶ oder NR¹⁷R¹⁸ steht, mit R¹⁶ = C₁-C₄-Alkyl, wie Methyl, Ethyl, Propyl, 1-Methylethyl, Butyl, 1-Methylpropyl, 2-Methylpropyl, 1,1-Dimethylethyl, insbesondere Methyl, und R¹⁷, R¹⁸ = unabhängig voneinander Wasserstoff, C₁-C₄-Alkyl, wie Methyl, Ethyl, Propyl, 1-Methylethyl, Butyl, 1-Methylpropyloxy, 2-Methylpropyl, 1,1-Dimethylethyl, insbesondere Methyl, oder R¹⁷ und R¹⁸ bilden zusammen mit dem Stickstoffatom, an das sie gebunden sind, einen 5- bis 7-gliedrigen Heterocyclus mit 1 oder 2 gleichen oder verschiedenen Heteroatomen, vorzugsweise mit den Heteroatomen Stickstoff und/oder Sauerstoff, insbesondere Pyrrolidin, Piperidin oder Morpholin, umsetzt.

Die Picolinamidin-Derivate der Formel XI a bzw. XI b sind bekannt bzw. können in Analogie zu bekannten Verfahren hergestellt werden (z. B. EP 259139, EP 270362).

Die Dicarbonylverbindungen der Formel XII und die Ketone der Formel XIII sind bekannt bzw. lassen sich auf bekannte Weise darstellen (z. B. Chem. Ber. 97 (1964) 3407, GB 2095240).

Die Verbindungen der Formel I und ihre Salze und Metallkomplexe eignen sich als Fungizide.

### Herstellungsbeispiele

### Beispiel 1

### 4-Formyloxim-2-(2-pyridyl)pyrimidin (Verbindung Ic)

Zu einer Lösung von 2 g (10,8 mmol) 4-Formyl-2-(2-pyridyl)-pyrimidin in 50 ml Methanol werden 0,75 g (10,8 mmol) Hydroxylammoniumchlorid und 1,82 g (21,6 mmol) Natriumhydrogencarbonat zugefügt. Nach 12stündigem Rühren bei Raumtemperatur (20°C) versetzt man den Reaktionsansatz mit 100 ml ges. NH₄Cl-Lsg., extrahiert dreimal mit je 50 ml Dichlormethan und trocknet die vereinigten organischen Phasen über Natriumsulfat. Nach dem Abdestillieren des Lösungsmittels im Vakuum wird ein organgefarbener Festkörper erhalten. Ausbeute: 46 % d. Theorie, Fp = 190°C.

Verwendet man anstelle von 4-Formyl-2-(2-pyridyl)pyrimidin 6-Formyl-4-methyl-2-(6-methyl-2-pyridyl)pyrimidin und anstelle von Hydroxylammoniumchlorid O-n-Butylhydroxylammoniumchlorid, so erhält man in entsprechender Weise 6-Formyl-O-n-butyloxim-4-methyl-2-(6-methyl-2-pyridyl)pyrimidin (Verbindung 133c).
Ausbeute: 50 % d. Theorie eines gelblichen Öls nach Chromatographie an Aluminiumoxid (n-Hexan/Methyl-tert.-butylether).
IR: 2959, 1577, 1535 1365, 1046 cm⁻¹.

Verwendet man anstelle von 4-Formyl-2-(2-pyridyl)pyrimidin 6-Formyl-2,2'-bipyridin und anstelle von Hydroxylammoniumchlorid O-Phenylbutylhydroxylammoniumchlorid, so erhält man in entsprechender Weise 6-Formyl-O-4-phenylbutyloxim-2,2'-bipyridin -2,2'-bipyridin (Verbindung 105c).
Ausbeute: 76 % d. Theorie, Fp = 76 - 78°C.

In entsprechender Weise können die in Tabelle 1 aufgelisteten Verbindungen hergestellt werden.

### Beispiel 2

### 5-Chlor-4-O-i-butylhydroxylamino-6-methyl-2-(6-methyl-2-pyridyl)pyrimidin (Verbindung 66 d).

Zu einer Suspension von 1,38 g (11 mmol) O-i-Butylhydroxylammoniumchlorid in 25 ml Ethanol wird eine Mischung von 1,11 g (11 mmol) Triethylamin und 25 ml Ethanol getropft. Nach 15minütigem Rühren bei Raumtemperatur fügt man 2,54 g (10 mmol) 4,5-Dichlor-6-methyl-2-(6-methyl-2-pyridyl)pyrimidin zu und erhitzt dann den Reaktionsansatz 6 Stunden unter Rückfluß. Man destilliert das Lösungsmittel im Vakuum ab und reinigt den Rückstand durch Chromatographie an Aluminiumoxid (n-Hexan/Methyltert.-butylether).
Die Titelverbindung wird in Form orangefarbener Kristalle erhalten.
Ausbeute: 65 % d. Theorie, Fp = 108 - 110°C.

In entsprechender Weise können die in Tabelle 2 aufgelisteten Verbindungen hergestellt werden.

### Herstellung der Zwischenstufen

### Beispiel 3

### 4-Formyldimethylacetal-2-(2-pyridyl)pyrimidin (Verbindung 1.3)

Zu einer Lösung von Natriummethanolat in Methanol - bereitet aus 7 g (0,3 mol) Natrium und 200 ml Methanol - fügt man eine Lösung von 40g (0,25 mol) 2-Pyridinylamidin-hydrochlorid in 100 ml Methanol zu und rührt 10 Minuten bei Raumtemperatur. Anschließend tropft man eine Lösung von 43,9 g (0,25 mol) (β-Dimethylamino-vinyl)glyoxal-dimethylacetal in 100 ml Methanol zu und erhitzt 7 Stunden unter Rückfluß. Danach wird der Reaktionsansatz im Vakuum eingeengt und mit Wasser/Essigsäure neutralisiert.
Dreimalige Extraktion mit je 150 ml Dichlormethan, anschließendes Waschen mit Wasser (3 x je 50 ml), Trocknen über Natriumsulfat und Abdestillieren des Lösungsmittels im Vakuum liefern die Titelverbindung in Form bräunlicher Kristalle.
Ausbeute: 47 % d. Theorie, Fp = 42°C.

Verwendet man anstelle von 2-Pyridinylamidin-hydrochlorid 6-Methyl-2-pyridinylamidin-hydrochlorid und anstelle von (β-Dimethylamino-vinyl)glyoxal-dimethylacetal Diethoxyacetylaceton, so erhält man in entsprechender Weise 4-Formyldiethylacetal-6-methyl-2-(6-methyl-2-pyridyl)pyrimidin (Verbindung 4.3).
Ausbeute: 27 % d. Theorie eines gelben Öls, das beim Stehen langsam kristallisiert.
IR: 2975, 1581, 1372, 1112, 1062 cm⁻¹.

In entsprechender Weise können die in Tabelle 3 aufgelisteten Verbindungen hergestellt werden.

### Beispiel 4

### 4-Formyl-2-(2-pyridyl)pyrimidin (Verbindung 1.4)

Eine Lösung von 10 g (43,3 mmol) 4-Formyldimethylacetal-2-(2-pyridyl)pyrimidin in 43,3 ml 1 Salzsäure werden 2 Stunden zum Sieden erhitzt. Nach dem Abkühlen wird mittels Na₂CO₃-Lösung neutralisiert und dreimal mit je 50 ml Dichlormethan extrahiert. Die vereinigten organischen Phasen werden über Natriumsulfat getrocknet und das Lösungsmittel wird im Vakuum abdestilliert, woraufhin die Titelverbindung in Form bräunlicher Kristalle zurückbleibt.
Ausbeute: 62 % d. Theorie, Fp = 110 - 112°C.

Verwendet man anstelle von 4-Formyldimethylacetal-2-(2-pyridyl)-pyrimidin 4-Formyldiethylacetal-6-methyl-2-(6-methyl-2-pyridyl)-pyrimidin, so erhält man in entsprechender Weise 4-Formyl-6-methyl-2-(6-methyl-2-pyridyl)pyrimidin (Verbindung 3.4).
Ausbeute: 69 % d. Theorie eines bräunlichen Harzes.

In entsprechender Weise können die in Tabelle 4 aufgelisteten Verbindungen hergestellt werden.

### Beispiel 5

### 6-Formyl-2,2'-bipyridin (Verbindung bekannt aus Aust. J. Chem. 44 (1991) 331)

Zu einer Lösung von 19,4 ml (31 mmol) 1,6 M Butyllithium-in-Hexan-Lösung in 100 ml Diethylether wird bei -80°C unter Stickstoffatmosphäre eine Lösung von 7,05 g (30 mmol) 6-Brom-2,2'-bipyridin in 50 ml Diethylether zugefügt. Nach erfolgter Zugabe rührt man noch 30 Minuten bei dieser Temperatur und fügt dann bei -80 bis -90°C eine Lösung von 4,39 g (60 mmol) Dimethylformamid in 30 ml Diethylether zu. Nach weiterem Rühren bei dieser Temperatur für 1,5 Stunden wird bei -60 bis -50°C mit 150ml viertelkonzentrierter Salzsäure hydrolysiert. Der Reaktionsansatz wird danach bei Raumtemperatur durch Zugabe von festem Natriumhydrogencarbonat neutralisiert, die Phasen werden getrennt und die wässrige Phase wird noch einmal mit 50 ml Diethylether extrahiert.
Die organischen Phasen werden vereinigt, über Natriumsulfat getrocknet und schließlich wird das Lösungsmittel im Vakuum abdestilliert. Die Titelverbindung wird als rotbraunes Öl erhalten, das beim Stehen kristallisiert.
Ausbeute: 96 % d. Theorie, Fp = 41 - 43°C.

In entsprechender Weise können die in Tabelle 4 aufgelisteten Verbindungen hergestellt werden.

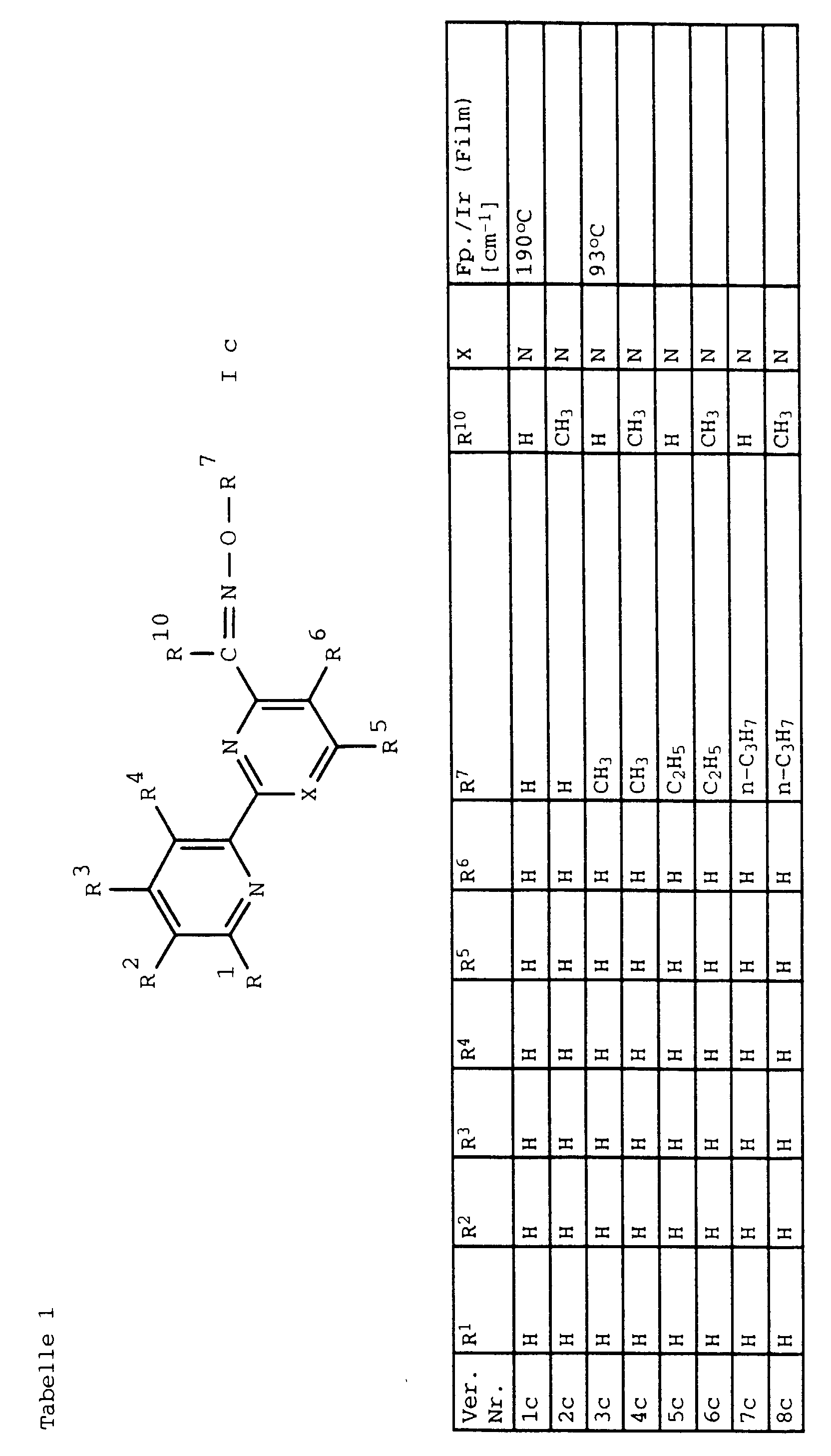

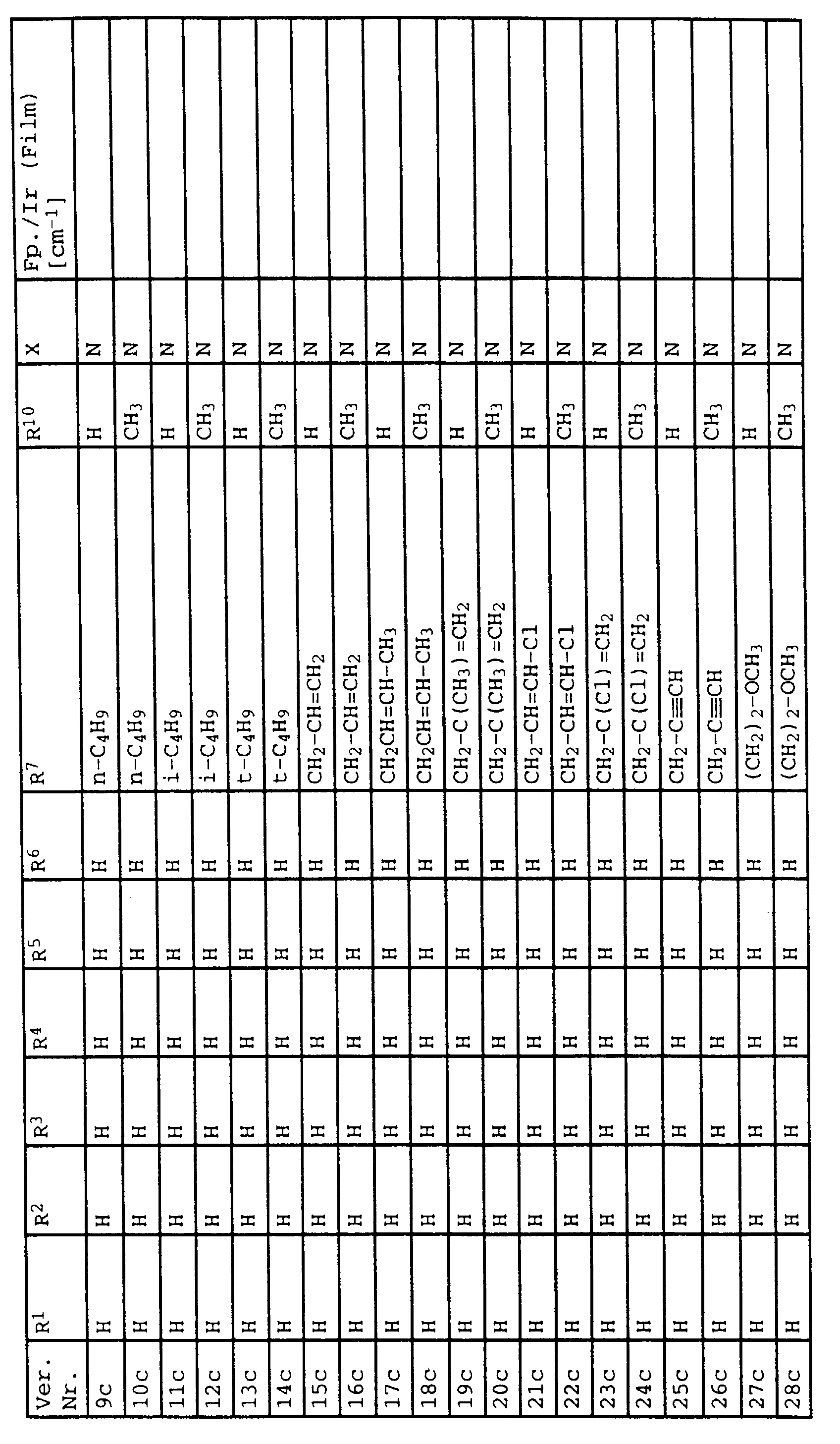

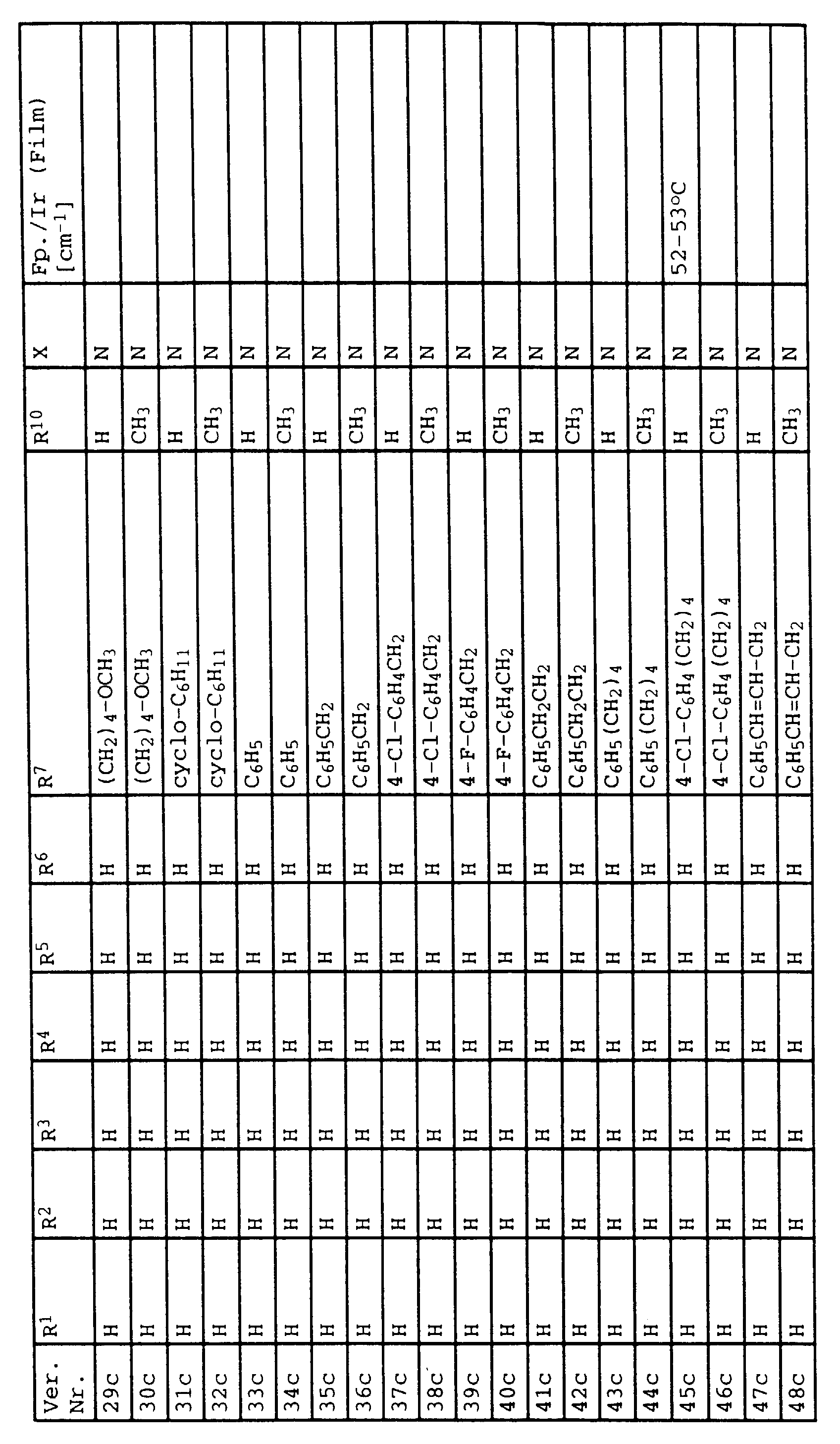

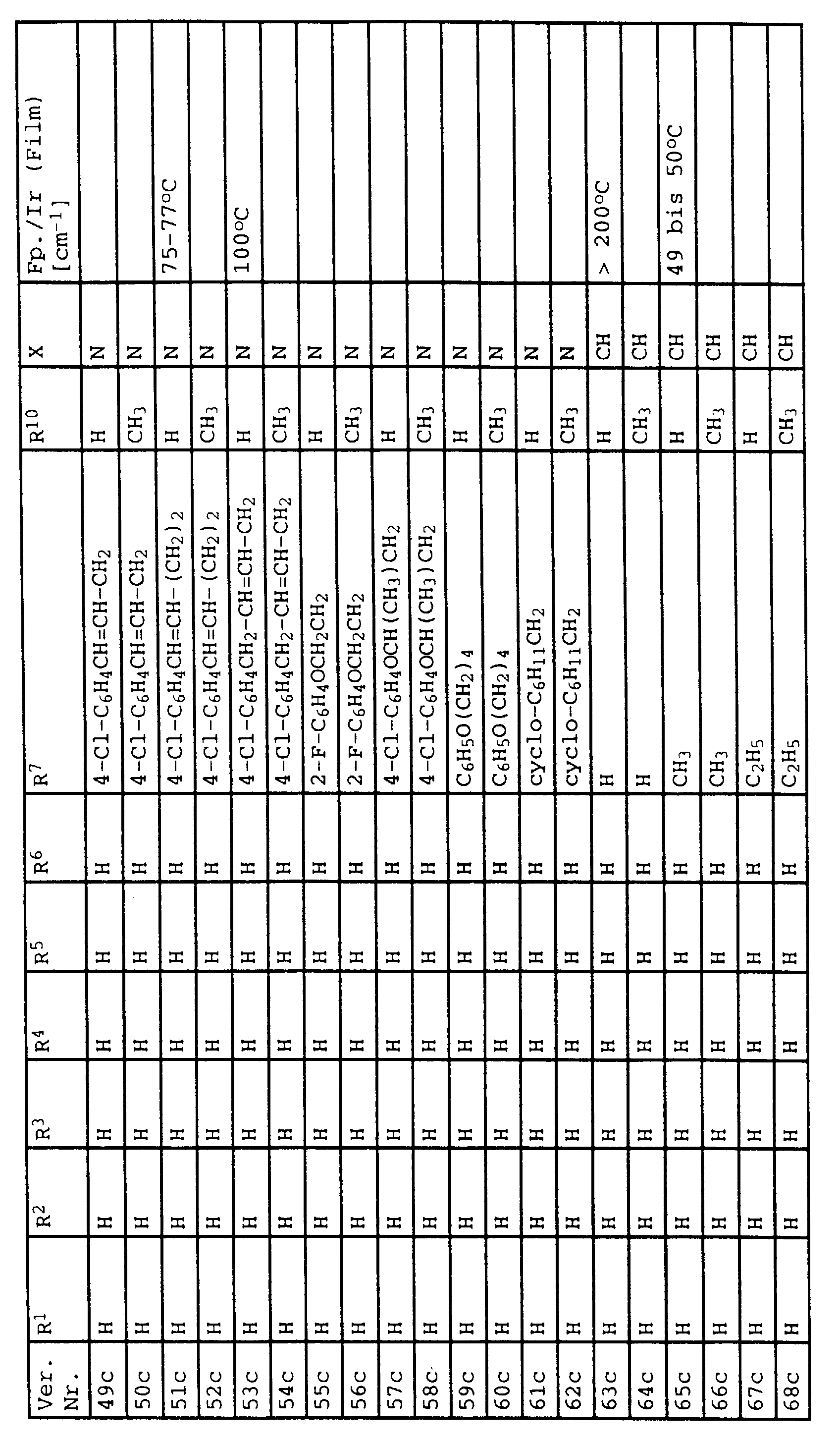

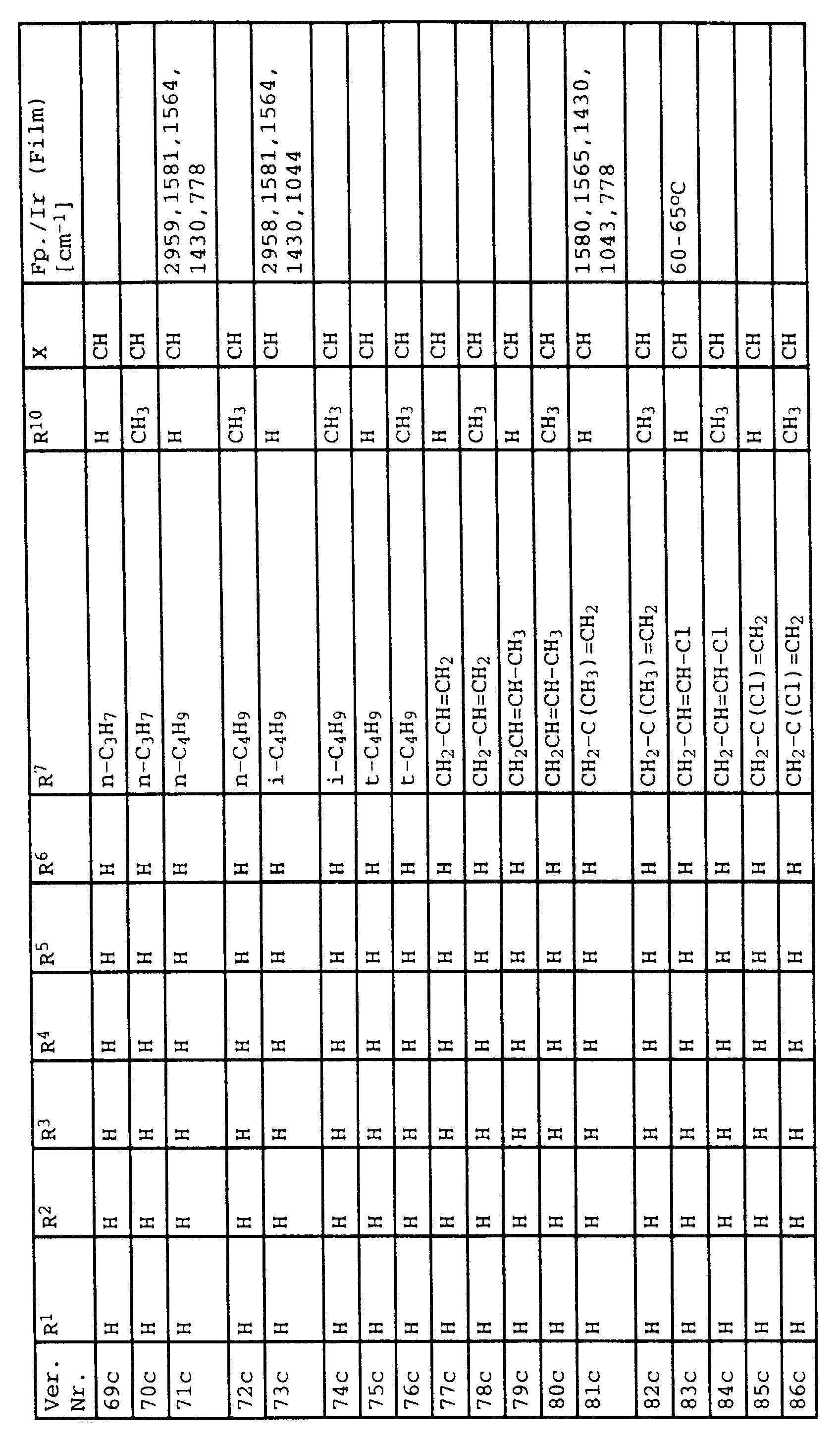

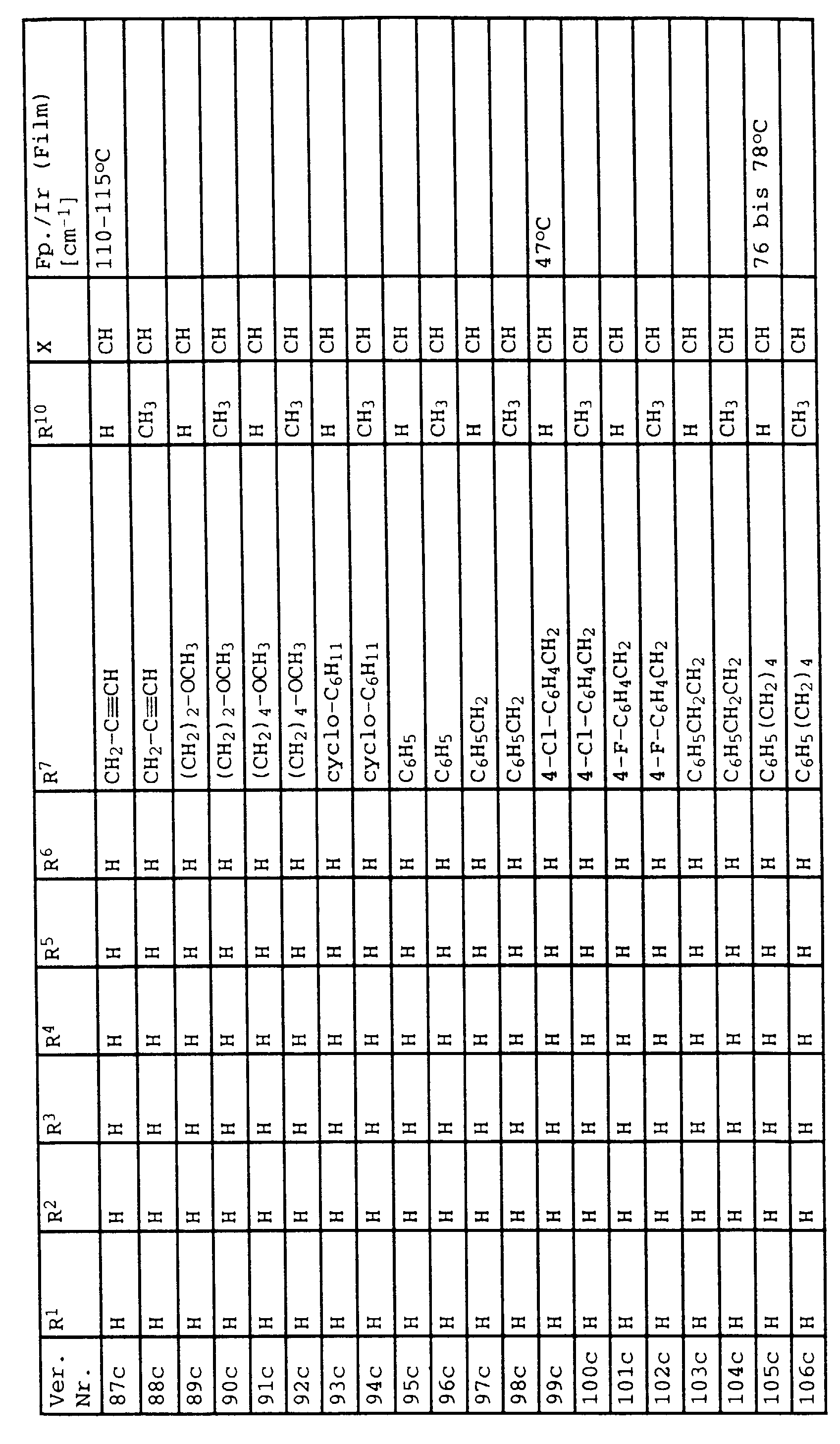

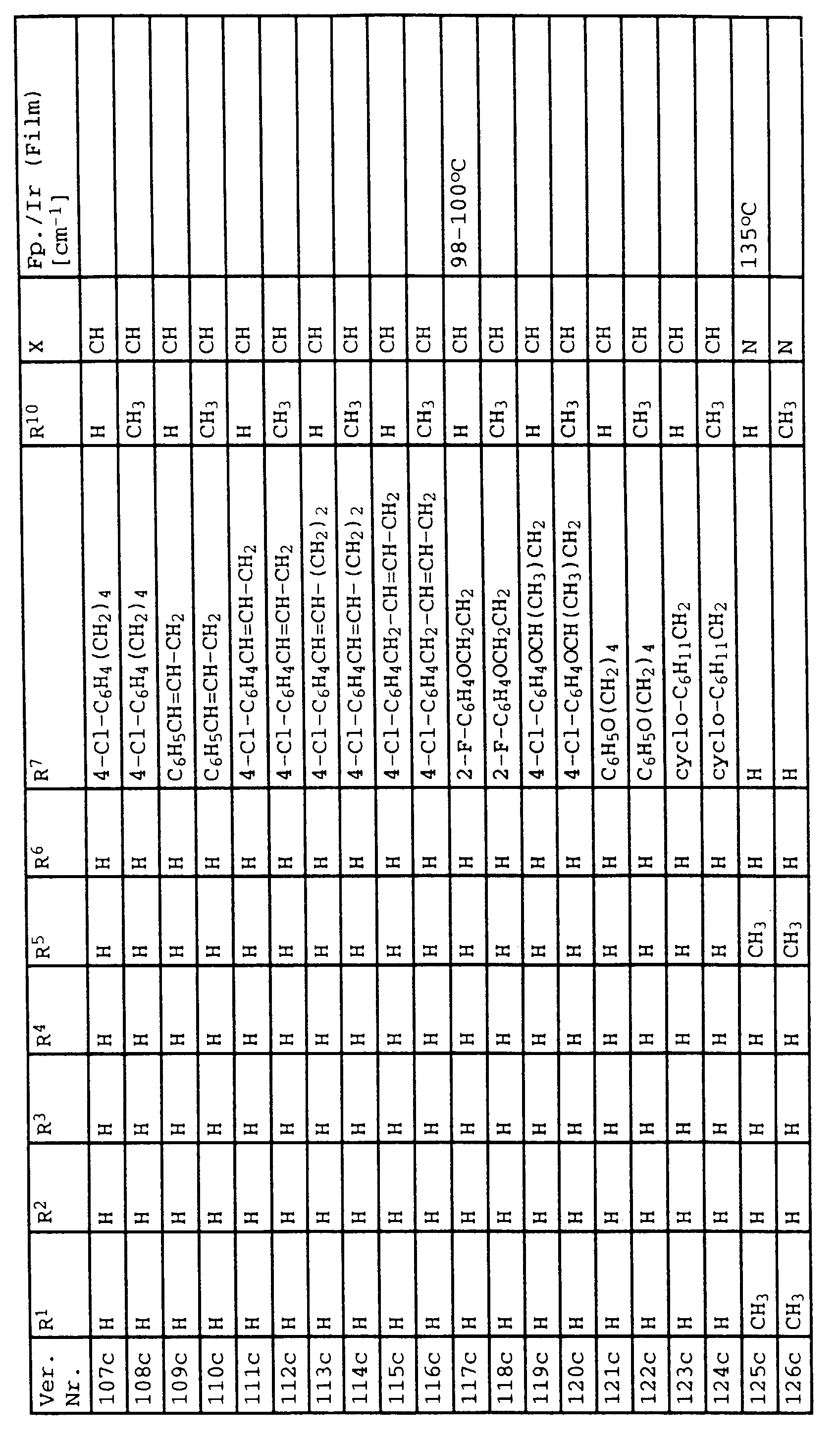

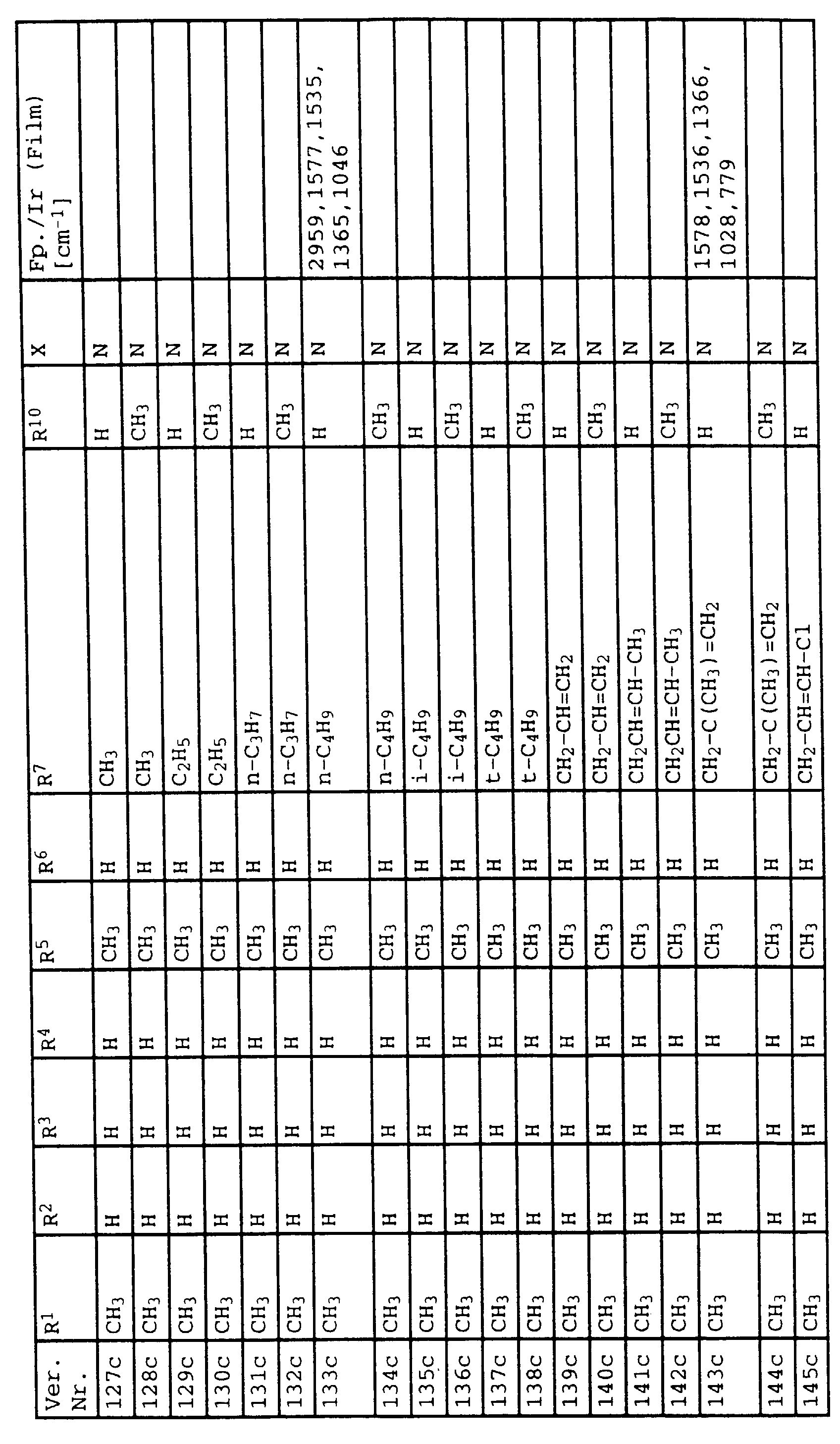

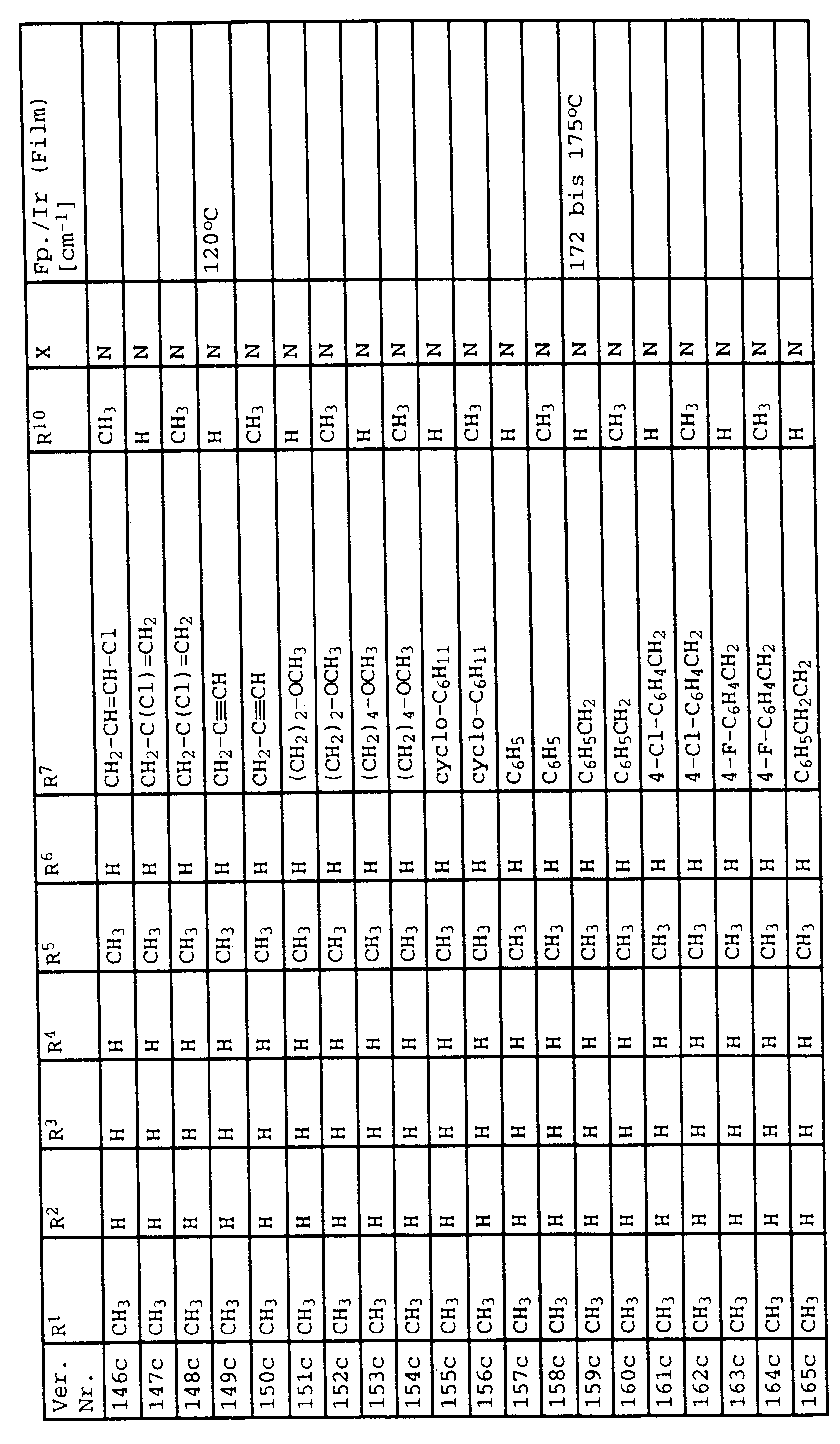

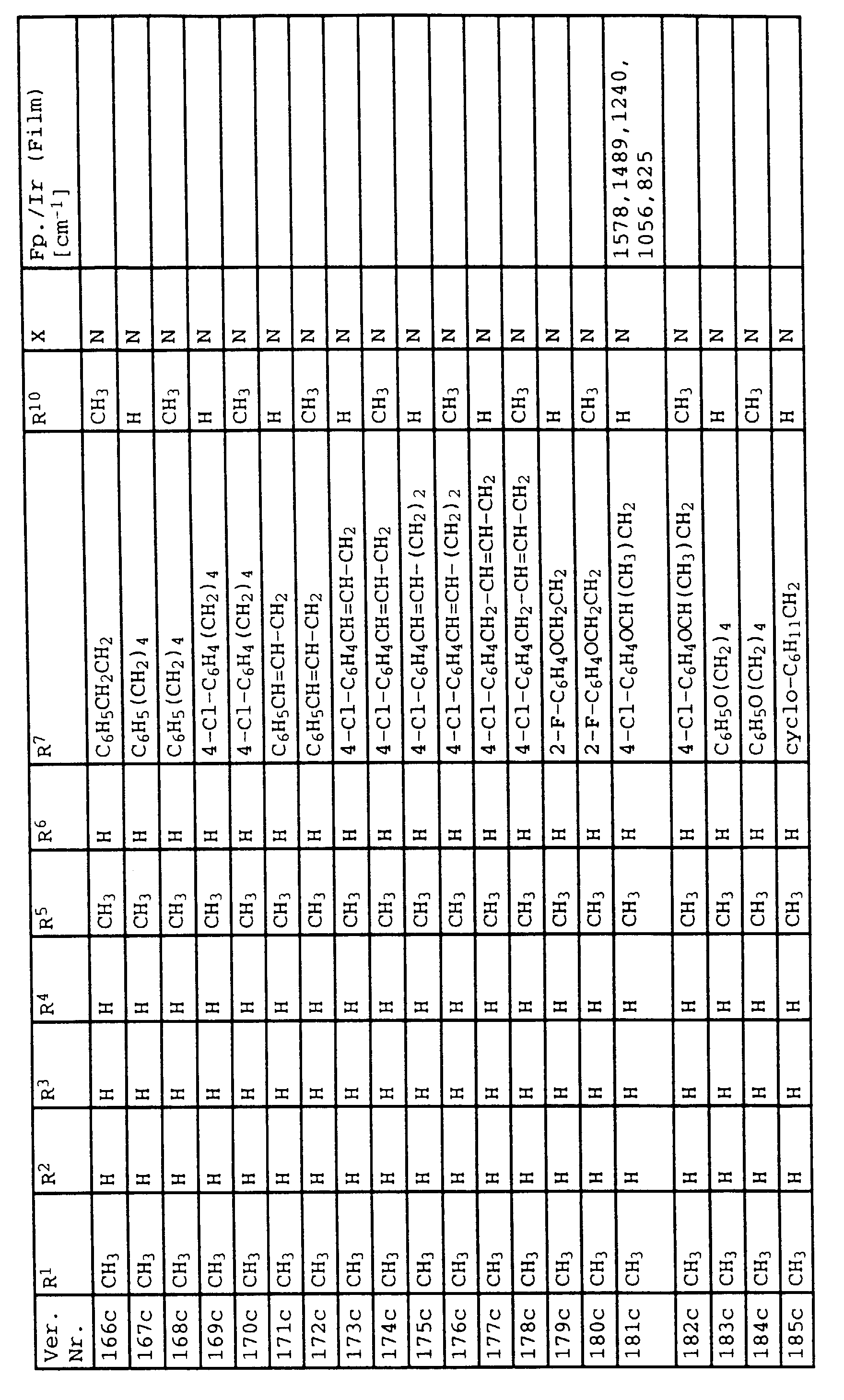

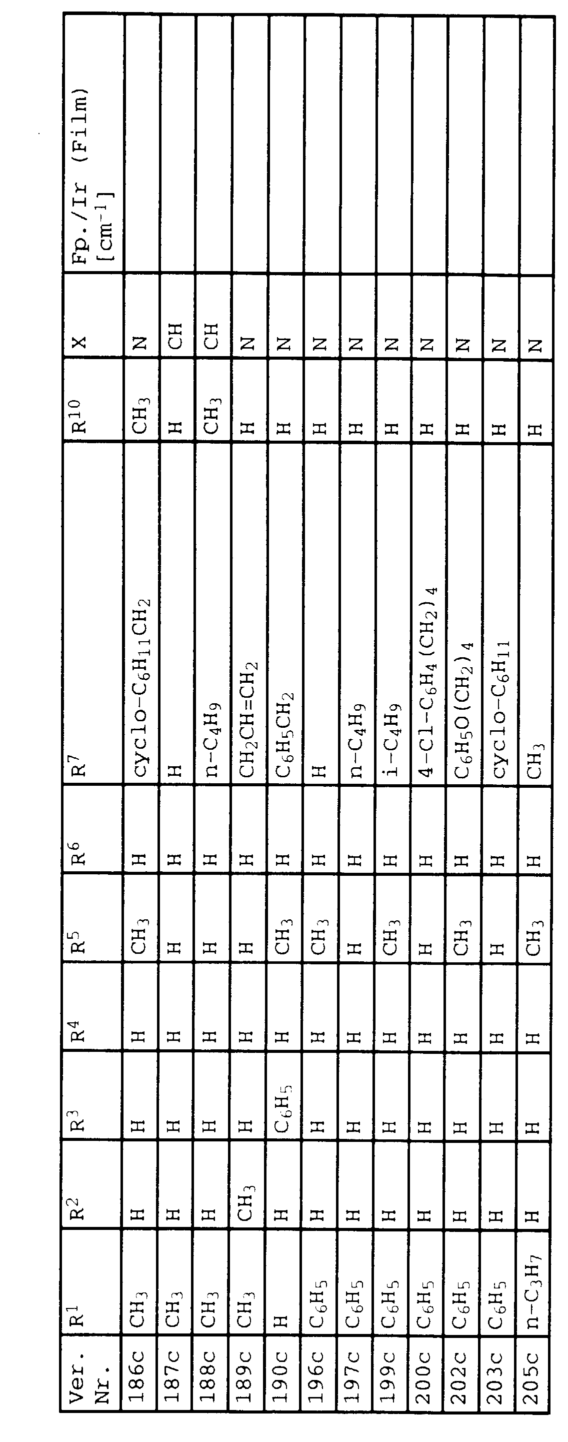

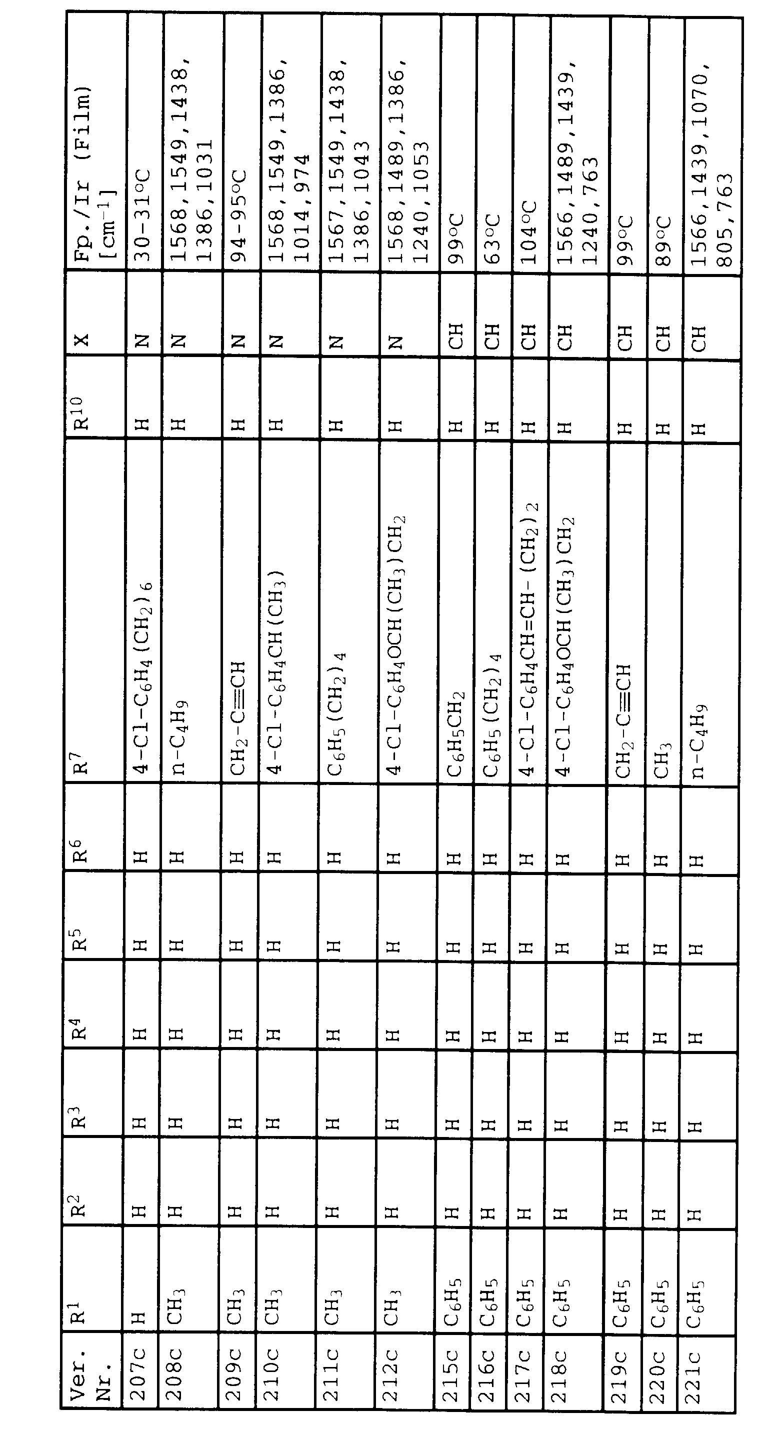

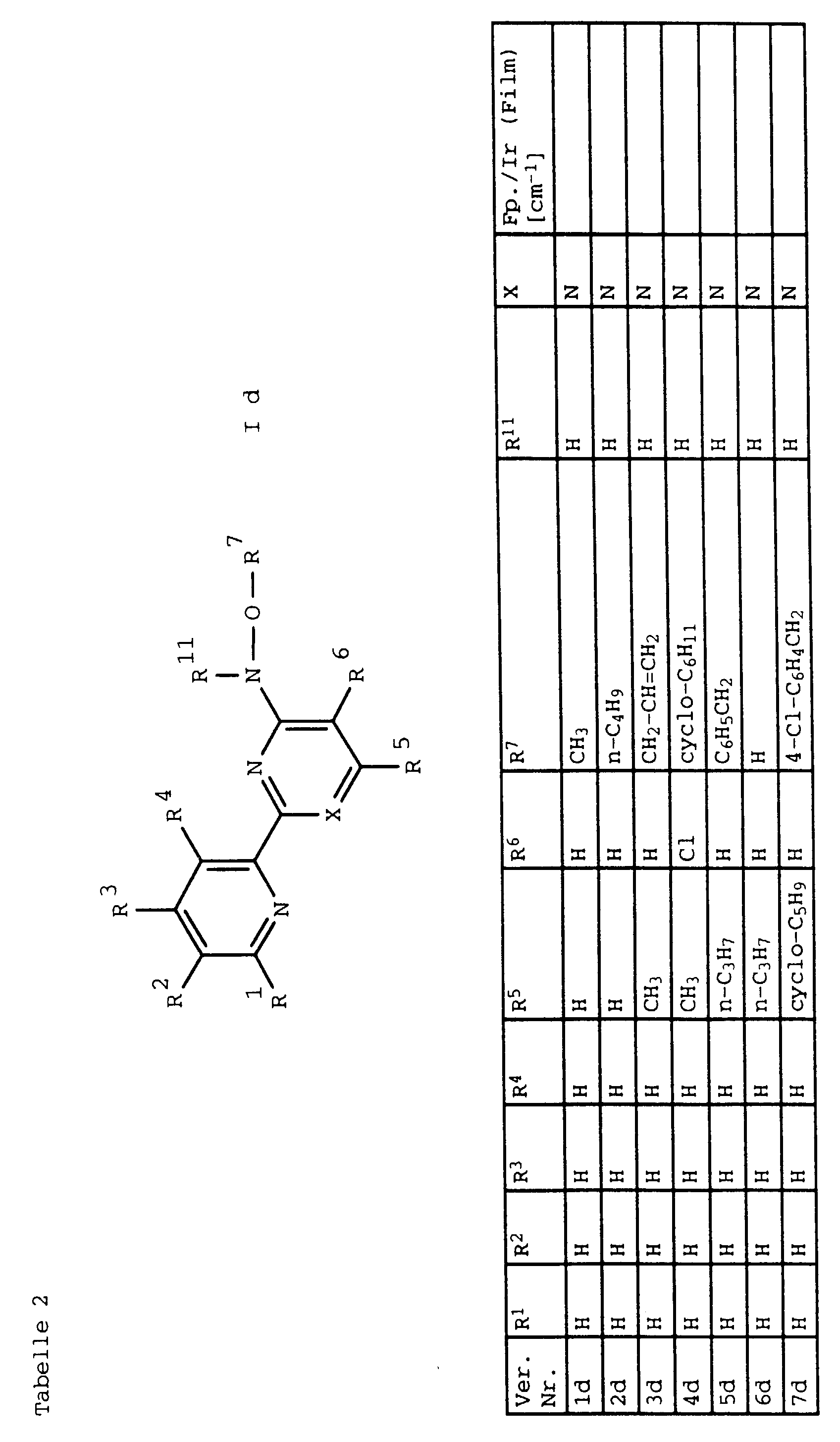

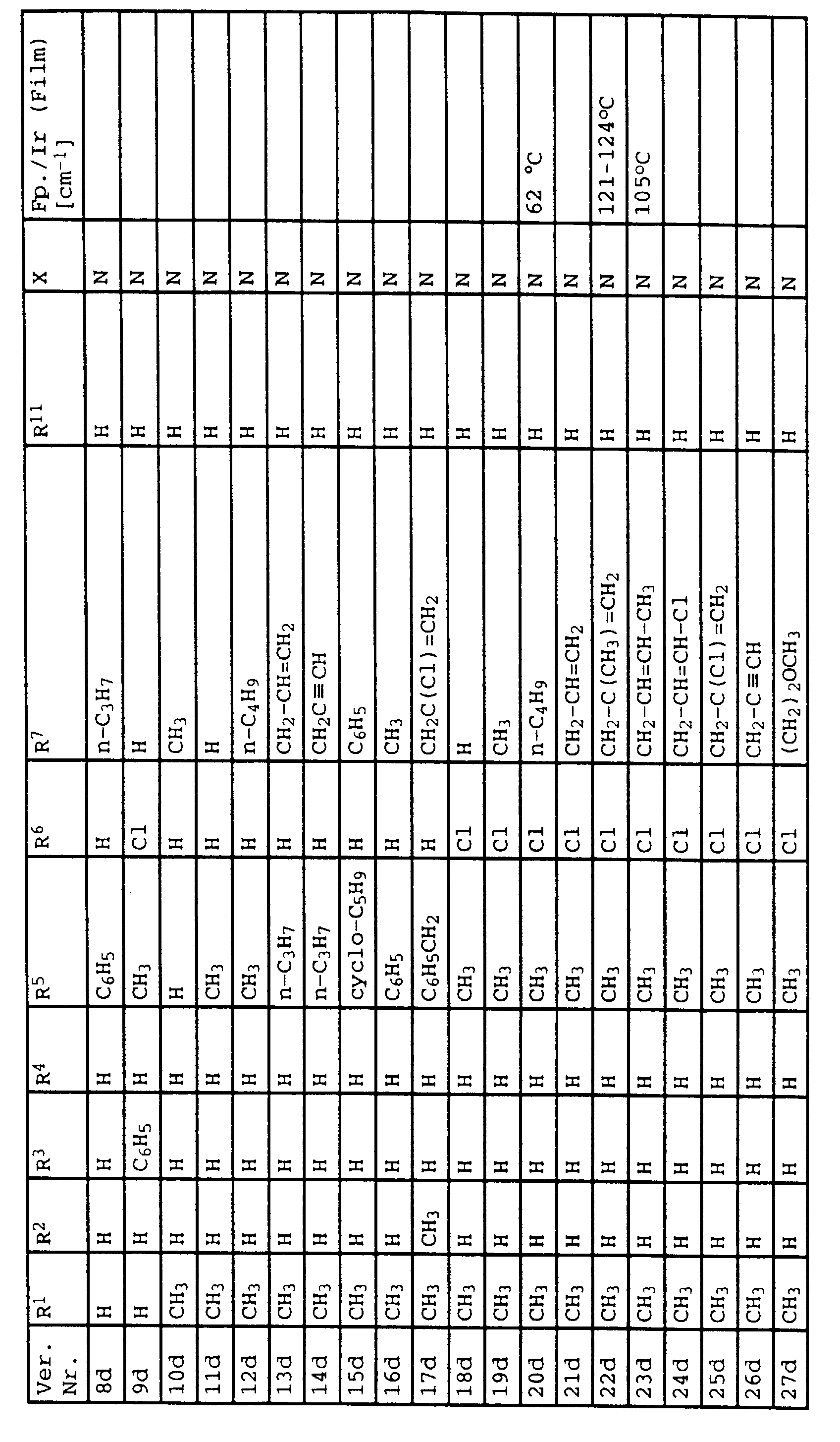

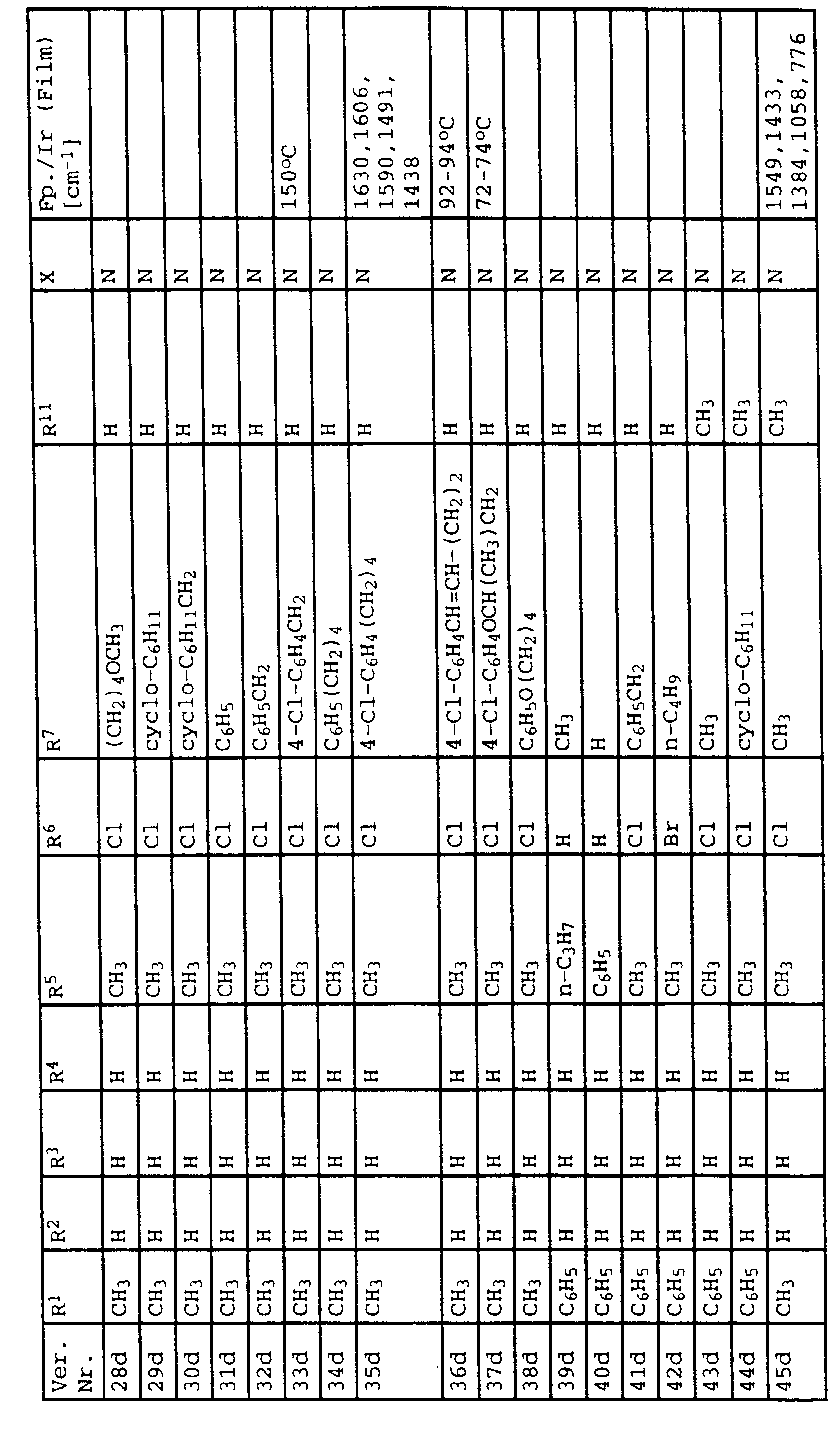

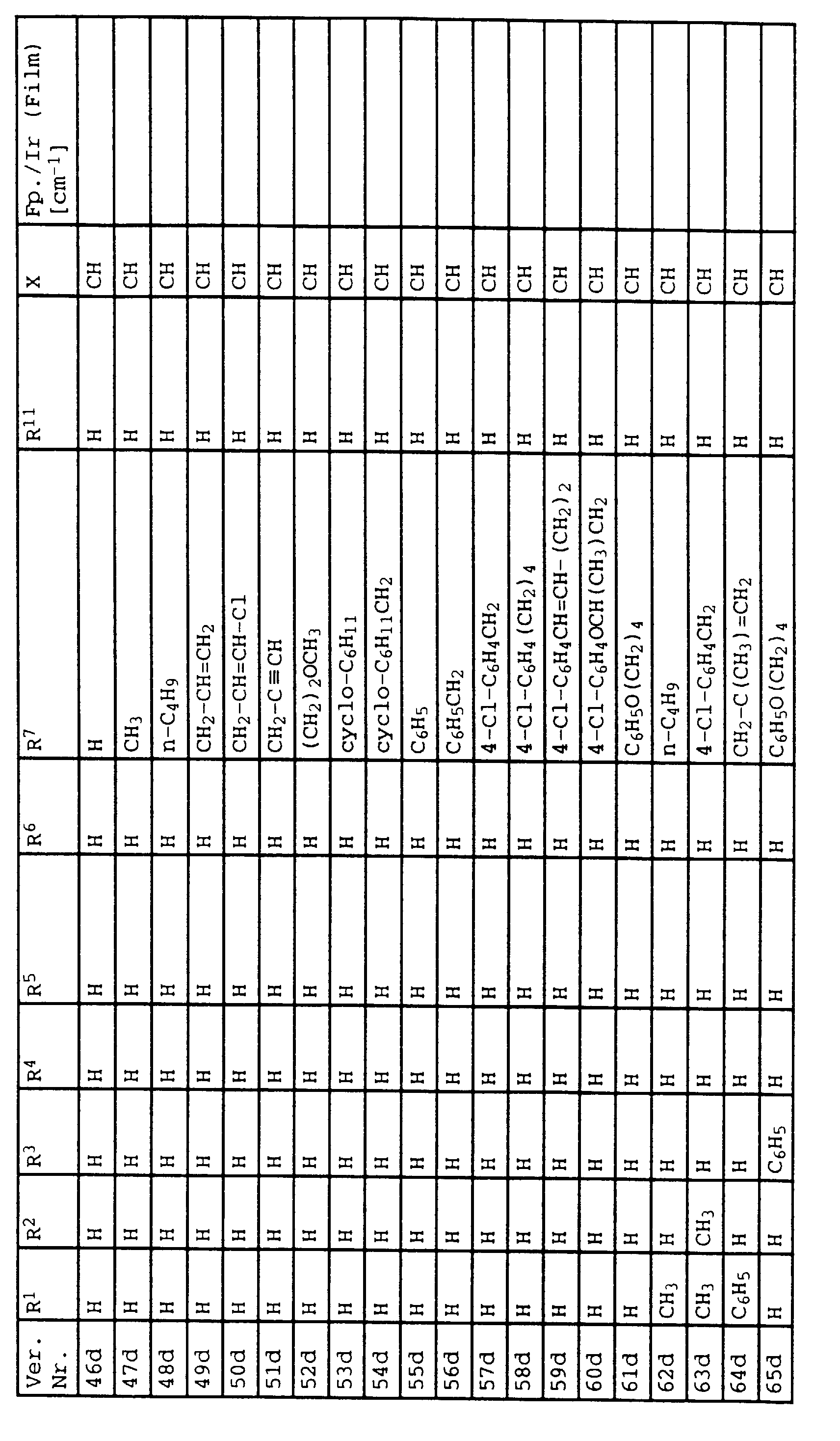

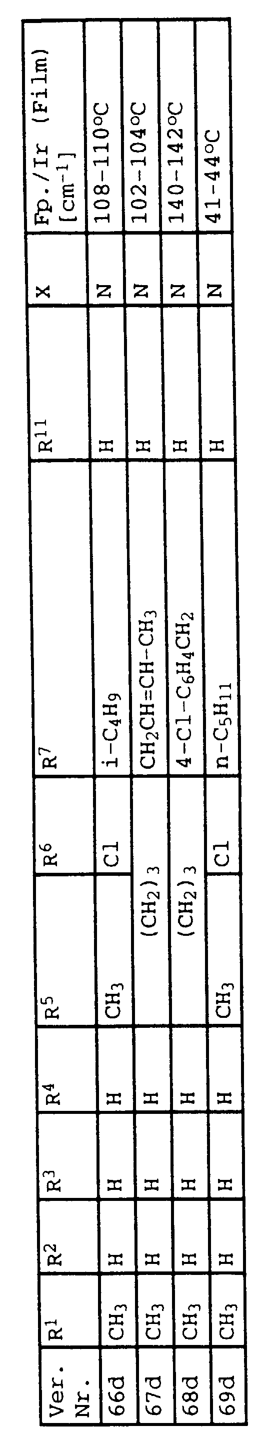

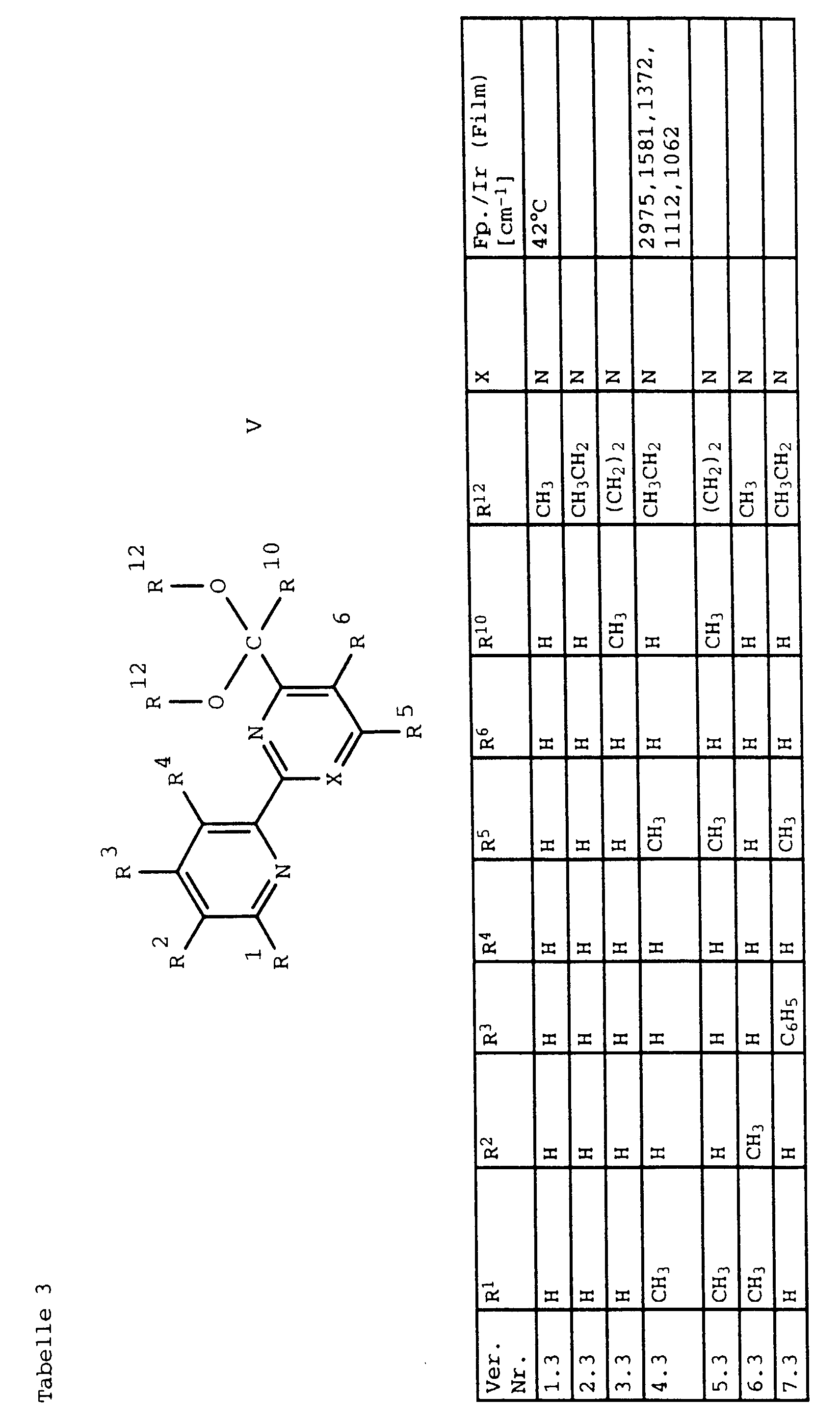

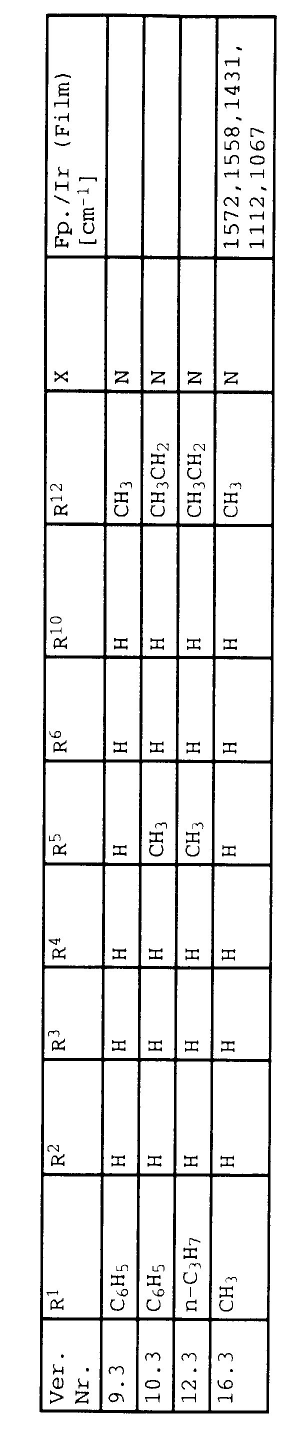

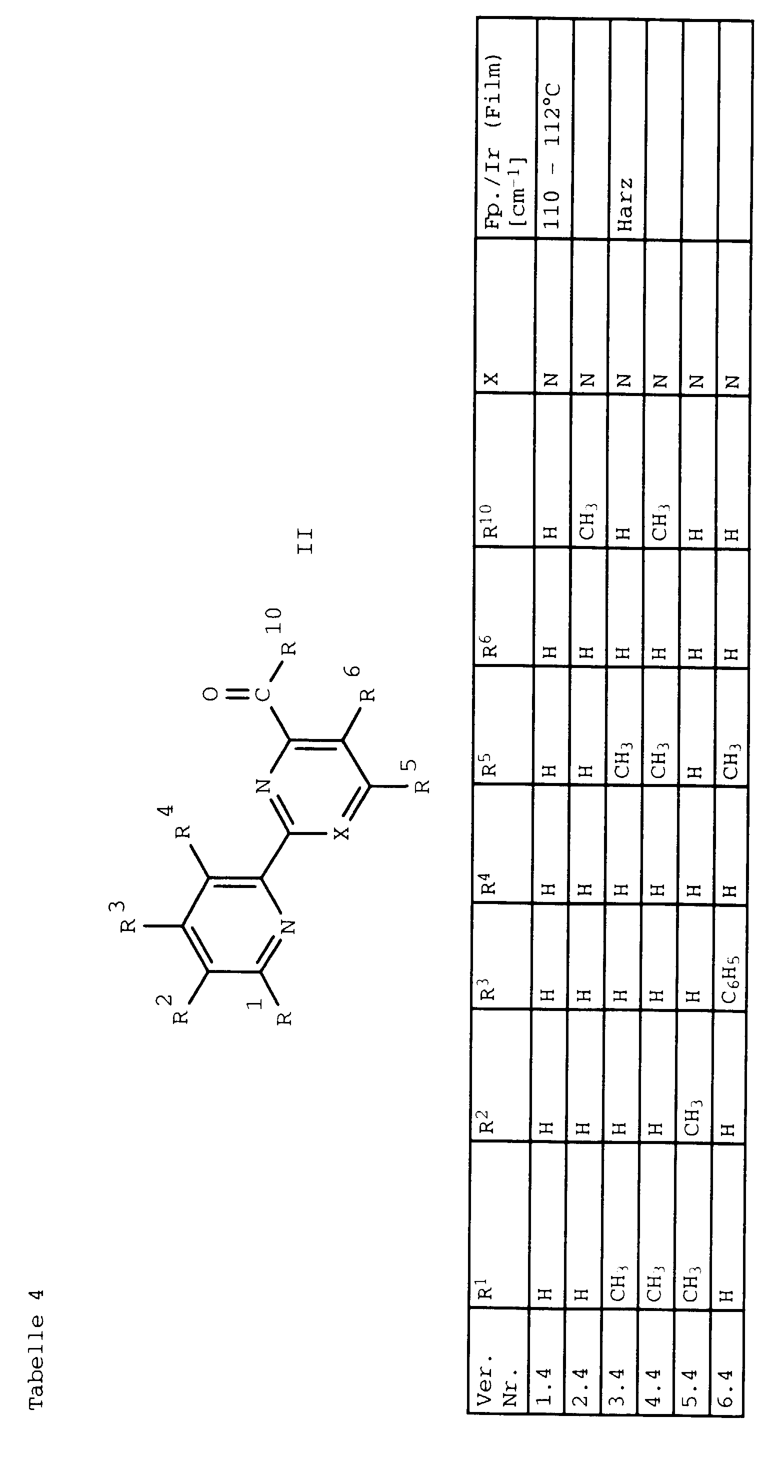

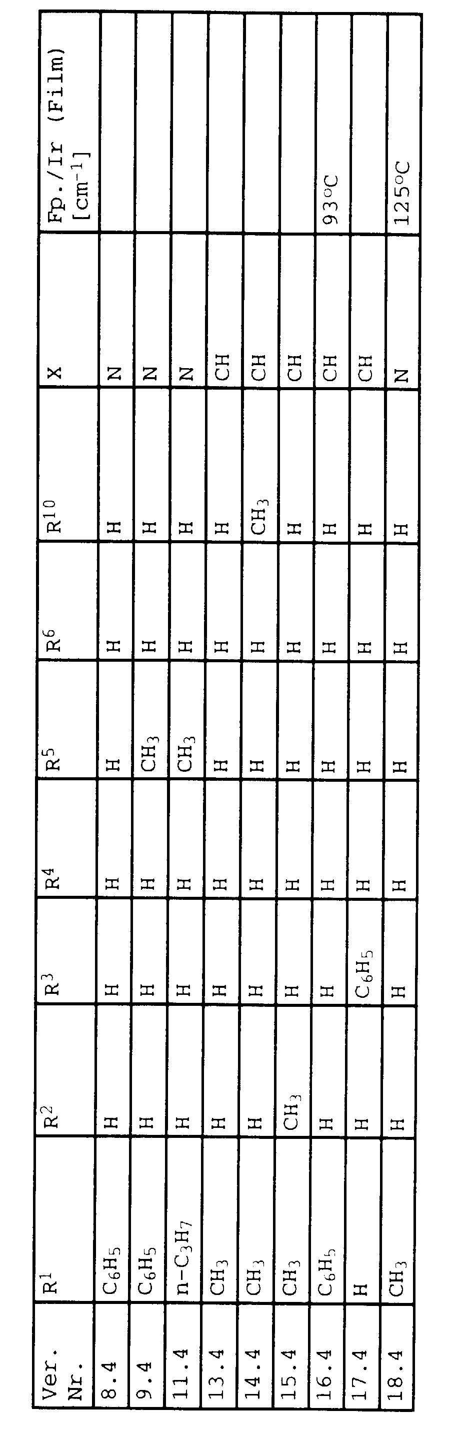

Die neuen Verbindungen eignen sich als Fungizide, Insektizide, Nematizide und zur Regulierung des Pflanzenwachstums.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden kannen. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen der Verbindung lc und 10 Gew.-Teilen N-Methyl-a-pyrrolidon, die zur Anwendung in Form kleinster Tropfen geeignet ist;
II. eine Mischung aus 20 Gew.-Teilen der Verbindung 3c, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung 20d, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung 63c, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung 65c, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung 71c und 97 Gew.-Teilen feinteiligem Kaolin; dieses Stäubemittel enthält 3 Gew.-% Wirkstoff;
VII. eine innige Mischung aus 30 Gew.-Teilen der Verbindung 73c, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung 133c, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung 159c, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

### Anwendungsbeispiel 1

### Wirksamkeit gegen Pyricularia oryzae (protektiv)

Blätter von in Töpfen gewachsenen Reiskeimlingen der Sorte "Tai-Nong 67" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 - 24°C und 95 - 99 % relativer Luftfeuchtigkeit aufgestellt. Nach 6 Tagen wurde das Ausmaß des Krankheitsbefalls ermittelt.

Das Ergebnis des Versuches zeigt, daß die Verbindungen 3c, 63c, 65c, 71c, 133c, 159c und 20d bei der Anwendung als 250 ppm Wirkstoff enthaltende wäßrige Spritzbrühe eine gute fungizide Wirkung zeigen (90 %).

## Patentansprüche

1. Substituierte Pyridin-Derivate der Formel I, 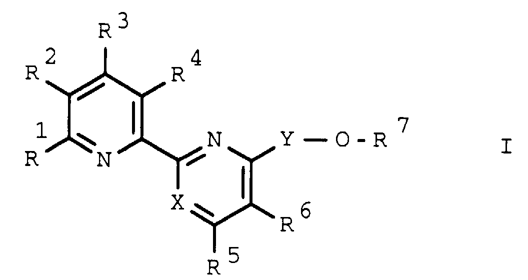 worin
R¹ = Wasserstoff, C₁-C₆-Alkyl, C₁-C₄-Alkoxy-C₁-C₄-alkyl, C₁-C₄-Alkylthio-C₁-C₄-alkyl, C₂-C₆-Alkenyl, C₂-C₆-Alkinyl, C₃-C₇-Cycloalkyl, wobei der Cycloalkylrest bis zu dreifach durch C₁-C₄-Alkyl substituiert sein kann, Halogen, Phenyl, Phenoxy-C₁-C₄-alkyl, Phenylmercapto-C₁-C₄-alkyl, Phenyl-C₁-C₄-alkyl, Phenoxy, Phenylmercapto,
wobei die sechs letztgenannten Reste im Phenylteil bis zu dreifach durch Halogen, Nitro, Cyano, C₁-C₄-Alkyl,
C₁-C₄-Alkoxy, C₁-C₄-Alkylthio, C₁-C₄-Haloalkyl oder C₁-C₄-Haloalkoxy substituiert sein können,
R², R³, R⁴ = unabhängig voneinander Wasserstoff, C₁-C₆-Alkyl, Phenyl, wobei der Phenylrest bis zu dreifach durch Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein kann,
R⁵ = Wasserstoff, C₁-C₆-Alkyl, C₃-C₇-Cycloalkyl, C₃-C₇-Cycloalkyl-C₁-C₄-alkyl, wobei die beiden letztgenannten Reste im Cycloalkylteil bis zu dreifach durch C₁-C₄-Alkyl substituiert sein können, C₁-C₄-Haloalkyl, C₁-C₄-Alkoxy-C₁-C₄-alkyl, C₁-C₄-Alkylthio-C₁-C₄-alkyl, C₂-C₆-Alkenyl, C₂-C₆-Alkinyl, Phenyl, Phenyl-C₁-C₄-alkyl, Phenoxy-C₁-C₄-alkyl, Phenylmercapto-C₁-C₄-alkyl, wobei die vier letztgenannten Reste im Phenylteil bis zu dreifach durch Halogen, Nitro, Cyano, C₁-C₄-Alkyl, C₁-C₄-Alkoxy, C₁-C₄-Alkylthio, C₁-C₄-Haloalkyl oder C₁-C₄-Haloalkoxy substituiert sein können,
R⁶ = Wasserstoff, C₁-C₄-Alkyl, C₁-C₄-Alkoxy, C₁-C₄-Alkoxycarbonyl, Halogen, Phenyl, wobei der Phenylrest bis zu dreifach durch Halogen, Nitro, Cyano, C₁-C₄-Alkyl, C₁-C₄-Alkoxy, C₁-C₄-Alkylthio, C₁-C₄-Raloalkyl oder C₁-C₄-Haloalkoxy substituiert sein kann, oder
R⁵ und R⁶ bilden zusammen eine Polymethylenkette der Formel -(CH₂)ₘ- mit m = 3 oder 4,
R⁷ = Wasserstoff, C₁-C₁₂-Alkyl, C₃-C₁₂-Alkenyl, C₃-C₈-Alkinyl, wobei die drei letztgenannten Gruppen bis zu dreifach durch Halogen substituiert sein können, C₁-C₆-Alkoxy-C₂-C₁₀-alkyl, monocyclisches oder polycyclisches C₃-C₁₀-Cycloalkyl oder C₃-C₁₀-Cycloalkylmethyl, wobei diese Ringe bis zu dreifach durch C₁-C₄-Alkyl oder durch einen Phenylrest substituiert sein können,
monocyclisches oder polycyclisches C₅-C₁₀-Cycloalkenyl oder C₅-C₁₀-Cycloalkenylmethyl, wobei diese Ringe bis zu dreifach durch C₁-C₄-Alkyl oder durch einen Phenylrest substituiert sein können,
Phenyl, Phenyl-C₁-C₆-alkyl, Phenyl-C₃-C₆-alkenyl, Phenoxy-C₂-C₆-alkyl, wobei die vier letztgenannten Reste im Phenylteil bis zu dreifach durch Halogen, Nitro, Cyano, C₁-C₄-Alkyl, C₁-C₄-Haloalkyl, C₁-C₄-Alkoxy, C₁-C₄-Haloalkoxy oder C₁-C₄-Alkylthio substituiert sein können,
X = CH oder N, 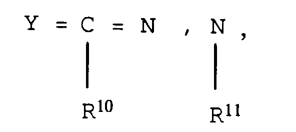
R¹⁰ = Wasserstoff, C₁-C₆-Alkyl,
R¹¹ = Wasserstoff, C₁-C₆-Alkyl, C₃-C₈-cycloalkyl, wobei der Cycloalkylrest bis zu dreifach durch C₁-C₄-Alkyl oder durch einen Phenylrest substituiert sein kann, Phenyl, Phenyl-C₁-C₄-alkyl, wobei die beiden letztgenannten Reste im Phenylteil bis zu dreifach durch Halogen, C₁-C₄-Alkyl, C₁-C₄-Alkoxy oder C₁-C₄-Haloalkyl substituiert sein können,
bedeuten, sowie deren pflanzenverträglichen Säureadditionssalze und Metallsalzkomplexe,

2. Substituierte Pyridin-Derivate der Formel I gemäß Anspruch 1, worin
R¹ = Wasserstoff, C₁-C₆-Alkyl, C₁-C₃-Alkoxy-C₁-C₂-alkyl, Phenyl, Phenyl-C₁-C₂-alkyl, Phenoxy-C₁-C₂-alkyl, wobei die drei letztgenannten Reste im Phenylteil bis zu dreifach durch Halogen oder C₁-C₄-Alkyl substituiert sein können,
R², R³ = unabhängig voneinander Wasserstoff, C₁-C₃-Alkyl, Phenyl, wobei der Phenylrest bis zu dreifach durch Halogen oder C₁-C₄-Alkyl substituiert sein kann,
R⁴ = Wasserstoff
R⁵ = Wasserstoff, C₁-C₆-Alkyl, C₃-C₆-Cycloalkyl, C₅-C₆-Cycloalkyl -C₁-C₃-alkyl, Phenyl, Phenyl-C₁-C₂-alkyl, wobei die beiden letztgenannten Reste im Phenylteil unsubstituiert oder bis zu dreifach durch Halogen, C₁-C₄-Alkyl oder C₁-C₄-Alkoxy substituiert sein können, C₁-C₃-Haloalkyl, C₁-C₄-Alkoxy,C₁-C₄-Alkylthio,
R⁶= Wasserstoff, C₁-C₄-Alkyl, Halogen, Phenyl, C₁-C₃-Alkoxy oder
R⁵ und R⁶ bilden zusammen eine Polymethylenkette der Formel -(CH₂)ₘ- mit m = 3 oder 4,
R⁷ = Wasserstoff, C₁-C₆-Alkyl, C₃-C₆-Alkenyl, C₃-C₆-Alkinyl, wobei die drei letztgenannten Gruppen bis zu dreifach durch Halogen substituiert sein können,
C₁-C₃-Alkoxy-C₂-C₆-alkyl,
monocyclisches oder polycyclisches C₃-C₁₀-Cycloalkyl oder C₃-C₁₀-Cycloalkylmethyl, wobei diese Ringe bis zu zweifach durch C₁-C₄-Alkyl oder durch einen Phenylrest substituiert sein können,
monocyclisches oder polycyclisches C₅-C₁₀-Cycloalkenyl oder C₅-C₁₀-Cycloalkenylmethyl, wobei diese Ringe bis zu zweifach durch C₁-C₄-Alkyl oder durch einen Phenylrest substituiert sein können,
Phenyl, Phenyl-C₁-C₆-alkyl, Phenyl-C₃-C₆-alkenyl, Phenoxy-C₂-C₆-alkyl, wobei die vier letztgenannten Reste im Phenylteil bis zu dreifach durch Halogen, C₁-C₄-Alkyl, C₁-C₃-Haloalkyl oder C₁-C₄-Alkoxy substituiert sein können,
X = CH oder N, 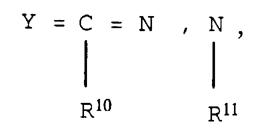
R¹⁰ = Wasserstoff, C₁-C₄-Alkyl,
R¹¹ = Wasserstoff, C₁-C₄-Alkyl, C₃-C₈-Cycloalkyl, wobei der Cycloalkylrest bis zu zweifach durch C₁-C₄-Alkyl oder durch einen Phenylrest substituiert sein kann, Phenyl, Phenyl-C₁-C₂-alkyl, wobei die beiden letztgenannten Reste im Phenylteil bis zu dreifach durch Halogen, C₁-C₄-Alkyl, C₁-C₄-Alkoxy oder C₁-C₃-Haloalkyl substituiert sein können,
bedeuten, sowie deren pflanzenverträglichen Säureadditionssalze und Metallsalzkomplexe.

3. Verfahren zur Herstellung von subst. Pyridin-Derivaten der Formel I gemäß Anspruch 1 dadurch gekennzeichnet, daß man
a) eine Verbindung der Formel II, 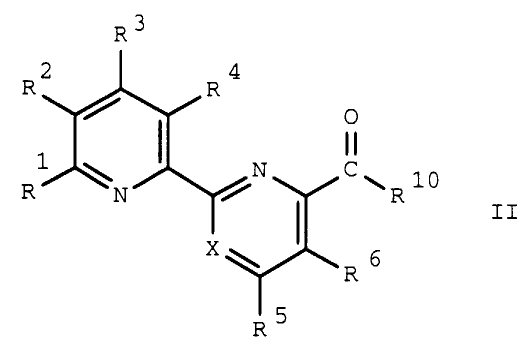 worin R¹ - R⁶, R¹⁰ und X die Bedeutung wie in Formel I gemäß Anspruch 1 besitzen,
mit einem Hydroxylamin-Salz in Gegenwart einer Base zu einem Oxim der Formel I a 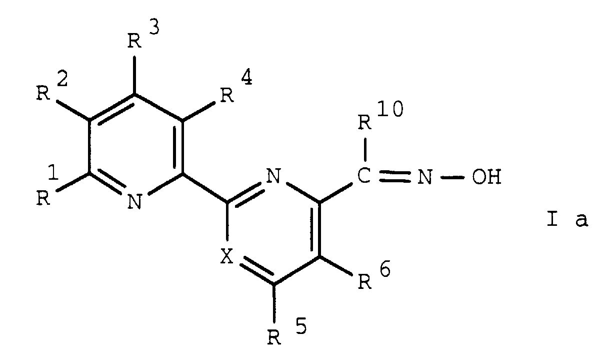 umsetzt, und das Oxim der Formel I a mittels einer Base in ein Oximsalz der Formel I b 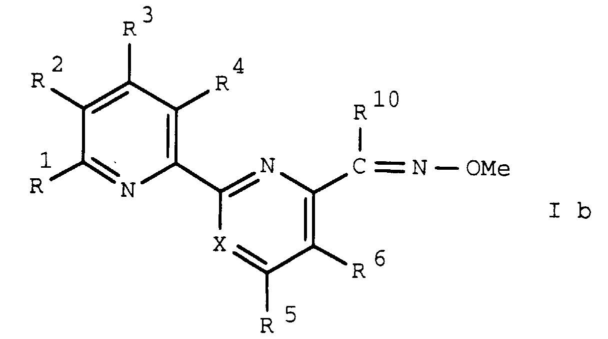 überführt, wobei Me ein Alkali- oder Erdalkaliatom darstellt,
und das Oximsalz der Formel I b mit einer Verbindung der Formel III,
R⁷-X' III
worin R⁷ die Bedeutung wie in Formel I gemäß Anspruch 1 besitzt und X' eine nucleofuge Abgangsgruppe darstellt, zu einer Verbindung der Formel I c 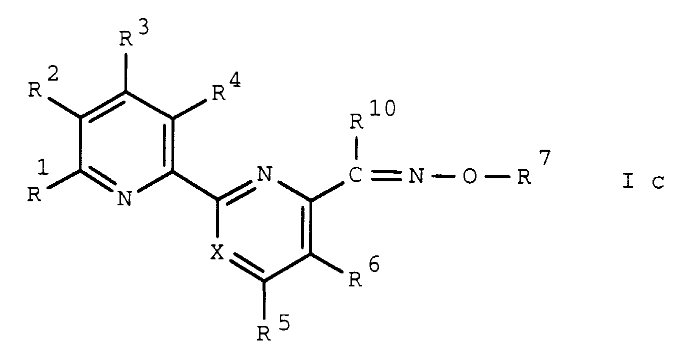 umsetzt, oder
b) eine Verbindung der Formel IV a
H₂N-O-R⁷ • HA IV a
mit einer Verbindung der Formel II 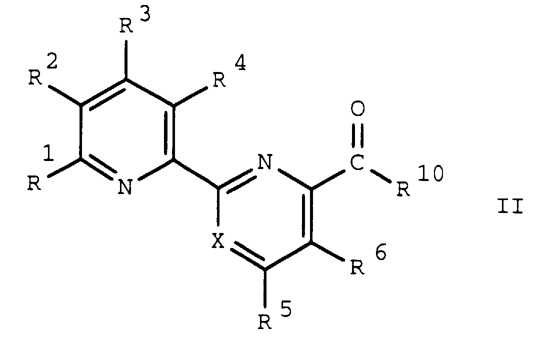 oder einer Verbindung der Formel V, 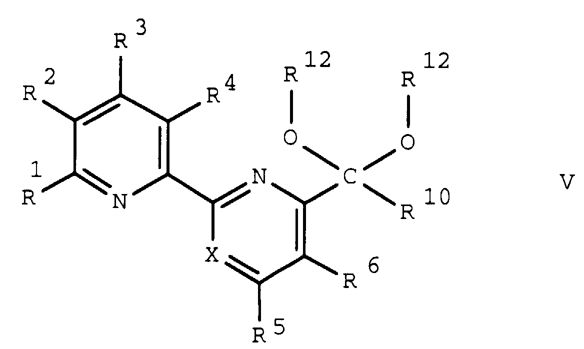 worin R¹ - R⁷, R¹⁰ und X die Bedeutung wie in Formel I gemäß Anspruch 1 besitzen, HA eine anorganische oder organische Säure und R¹² = C₁-C₄-Alkyl oder die beiden Reste R¹² zusammen eine Polymethylenkette der Formel -(CH₂)ₙ- mit n = 2 oder 3 darstellen,
gegebenenfalls in Gegenwart einer Base zu einer Verbindung der Formel I c umsetzt, oder
c) eine Verbindung der Formel VI 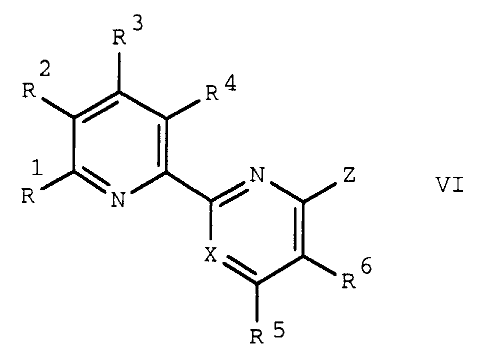 mit einer Verbindung der Formel IV b bzw. IV c 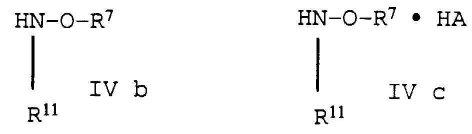 worin R¹ - R⁷, R¹¹ und X die Bedeutung wie in Formel I gemäß Anspruch 1 besitzen, Z für Halogen und HA für eine anorganische oder organische Säure stehen,
gegebenenfalls in Gegenwart einer Base zu einer Verbindung der Formel I d 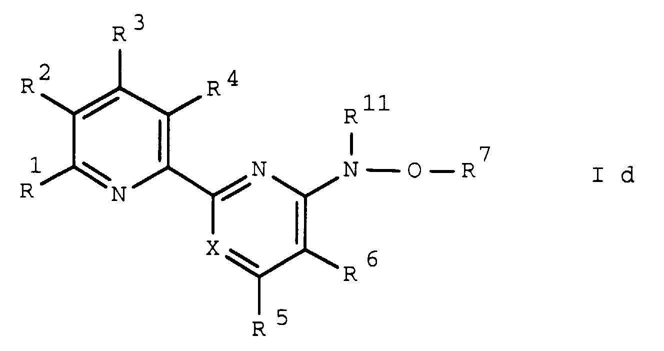 umsetzt.

4. Verwendung der subst. Pyridin-Derivate der Formel I, deren pflanzenverträglichen Säureadditionssalze und Metallsalzkomplexe gemäß Anspruch 1 als Fungizide.

5. Fungizides Mittel, enthaltend mindestens ein subst. Pyridin-Derivat der Formel I, dessen pflanzenverträgliches Säureadditionssalz oder Metallsalzkomplex gemäß Anspruch 1 und einen flüssigen oder festen Trägerstoff.

6. Verfahren zur Bekämpfung von Pilzen dadurch gekennzeichnet, daß man eine fungizid wirksame Menge eines subst. Pyridin-Derivats der Formel I, dessen pflanzenverträglichem Säureadditionssalz oder Metallsalzkomplex gemäß Anspruch 1 auf Pilze, vom Pilzbefall bedrohte Pflanzen, deren Lebensraum oder auf das Saatgut der bedrohten Pflanzen einwirken läßt.

7. Verbindungen der Formel II, 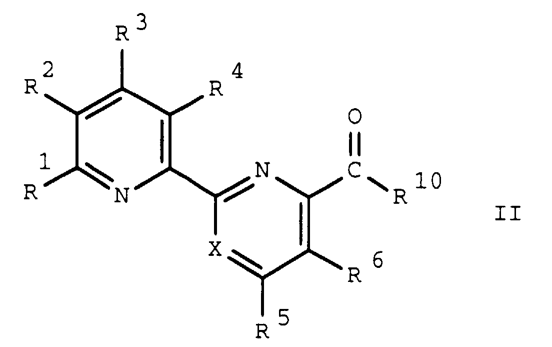 worin R¹ - R⁶, R¹⁰ und X die Bedeutung wie in Formel I gemäß Anspruch 1 besitzen, ausgenommen die Verbindungen 6-Formyl-2,2'-bipyridin, 6-Acetyl-2,2'-bipyridin.

8. Verbindungen der Formel II gemäß Anspruch 7, worin R¹ - R⁶, R¹⁰ und X die Bedeutung wie in Formel I gemäß Anspruch 2 besitzen.

9. Verbindungen der Formel V, 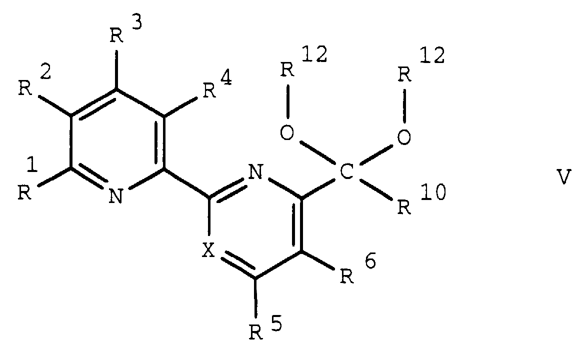 worin R¹ - R⁶, R¹⁰ und X die Bedeutung wie in Formel I gemäß Anspruch 1 besitzen und R¹² = C₁-C₄-Alkyl oder die beiden Reste R¹² zusammen eine Polymethylenkette der Formel -(CH₂)ₙ- mit n = 2 oder 3 darstellen.

10. Verbindungen der Formel V gemäß Anspruch 9, worin R¹ - R⁶, R¹⁰ und X die Bedeutung wie in Formel I gemäß Anspruch 2 und R¹² die Bedeutung wie in Formel V gemäß Anspruch 9 besitzen.

11. Verfahren zur Herstellung von Verbindungen der Formel II gemäß Anspruch 7 dadurch gekennzeichnet, daß man
a) eine Verbindung der Formel V gemäß Anspruch 9 oder 10 in Gegenwart einer anorganischen oder organischen Säure hydrolysiert, oder
b) eine Verbindung der Formel VII, 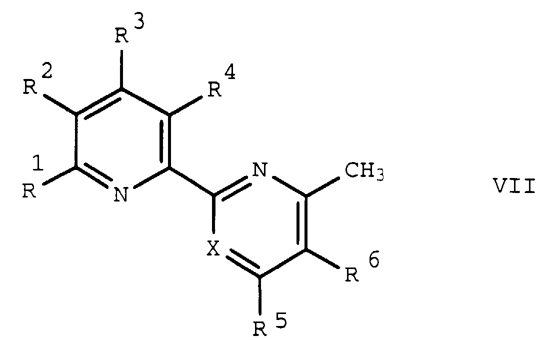 worin R¹ - R⁶ und X die Bedeutung wie in Formel I gemäß Anspruch 1 besitzen, mit einem Oxidationsmittel zu einer Verbindung der Formel II a 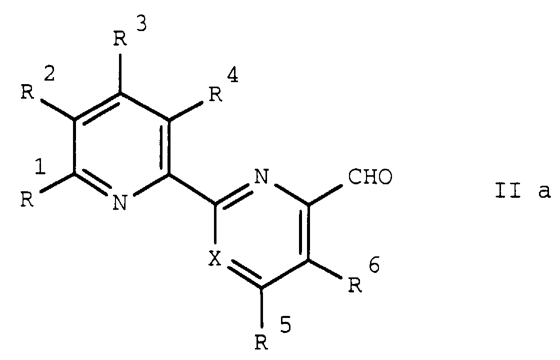 umsetzt, oder
c) eine Verbindung der Formel VI a, 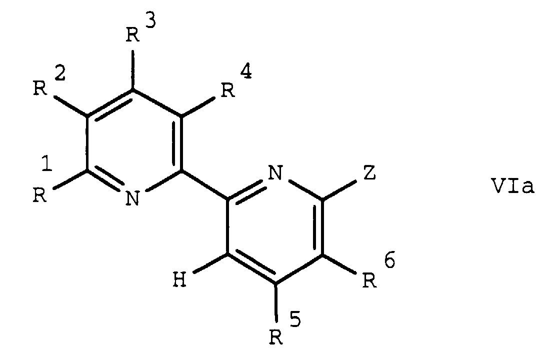 worin R¹ - R⁶ die Bedeutung wie in Formel I gemäß Anspruch 1 besitzen und Z für Halogen steht,
α) mit einer Li-organischen Verbindung der Formel VIII,
R¹³-Li VIII,
worin R¹³ = C₁-C₆-Alkyl, Phenyl darstellt, zu einer Verbindung der Formel IX a, 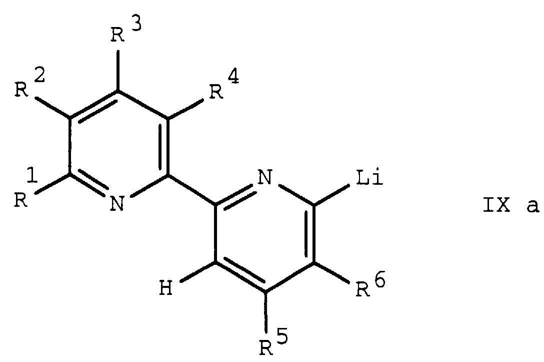 oder
β) mit Mg zu einer Verbindung der Formel IX b 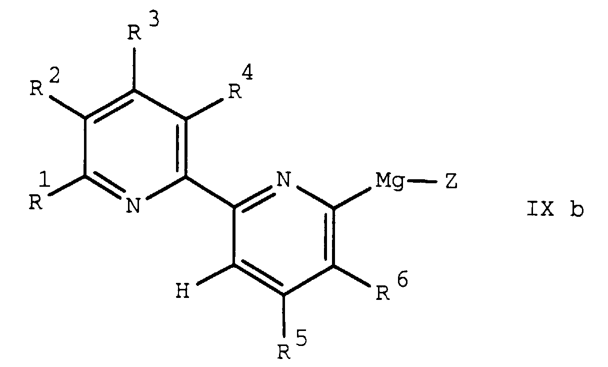 umsetzt, und die metallorganische Verbindung der Formel IX a bzw. IX b mit einer Verbindung der Formel X, 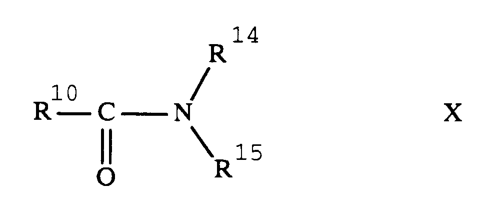 worin R¹⁰ die Bedeutung wie in Formel I gemäß Anspruch 1 besitzt und R¹⁴, R¹⁵ = unabhängig voneinander C₁-C₄-Alkyl, C₁-C₄-Alkoxy, C₅-C₇-Cycloalkyl darstellen oder R¹⁴ und R¹⁵ zusammen mit dem Stickstoffatom, an das sie gebunden sind, einen 5 - 7-gliedrigen Heterocyclus mit 1 oder 2 gleichen oder verschiedenen Heteroatomen, vorzugsweise mit den Heteroatomen Stickstoff und/oder Sauerstoff, bilden, umsetzt.

12. Verfahren zur Herstellung von Verbindungen der Formel V a, 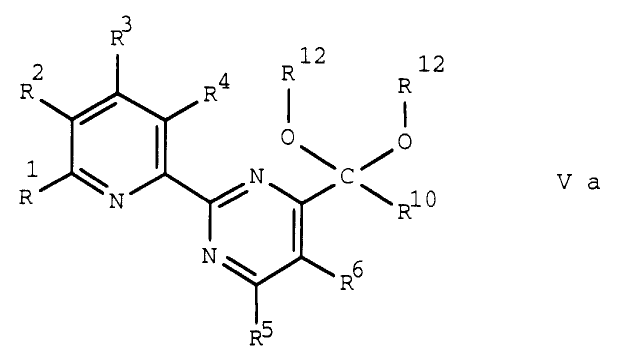 worin R¹ - R⁶, R¹⁰ und R¹² die Bedeutung wie in Formel V gemäß Anspruch 9 oder 10 besitzen, dadurch gekennzeichnet, daß man ein Picolinamidin-Derivat der Formel XI a bzw. XI b, 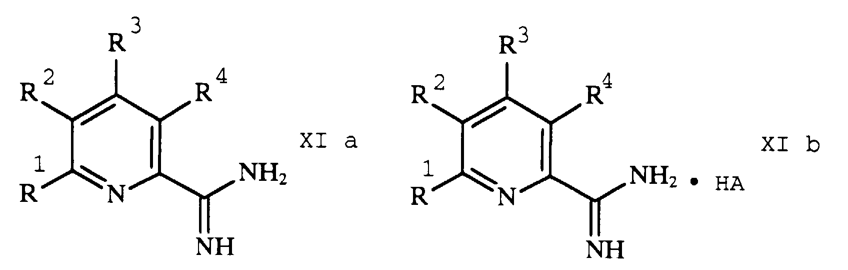 worin R¹ - R⁴ die Bedeutung wie in Formel I gemäß Anspruch 1 besitzen und HA für eine anorganische oder organische Säure steht,
α) mit einer Dicarbonylverbindung der Formel XII, 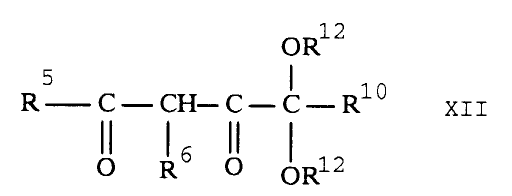 worin R⁶ und R¹⁰ die Bedeutung wie in Formel I gemäß Anspruch 1, R⁵ die Bedeutung wie in Formel I gemäß Anspruch 1, und R¹² die Bedeutung wie in Formel V gemäß Anspruch 9 oder 10 besitzen, oder
β) mit einem Keton der Formel XIII, 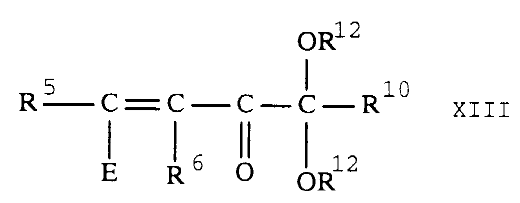 worin R⁶ und R¹⁰ die Bedeutung wie in Formel I gemäß Anspruch 1, R⁵ die Bedeutung wie in Formel I gemäß Anspruch 1, und R¹² die Bedeutung wie in Formel V gemäß Anspruch 9 oder 10 besitzen,
und E für OR¹⁶ oder NR¹⁷R¹⁸ steht, mit R¹⁶ = C₁-C₄-Alkyl und R¹⁷, R¹⁸ = unabhängig voneinander Wasserstoff, C₁-C₄-Alkyl oder R¹⁷ und R¹⁸ bilden zusammen mit dem Stickstoffatom, an das sie gebunden sind, einen 5- bis 7-gliedrigen Heterocyclus mit 1 oder 2 gleichen oder verschiedenen Heteroatomen, vorzugsweise mit den Heteroatomen Stickstoff und/oder Sauerstoff,
gegebenenfalls in Gegenwart einer Base umsetzt.

## Claims

1. A substituted pyridine derivative of the formula I 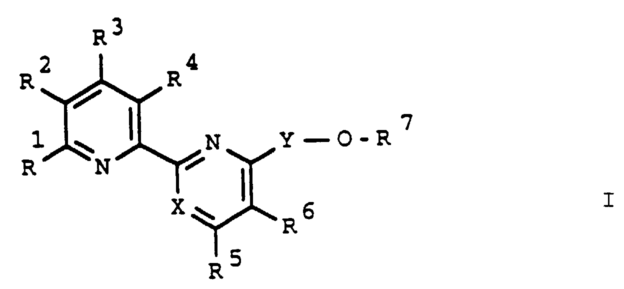 where
R¹ is hydrogen, C₁-C₆-alkyl, C₁-C₄-alkoxy-C₁-C₄-alkyl, C₁-C₄-alkylthio-C₁-C₄-alkyl, C₂-C₆-alkenyl, C₂-C₆-alkynyl, C₃-C₇-cycloalkyl, where the cycloalkyl radical may be monosubstituted, disubstituted or trisubstituted by C₁-C₄-alkyl, or halogen, phenyl, phenoxy-C₁-C₄-alkyl, phenylmercapto-C₁-C₄-alkyl, phenyl-C₁-C₄-alkyl, phenoxy or phenylmercapto, where the six last-mentioned radicals may be monosubstituted, disubstituted or trisubstituted in the phenyl moiety by halogen, nitro, cyano, C₁-C₄-alkyl, C₁-C₄-alkoxy, C₁-C₄-alkylthio, C₁-C₄-haloalkyl or C₁-C₄-haloalkoxy,
R², R³ and R⁴ independently of one another are each hydrogen, C₁-C₆-alkyl or phenyl where the phenyl radical may be monosubstituted, disubstituted or trisubstituted by halogen, nitro, cyano, C₁-C₄-alkyl, C₁-C₄-alkoxy, C₁-C₄-alkylthio, C₁-C₄-haloalkyl or C₁-C₄-haloalkoxy,
R⁵ is hydrogen, C₁-C₆-alkyl, C₃-C₇-cycloalkyl, C₃-C₇-cycloalkyl-C₁-C₄-alkyl, where the two last-mentioned radicals may be monosubstituted, disubstituted or trisubstituted in the cycloalkyl moiety by C₁-C₄-alkyl, or C₁-C₄-haloalkyl, C₁-C₄-alkoxy-C₁-C₄-alkyl, C₁-C₄-alkylthio-C₁-C₄-alkyl, C₂-C₆-alkenyl, C₂-C₆-alkynyl, phenyl, phenyl-C₁-C₄-alkyl, phenoxy-C₁-C₄-alkyl or phenylmercapto-C₁-C₄-alkyl, where the four last-mentioned radicals may be monosubstituted, disubstituted or trisubstituted in the phenyl moiety by halogen, nitro, cyano, C₁-C₄-alkyl, C₁-C₄-alkoxy, C₁-C₄-alkylthio, C₁-C₄-haloalkyl or C₁-C₄-haloalkoxy,
R⁶ is hydrogen, C₁-C₄-alkyl, C₁-C₄-alkoxy, C₁-C₄-alkoxycarbonyl, halogen or phenyl, where the phenyl radical may be monosubstituted, disubstituted or trisubstituted by halogen, nitro, cyano, C₁-C₄-alkyl, C₁-C₄-alkoxy, C₁-C₄-alkylthio, C₁-C₄-haloalkyl or C₁-C₄-haloalkoxy, or R⁵ and R⁶ together form a polymethylene chain of the formula -(CH₂)ₘ- in which m is 3 or 4,
R⁷ is hydrogen, C₁-C₁₂-alkyl, C₃-C₁₂-alkenyl, C₃-C₈-alkynyl, where the three last-mentioned groups may be monosubstituted, disubstituted or trisubstituted by halogen, or C₁-C₆-alkoxy-C₂-C₁₀-alkyl, monocyclic or polycyclic C₃-C₁₀-cycloalkyl or C₃-C₁₀-cycloalkylmethyl, where these rings may be monosubstituted, disubstituted or trisubstituted by C₁-C₄-alkyl or monosubstituted by phenyl, or monocyclic or polycyclic C₅-C₁₀-cycloalkenyl or C₅-C₁₀-cycloalkenylmethyl, where these rings may be monosubstituted, disubstituted or trisubstituted by C₁-C₄-alkyl or monosubstituted by phenyl, or phenyl, phenyl-C₁-C₆-alkyl, phenyl-C₃-C₆-alkenyl or phenoxy-C₂-C₆-alkyl, where the four last-mentioned radicals may be monosubstituted, disubstituted or trisubstituted in the phenyl moiety by halogen, nitro, cyano, C₁-C₄-alkyl, C₁-C₄-haloalkyl, C₁-C₄-alkoxy, C₁-C₄-haloalkoxy or C₁-C₄-alkylthio,
X is CH or N,
Y is 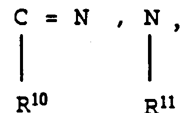
R¹⁰ is hydrogen or C₁-C₆-alkyl and
R¹¹ is hydrogen, C₁-C₆-alkyl or C₃-C₈-cycloalkyl, where the cycloalkyl radical may be monosubstituted, disubstituted or trisubstituted by C₁-C₄-alkyl or monosubstituted by phenyl, or phenyl or phenyl-C₁-C₄-alkyl, where the two last-mentioned radicals may be monosubstituted, disubstituted or trisubstituted in the phenyl moiety by halogen, C₁-C₄-alkyl, C₁-C₄-alkoxy or C₁-C₄-haloalkyl, and its plant-tolerated acid addition salts and metal salt complexes.

2. A substituted pyridine derivative of the formula I as claimed in claim 1, where
R¹ is hydrogen, C₁-C₆-alkyl, C₁-C₃-alkoxy-C₁- or C₂-alkyl, phenyl, phenyl-C₁- or C₂-alkyl or phenoxy-C₁- or C₂-alkyl, where the three last-mentioned radicals may be monosubstituted, disubstituted or trisubstituted in the phenyl moiety by halogen or C₁-C₄-alkyl,
R² and R³ independently of one another are each hydrogen, C₁-C₃-alkyl or phenyl, where the phenyl radical may be monosubstituted, disubstituted or trisubstituted by halogen or C₁-C₄-alkyl,
R⁴ is hydrogen,
R⁵ is hydrogen, C₁-C₆-alkyl, C₃-C₆-cycloalkyl, C₅- or C₆-cycloalkyl-C₁-C₃-alkyl, phenyl or phenyl-C₁- or C₂-alkyl, where the two last-mentioned radicals may be unsubstituted or monosubstituted, disubstituted or trisubstituted in the phenyl moiety by halogen, C₁-C₄-alkyl or C₁-C₄-alkoxy, or C₁-C₃-haloalkyl, C₁-C₄-alkoxy or C₁-C₄-alkylthio,
R⁶ is hydrogen, C₁-C₄-alkyl, halogen, phenyl or C₁-C₃-alkoxy, or
R⁵ and R⁶ together form a polymethylene chain of the formula -(CH₂)ₘ- in which m is 3 or 4,
R⁷ is hydrogen, C₁-C₆-alkyl, C₃-C₆-alkenyl or C₃-C₆-alkynyl, where the three last-mentioned groups may be monosubstituted, disubstituted or trisubstituted by halogen, or C₁-C₃-alkoxy-C₂-C₆-alkyl, monocyclic or polycyclic C₃-C₁₀-cycloalkyl or C₃-C₁₀-cycloalkylmethyl, where these rings may be monosubstituted or disubstituted by C₁-C₄-alkyl or monosubstituted by phenyl, or monocyclic or polycyclic C₅-C₁₀-cycloalkenyl or C₅-C₁₀-cycloalkenylmethyl, where these rings may be monosubstituted or disubstituted by C₁-C₄-alkyl or monosubstituted by phenyl, or phenyl, phenyl-C₁-C₆-alkyl, phenyl-C₃-C₆-alkenyl or phenoxy-C₂-C₆-alkyl, where the four last-mentioned radicals may be monosubstituted, disubstituted or trisubstituted in the phenyl moiety by halogen, C₁-C₄-alkyl, C₁-C₃-haloalkyl or C₁-C₄-alkoxy,
X is CH or N,
Y is 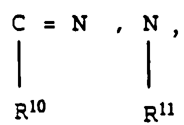
R¹⁰ is hydrogen or C₁-C₄-alkyl and
R¹¹ is hydrogen, C₁-C₄-alkyl or C₃-C₈-cycloalkyl, where the cycloalkyl radical may be monosubstituted or disubstituted by C₁-C₄-alkyl or monosubstituted by phenyl, or phenyl or phenyl-C₁- or C₂-alkyl, where the two last-mentioned radicals may be monosubstituted, disubstituted or trisubstituted in the phenyl moiety by halogen, C₁-C₄-alkyl, C₁-C₄-alkoxy or C₁-C₃-haloalkyl,
and its plant-tolerated acid addition salts and metal salt complexes.

3. A process for the preparation of a substituted pyridine derivative of the formula I as claimed in claim 1, wherein
a) a compound of the formula II 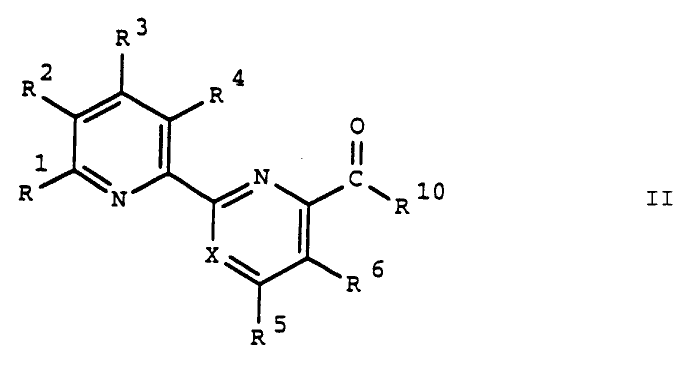 where R¹-R⁶, R¹⁰ and X have the meanings as in the formula I as claimed in claim 1, is reacted with a hydroxylamine salt in the presence of a base to give an oxime of the formula I a 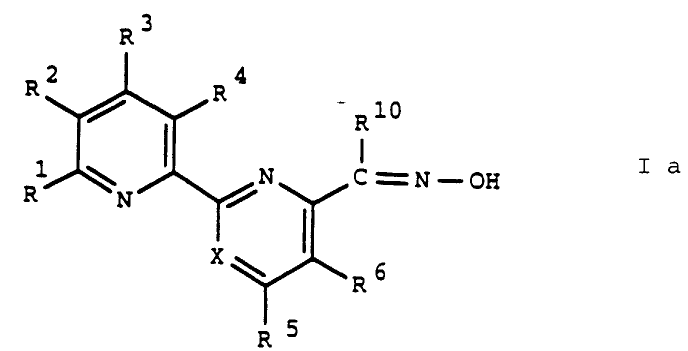 and the latter is converted by means of a base into an oxime salt of the formula I b 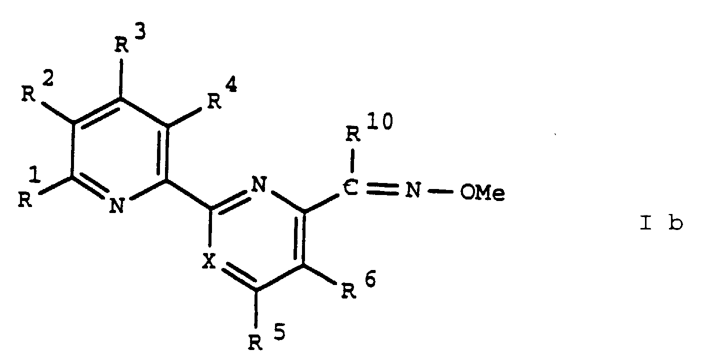 where Me is an alkali metal or alkaline earth metal atom, and the oxime salt of the formula I b is reacted with a compound of the formula III
R⁷-X' III
where R⁷ has the meanings as in the formula I as claimed in claim 1 and X' is a nucleofugic leaving group, to give a compound of the formula I c 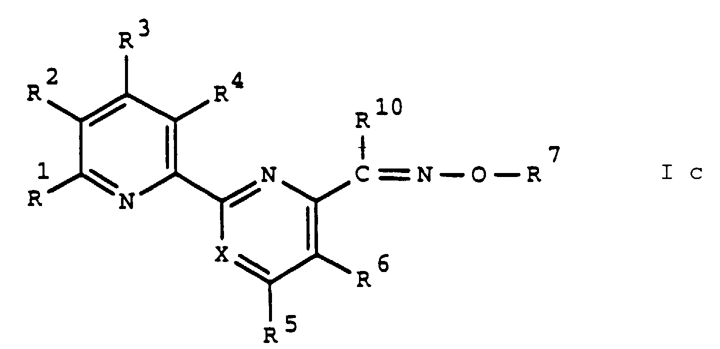 or
b) a compound of the formula IV a
H₂N-O-R⁷ · HA IV a
is reacted with a compound of the formula II 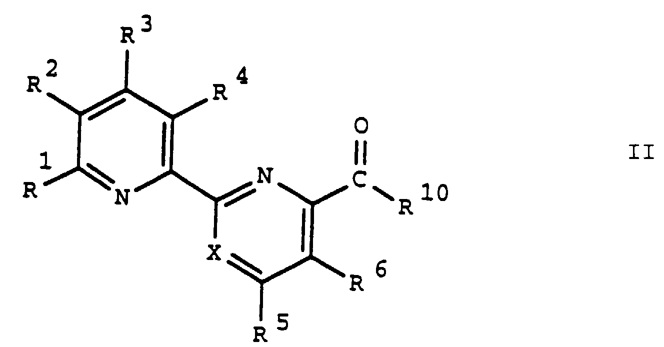 or with a compound of the formula V 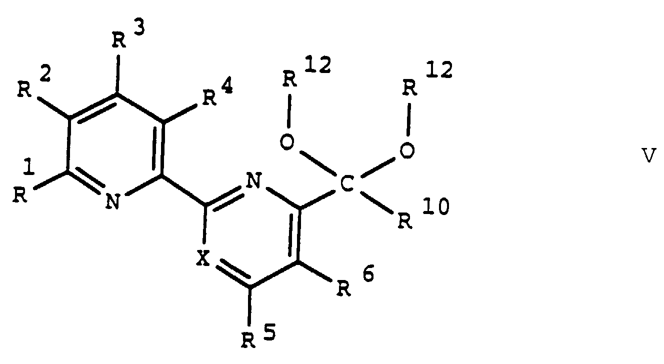 where R¹-R⁷, R¹⁰ and X have the meanings as in the formula I as claimed in claim 1, HA is an inorganic or organic acid, and R¹² is C₁-C₄-alkyl or the two radicals R¹² together form a polymethylene chain of the formula -(CH₂)ₙ- in which n is 2 or 3, in the presence or absence of a base to give a compound of the formula I c, or
c) a compound of the formula VI 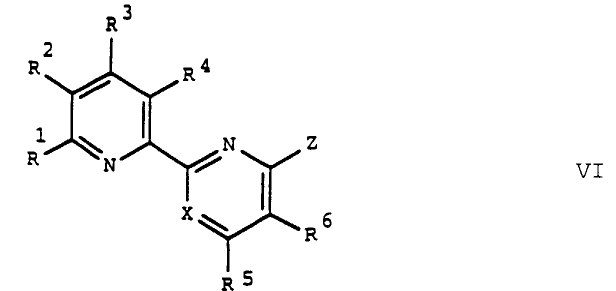 is reacted with a compound of the formula IV b or IV c 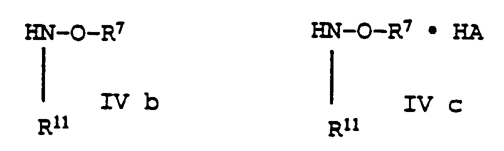 where R¹-R⁷, R¹¹ and X have the meanings as in the formula I as claimed in claim 1, Z is halogen and HA is an inorganic or organic acid, in the presence or absence of a base to give a compound of the formula I d

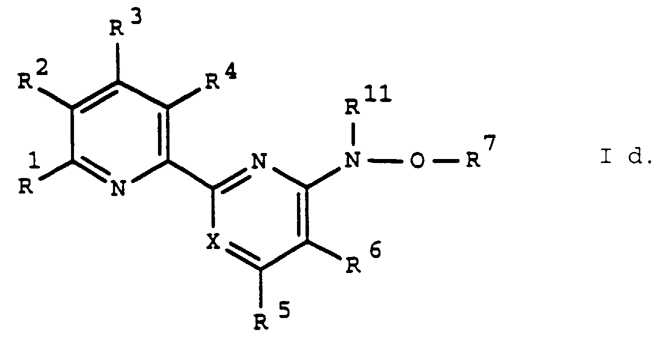

4. Use of a substituted pyridine derivative of the formula I or of its plant-tolerated acid addition salts or metal salt complexes as claimed in claim 1 as a fungicide.

5. A fungicide containing at least one substituted pyridine derivative of the formula I or its plant-tolerated acid addition salt or metal salt complex as claimed in claim 1 and a liquid or solid carrier.

6. A method for controlling fungi, wherein a fungicidal amount of a substituted pyridine derivative of the formula I or of its plant-tolerated acid addition salt or metal salt complex as claimed in claim 1 is allowed to act on fungi, plants threatened by fungal attack, their habitat or the seed of the threatened plants.

7. A compound of the formula II 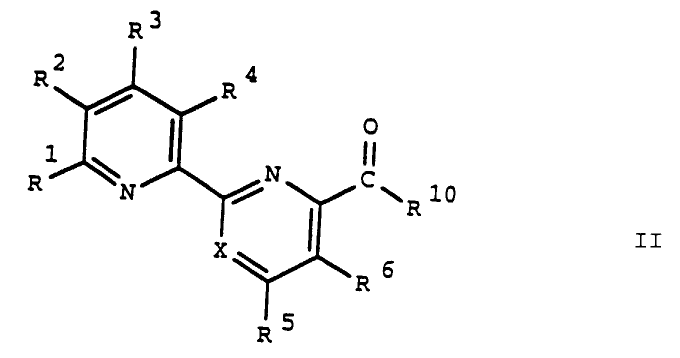 where R¹-R⁶, R¹⁰ and X have the meanings as in the formula I as claimed in claim 1, with the exception of the compounds 6-formyl-2,2'-bipyridine and 6-acetyl-2,2'-bipyridine.

8. A compound of the formula II as claimed in claim 7, where R¹-R⁶, R¹⁰ and X have the meanings as in the formula I as claimed in claim 2.

9. A compound of the formula V 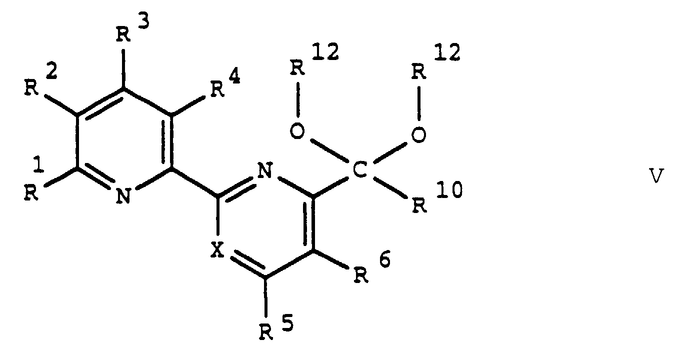 where R¹-R⁶, R¹⁰ and X have the meanings as in the formula I as claimed in claim 1 and R¹² is C₁-C₄-alkyl or the two radicals R¹² together form a polymethylene chain of the formula -(CH₂)ₙ- in which n is 2 or 3.

10. A compound of the formula V as claimed in claim 9, where R¹-R⁶, R¹⁰ and X have the meanings as in the formula I as claimed in claim 2 and R¹² has the meanings as in the formula V as claimed in claim 9.

11. A process for the preparation of a compound of the formula II as claimed in claim 7, wherein
a) a compound of the formula V as claimed in claim 9 or 10 is hydrolyzed in the presence of an inorganic or organic acid or
b) a compound of the formula VII 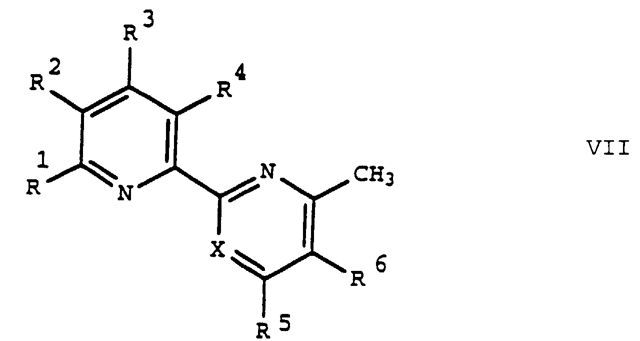 where R¹-R⁶ and X have the meanings as in the formula I as claimed in claim 1, is reacted with an oxidizing agent to give a compound of the formula II a 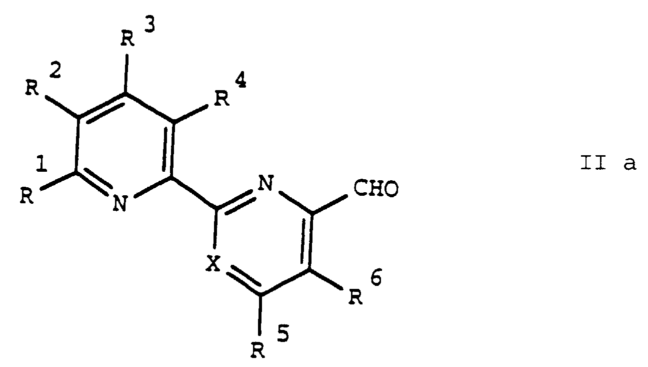 or
c) a compound of the formula VI a 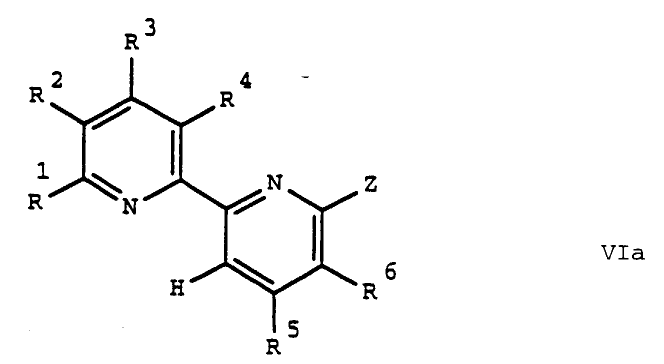 where R¹-R⁶ have the meanings as in the formula I as claimed in claim 1 and Z is halogen, is reacted
α) with an organolithium compound of the formula VIII
R¹³-Li VIII
where R¹³ is C₁-C₆-alkyl or phenyl, to give a compound of the formula IX a 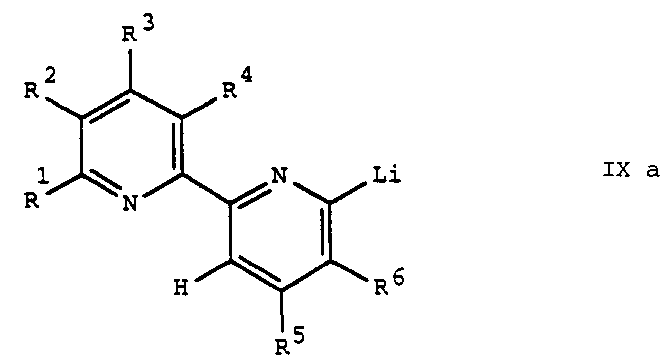 or
β) with Mg to give a compound of the formula IX b 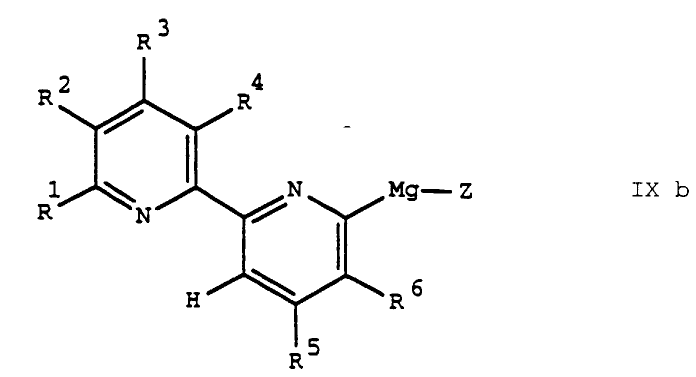 and the organometallic compound of the formula IX a or IX b is reacted with a compound of the formula X 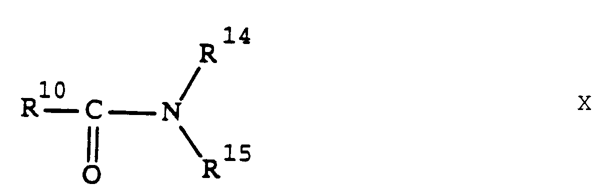 where R¹⁰ has the meanings as in the formula I as claimed in claim 1 and R¹⁴ and R¹⁵ independently of one another are each C₁-C₄-alkyl, C₁-C₄-alkoxy or C₅-C₇-cycloalkyl or R¹⁴ and R¹⁵, together with the nitrogen atom to which they are bonded, form a 5-membered to 7-membered heterocyclic structure having 1 or 2 identical or different hetero atoms, preferably nitrogen or oxygen.

12. A process for the preparation of a compound of the formula V a 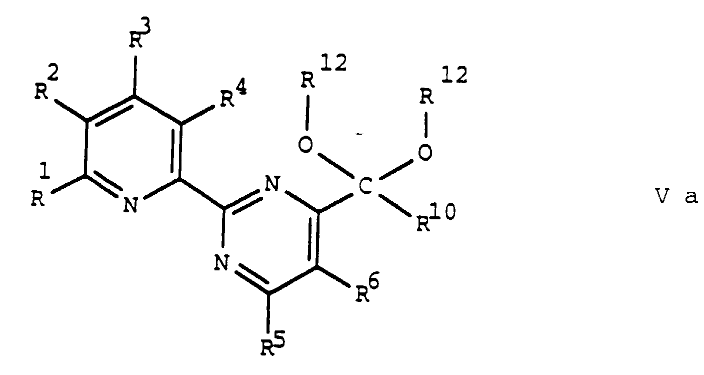 where R¹-R⁶, R¹⁰ and R¹² have the meanings as in the formula V as claimed in claim 9 or 10, wherein a picolin-amidine derivative of the formula XI a or XI b 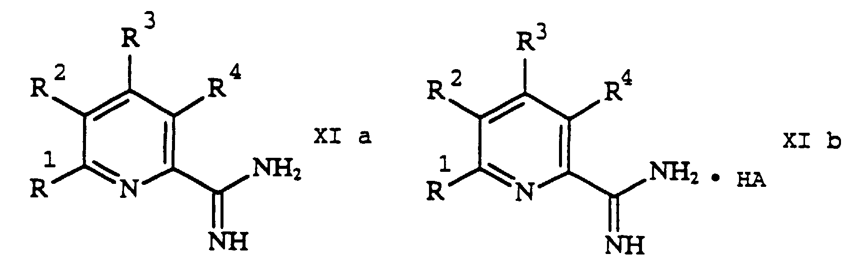 where R¹-R⁴ have the meanings as in the formula I as claimed in claim 1 and HA is an inorganic or organic acid, is reacted α) with a dicarbonyl compound of the formula XII 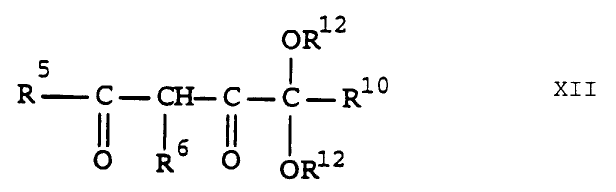 where R⁶ and R¹⁰ have the meanings as in the formula I as claimed in claim 1, R⁵ has the meanings as in the formula I as claimed in claim 1, and R¹² has the meanings as in the formula V as claimed in claim 9 or 10, or β) with a ketone of the formula XIII 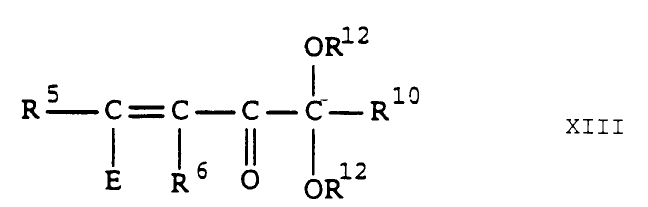 where R⁶ and R¹⁰ have the meanings as in the formula I as claimed in claim 1, R⁵ has the meanings as in the formula I as claimed in claim 1, R¹² has the meanings as in the formula V as claimed in claim 9 or 10, E is OR¹⁶ or NR¹⁷R¹⁸, R¹⁶ is C₁-C₄-alkyl and R¹⁷ and R¹⁸ independently of one another are each hydrogen or C₁-C₄-alkyl or R¹⁷ and R¹⁸, together with the nitrogen atom to which they are bonded, form a 5-membered to 7-membered heterocyclic structure having 1 or 2 identical or different hetero atoms, preferably nitrogen or oxygen, in the presence or absence of a base.

## Revendications

1. Dérivé de pyridine substitué de formule I, 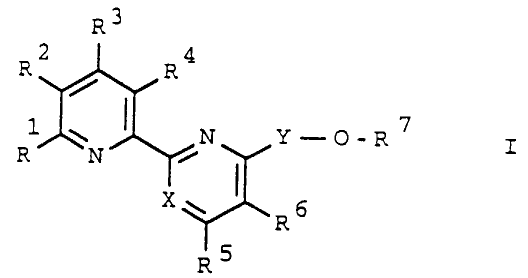 dans laquelle
R¹ = hydrogène, alkyle en C₁-C₆, C₁-C₄-alcoxy-C₁-C₄-alkyle, C₁-C₄-alkylthio-C₁-C₄-alkyle, C₂-C₆-alcényle, C₂-C₆-alkinyle, C₃-C₇-cycloalkyle, le résidu cycloalkyle pouvant être substitué jusqu'à 3 fois par C₁-C₄-alkyle, un halogène, phényl, phénoxy-C₁-C₄-alkyle, phénylmercapto-C₁-C₄-alkyle, phényl-C₁-C₄-alkyle, phénoxy, phénylmercapto,
les six derniers résidus cités pouvant être substitués dans la partie phényle jusqu'à trois fois par halogène, nitro, cyano, C₁-C₄-alkyle, C₁-C₄-alcoxy, C₁-C₄-alkylthio, C₁-C₄-haloalkyle ou C₁-C₄-haloalcoxy,
R², R³, R⁴ = indépendamment les uns des autres, hydrogène, C₁-C₆-alkyle, phényle, le résidu phényle pouvant être substitué jusqu'à trois fois par halogène, nitro, cyano, C₁-C₄-alkyle, C₁-C₄-alcoxy, C₁-C₄-alkylthio, C₁-C₄-halolkyle ou C₁-C₄-haloalcoxy,
R⁵ = hydrogène, C₁-C₆-alkyle, C₃-C₇-cycloalkyle, C₃-C₇-cycloalkyle-C₁-C₄-alkyle, les deux derniers résidus cités pouvant être substitués dans la partie cycloalkyle jusqu'à 3 fois par C₁-C₄-alkyle, C₁-C₄-haloalkyle, C₁-C₄-alcoxy-C₁-C₄-alkyle, C₁-C₄-alkylthio-C₁-C₄-alkyle, C₂-C₆-alcényle, C₂-C₆-alkinyle, phényle, phényl-C₁-C₄-alkyle, phénoxy-C₁-C₄-alkyle, phénylmercapto-C₁-C₄-alkyle, les quatre derniers résidus cités pouvant être substitués dans la partie phényle jusqu'à trois fois par halogène, nitro, cyano, C₁-C₄-alkyle, C₁-C₄-alcoxy, C₁-C₄-alkylthio, C₁-C₄-haloalkyle ou C₁-C₄-haloalcoxy,
R⁶ = hydrogène, C₁-C₄-alkyle, C₁-C₄-alcoxy, C₁-C₄-alcoxycarbonyle, halogène, phényle, le résidu phényle pouvant être substitué jusqu'à 3 fois par halogène, nitro, cyano, C₁-C₄-alkyle, C₁-C₄-alcoxy, C₁-C₄-alkylthio, C₁-C₄-haloalkyle ou C₁-C₄-haloalcoxy, ou
R⁵ et R⁶ constituent conjointement une chaîne de polyméthylène de formule -(CH₂)ₘ- avec m = 3 ou 4,
R⁷ = hydrogène, C₁-C₁₂-alkyle, C₃-C₁₂-alcényle, C₃-C₈-alkinyle, les trois derniers groupes cités pouvant être substitués jusqu'à 3 fois par halogène, C₁-C₆-alcoxy-C₂-C₁₀-alkyle, C₃-C₁₀-cycloalkyle ou C₃-C₁₀-cycloalkylméthyle, monocyclique ou polycyclique, ces anneaux pouvant être substitués jusqu'à trois fois par C₁-C₄-alkyle ou par un résidu phényle,
C₅-C₁₀-cyclohaloalcényle ou C₅-C₁₀-cycloalcénylméthyle, monocyclique ou polycyclique, ces anneaux pouvant être substitués jusqu'à trois fois par C₁-C₄-alkyle ou bien par un résidu phényle,
phényle, phényl-C₁-C₆-alkyle, phényl-C₃-C₆-alcényle phénoxy-C₂-C₆-alkyle, les quatre derniers résidus cités pouvant être substitués dans la partie phényle jusqu'à trois foix par halogène, nitro, cyano, C₁-C₄-alkyle, C₁-C₄-haloalkyle C₁-C₄-alcoxy, C₁-C₄-haloalcoxy, ou C₁-C₄-alkylthio,
X = CH ou N, 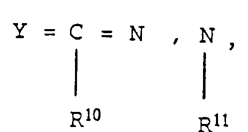
R¹⁰ = hydrogène, C₁-C₆-alkyle,
R¹¹ = hydrogène, C₁-C₆-alkyle, C₃-C₈-cycloalkyle, le résidu cycloalkyle pouvant être substitué jusqu'à trois fois par C₁-C₄-alkyle ou par un résidu phényle, phényle, phényl-C₁-C₆-alkyle, les deux derniers résidus cités pouvant être substitués dans la partie phényle jusqu'à trois par halogène, C₁-C₄-alkyle, C₁-C₄-alcoxy ou C₁-C₄-haloalkyle,
ainsi que leurs sels d'addition acide et complexes de sels métalliques compatibles avec des végétaux.

2. Dérivé de pyridine substitué de formule I selon la revendication 1, dans lequel
R¹ = hydrogène, C₁-C₆-alkyle, C₁-C₃-alcoxy-C₁-C₂-alkyle, phényle, phényl-C₁-C₂-alkyle , phénoxy-C₁-C₂-alkyle, les trois derniers résidus pouvant être substitués dans la partie phényle jusqu'à 3 fois par halogène ou C₁-C₄-alkyle,
R², R³ = indépendamment les uns des autres, hydrogène, C₁-C₃-alkyle, phényle, le résidu phényle pouvant être substitué jusqu'à trois fois par halogène, ou C₁-C₄-alkyle,
R⁴ = hydrogène
R⁵ = hydrogène, C₁-C₆-alkyle, C₃-C₆-cycloalkyle, C₅-C₆-cycloalkyle-C₁-C₃-alkyle, phényle, phényl-C₁-C₂-alkyle, les deux derniers résidus cités étant non substitués dans la partie phényle, ou bien pouvant être substitués jusqu'à 3 fois par halogène, C₁-C₄-alkyle, ou C₁-C₄-alcoxy, C₁-C₃-haloalkyle, C₁-C₄-alcoxy, C₁-C₄-alkylthio,
R⁶ = hydrogène, C₁-C₄-alkyle, halogène, phényle, C₁-C₃-alcoxy, ou
R⁵ et R⁶ constituent conjointement une chaîne de polyméthylène de formule -(CH₂)ₘ- avec m = 3 ou 4,
R⁷ = hydrogène, C₁-C₆-alkyle, C₃-C₆-alcényle, C₃-C₆-alkinyle, les trois derniers groupes cités pouvant être substitués jusqu'à 3 fois par un halogène,
C₁-C₃-alcoxy-C₂-C₆-alkyle,
C₃-C₁₀-cycloalkyle ou C₃-C₁₀-cycloalkylméthyle, monocyclique ou polycyclique, ces anneaux pouvant être substitués jusqu'à deux fois par C₁-C₄-alkyle ou par un résidu phényle,
C₅-C₁₀-cycloalcényle ou C₅-C₁₀-cycloalcénylméthyle, monocyclique ou polycyclique, ces anneaux pouvant être substitués jusqu'à deux fois par C₁-C₄-alkyle ou par un résidu phényle,
phényle, phényl-C₁-C₄-alkyle, phényl-C₃-C₆-alcényle, phénoxy-C₂-C₆-alkyle, les quatre derniers résidus cités dans la partie phényle pouvant être substitués jusqu'à trois par halogène, C₁-C₄-alkyle, C₁-C₃-haloalkyle ou C₁-C₄-alcoxy,
X = CH ou N 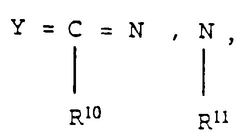
R¹⁰ = hydrogène, C₁-C₄-alkyle,
R¹¹ = hydrogène, C₁-C₄-alkyle, C₃-C₈-cycloalkyle, le résidu cycloalkyle pouvant être substitué jusqu'à deux fois par C₁-C₄-alkyle ou par un résidu phényle, phényl-C₁-C₂-alkyle, les deux derniers résidus cités dans la partie phényle pouvant être substitués jusqu'à trois fois par halogène, C₁-C₄-alkyle, C₁-C₄-alcoxy ou C₁-C₃-haloalkyle,
ainsi que leurs sels d'addition acide et complexes de sels métalliques compatibles avec des végétaux.

3. Procédé de préparation de dérivés de pyridine substitués de formule I selon la revendication 1, caractérisé par le fait qu'on met en réaction
a) une combinaison de formule II 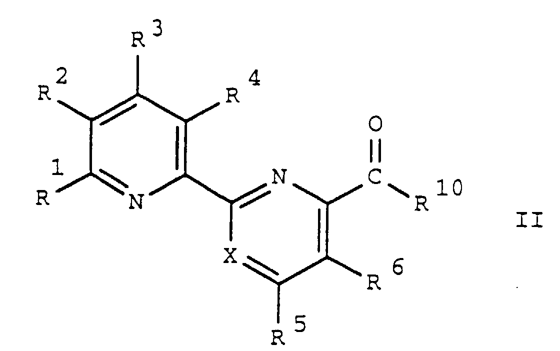 dans laquelle R¹ - R⁶, R¹⁰ et X ont la signification telle que dans la formule I de la revendication 1,
avec un sel d'hydroxylamine en présence d'une base pour donner un oxime de formule I a 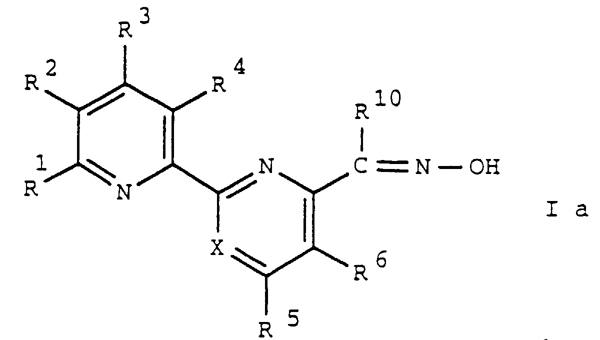 et on convertit l'oxyde de formule I a au moyen d'une base en un sel d'oxime de formule I b 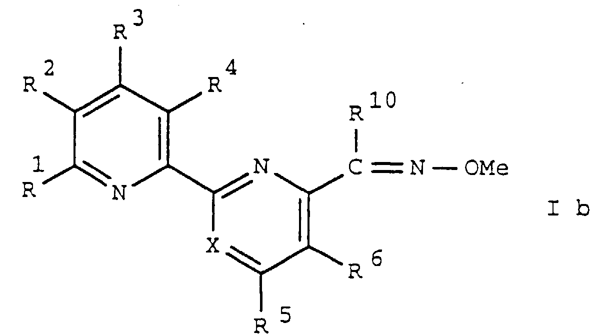 Me étant un atome alcalin ou alcalino-terreux, et le sel d'oxime de formule I b étant mis en réaction avec une combinaison de formule III,
R⁷-X' III
dans laquelle R⁷ a la signification telle que dans la formule I de la revendication 1 et X' constitue un groupe éliminable nucléofuge, pour donner une combinaison de formules I c 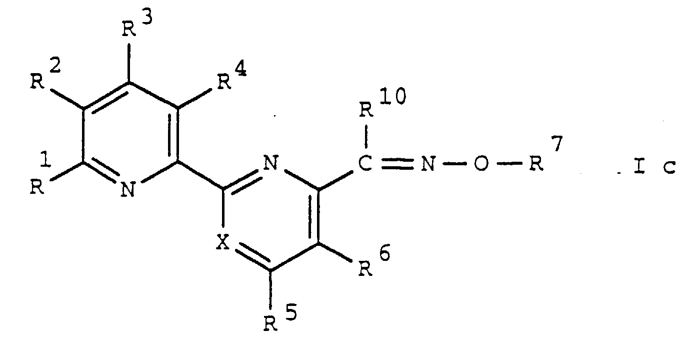 ou
b) une combinaison de formule IV a
H₂N-O-R⁷ · HA IV a
avec la combinaison de formule II 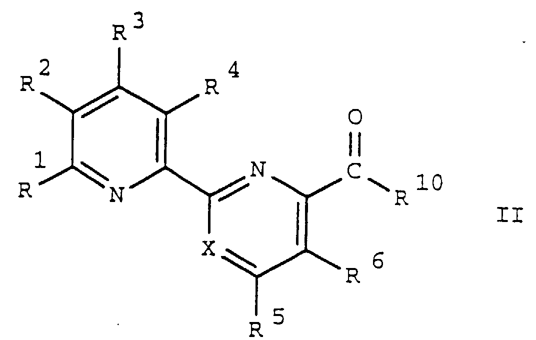 ou une combinaison de formule V 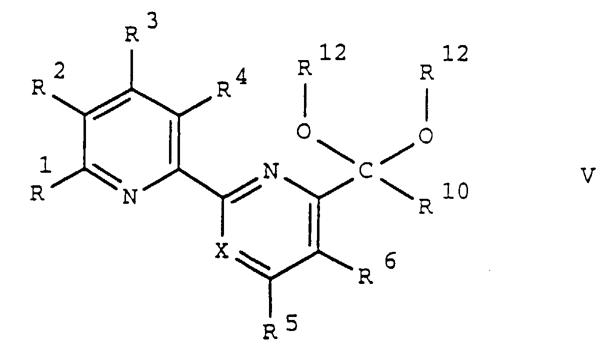 dans laquelle R¹-R⁷, R¹⁰ et X ont la signification telle que dans la formule I de la revendication 1, HA étant un acide minéral ou organique et R¹² = C₁-C₄-alkyle ou bien les deux résidus R¹² constituent conjointement une chaîne de polyméthylène de formule -(CH₂)ₙ-, avec n = 2 ou 3,
le cas échéant en présence d'une base pour donner une combinaison de formule I c, ou
c) une combinaison de formule VI 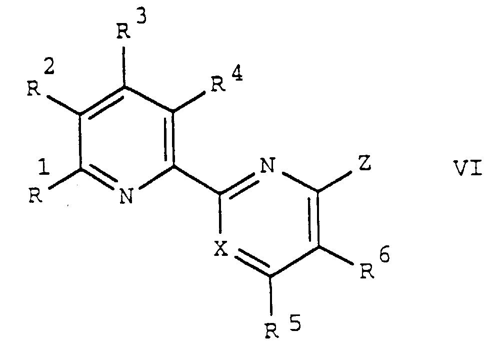 avec une combinaison de formule IV b ou IV c 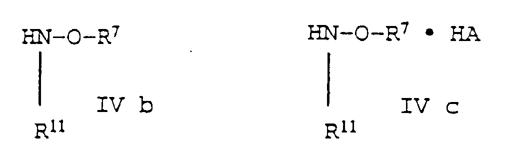 dans laquelle R¹ - R⁷, R¹¹ et X ont la signification telle que dans la formule I de la revendication 1, Z étant un halogène et HA un acide minéral ou organique,
le cas échéant en présence d'une base pour donner une combinaison de formule I d

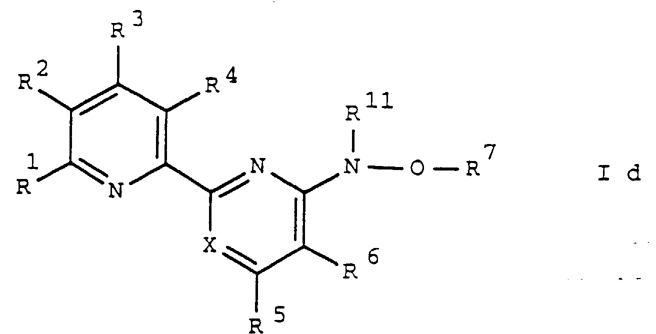

4. Utilisation des dérivés substitués de pyridine de formule I, de leurs sels d'addition acide compatibles et les complexes de sels métalliques compatibles avec les végétaux selon la revendication 1, à titre de fongicides.

5. Agent fongicide, contenant au moins un dérivé substitué de pyridine de formule I, son sel d'addition acide ou complexe de sel métallique compatible avec les végétaux selon la revendication 1 et un support liquide ou solide.

6. Procédé de lutte contre les champignons, caractérisé par le fait qu'on fait agir une quantité, efficace du point de vue fongicide, d'un dérivé substitué de pyridine de formule I, son sel d'addition acide ou complexe de sel métallique compatible avec les végétaux selon la revendication 1 sur des champignons, des plantes menacées d'une attaque par les champignons, leur biotope ou sur les semences des plantes menacées.

7. Combinaisons de formule II, 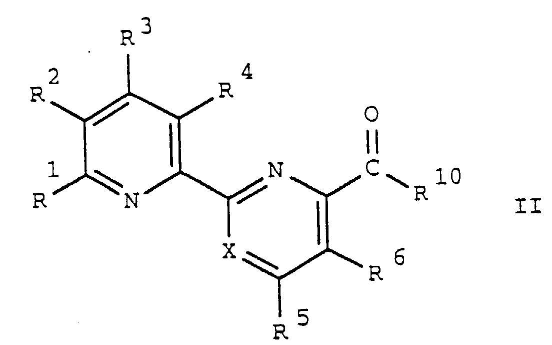 dans laquelle R¹ - R⁶, R¹⁰ et X ont la signification telle que dans la formule I de la revendication 1, à l'exception des combinaisons de 6-formyl-2,2'-bipyridine, 6-acétyl-2,2'-bipyridine.

8. Combinaisons de formule II selon la revendication 7, dans laquelle R¹ - R⁶, R¹⁰ et X ont la signification telle que dans la formule I de la revendication 2.

9. Combinaisons de formule V, 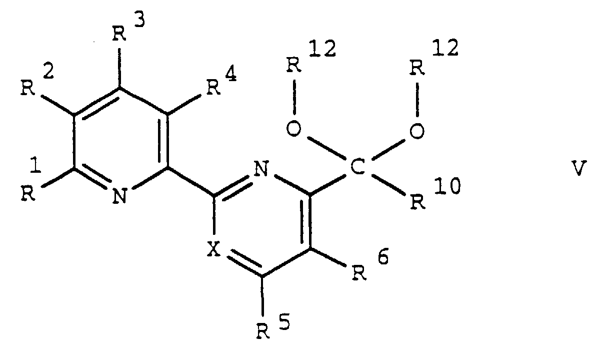 dans laquelle R¹ - R⁶, R¹⁰ et X ont la signification telle que dans la formule I de la revendication 1 et R¹² = C₁-C₄-alkyle ou les deux résidus R¹² conjointement constituent une chaîne de polyméthylène de formule -(CH₂)ₙ-, avec n = 2 ou 3.

10. Combinaisons de formule V selon la revendication 9, dans laquelle R¹ - R⁶, R¹⁰ et X ont la signification telle que dans la formule I de la revendication 2 et R¹² la signification telle que dans la formule V de la revendication 9.

11. Procédé de préparation de combinaisons de formule II selon la revendication 7, caractérisé en ce que :
a) on hydrolyse une combinaison de formule V selon la revendication 9 ou 10, en présence d'un sel minéral ou organique, ou
b) on met en réaction une combinaison de la formule VII, 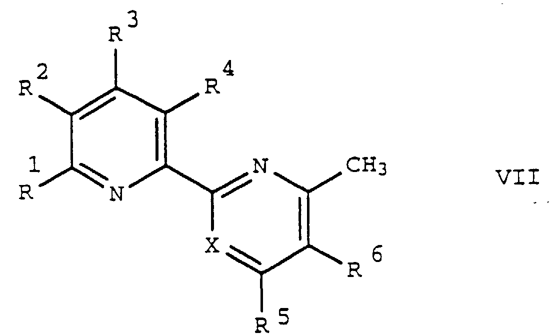 dans laquelle R¹ - R⁶ et X ont la signification telle que dans la formule I de la revendication 1, avec un agent d'oxydation pour donner une combinaison de formule II a 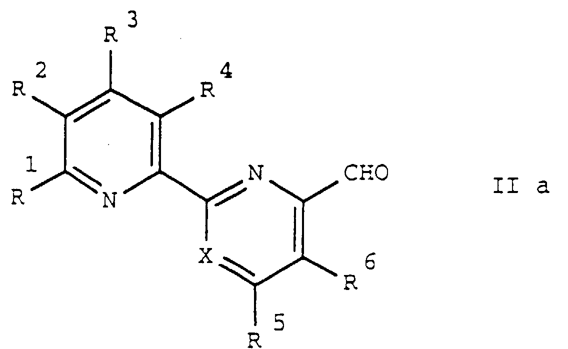 ou
c) une combinaison de la formule VI a, 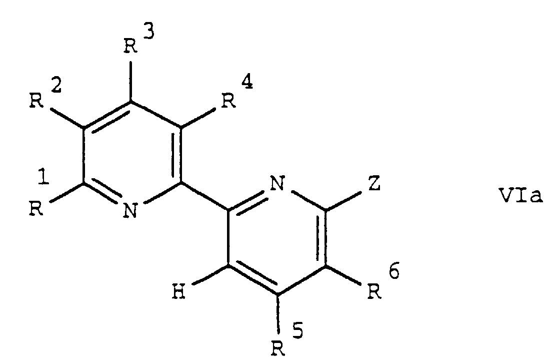 dans laquelle R¹ - R⁶ ont la signification telle que dans la formule I de la revendication 1 et Z étant un halogène,
α) avec une combinaison organique Li de formule VIII,
R¹³-Li VIII,
dans laquelle R¹³ = C₁-C₆-alkyle, phényle, pour donner une combinaison de formule IX a, 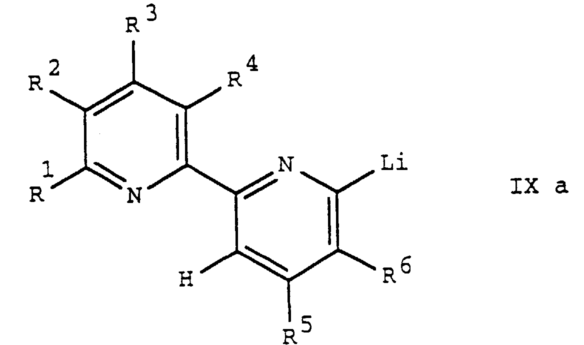 ou
β) avec Mg pour donner une combinaison de formule IX b 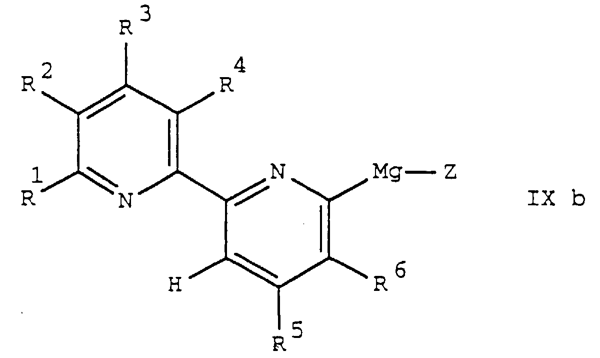 et on met en réaction la combinaison métallorganique de formule IX a, respectivement IX b, avec une combinaison de formule X 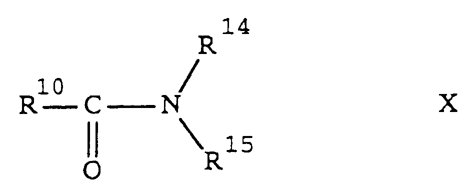
dans laquelle R¹⁰ a la signification telle dans la formule I de la revendication 1 et R¹⁴, R¹⁵ =, indépendamment les uns des autres, C₁-C₄-alkyle, C₁-C₄-alcoxy, C₅-C₇-cycloalkyle, ou R¹⁴ et R¹⁵, conjointement avec l'atome d'azote auquel ils sont liés, constituent un hétérocycle à 5-7 maillons, avec 1 ou 2 hétéroatomes identiques ou différents, de préférence avec les hétéroatomes d'azote et/ou d'oxygène.

12. Procédé de préparation de combinaisons de formule V a 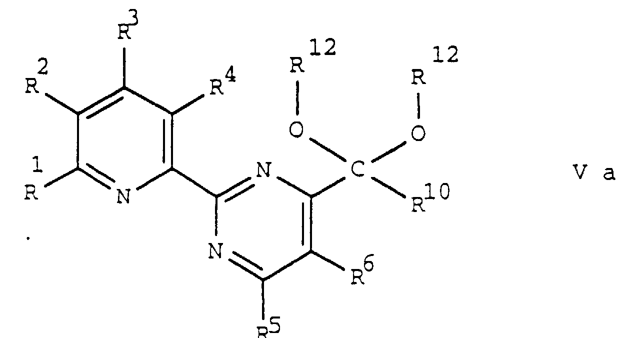 dans laquelle R¹ - R⁶, R¹⁰ et R¹² ont la signification comme dans la formule V de la revendication 9 ou 10, caractérisé par le fait qu'on met en réaction un dérivé de picolinamidine de formule XI a, respectivement XI b, 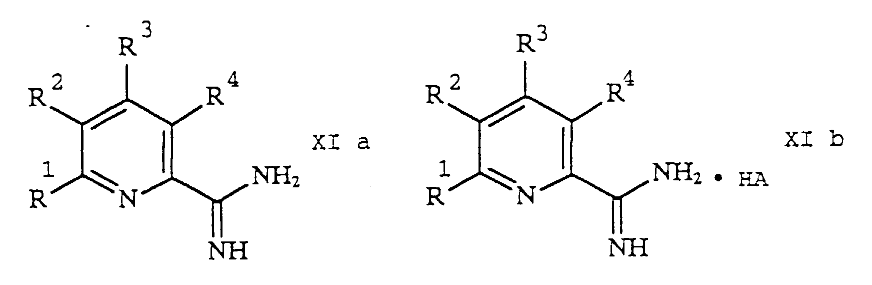 dans laquelle R¹ - R⁴ ont la signification telle que dans la formule I de la revendication 1 et HA étant un acide minéral ou organique,
α) avec un composé dicarbonyle de formule XII, 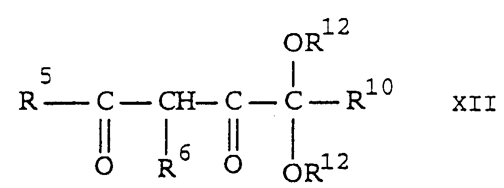 dans laquelle R⁶ et R¹⁰ ont la signification telle que dans la formule I de la revendication 1, R⁵ la signification telle que dans la formule I de la revendication 1 et R¹² la signification telle que dans la formule V de la revendication 9 ou 10, ou
β) avec une cétone de formule XIII, 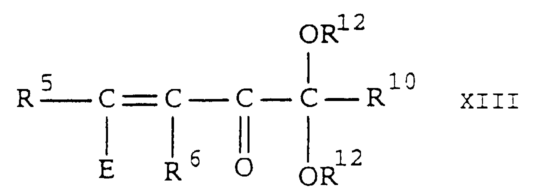 dans laquelle R⁶ et R¹⁰ ont la signification telle que dans la formule I de la revendication 1, R⁵ la signification telle que dans la formule I de la revendication 1 et R¹² la signification telle que dans la formule V de la revendication 9 ou 10,
et E étant OR¹⁶ ou NR¹⁶R¹⁸, avec R¹⁶ = C₁-C₄-alkyle et R¹⁷, R¹⁸, indépendamment les uns des autres, hydrogène, C₁-C₄-alkyle ou R¹⁷ et R¹⁸ constituent, conjointement avec l'atome d'azote auquel ils sont liés, un hétérocycle à 5 à 7 maillons avec 1 ou 2 hétéroatomes identiques ou différents, de préférence avec les hétéroatomes d'azote et/ou d'oxygène,
le cas échéant en présence d'une base.
